(19) Europäisches Patentamt
European Patent Office
Office européen des brevets

(11) EP 2 379 558 B1

(12) # EUROPÄISCHE PATENTSCHRIFT

(45) Veröffentlichungstag und Bekanntmachung des Hinweises auf die Patenterteilung:
**23.03.2016 Patentblatt 2016/12**

(21) Anmeldenummer: **09796971.1**

(22) Anmeldetag: **22.12.2009**

(51) Int Cl.:
***C07D 487/04*** (2006.01) ***A61K 31/519*** (2006.01)
***A61P 35/00*** (2006.01)

(86) Internationale Anmeldenummer:
**PCT/EP2009/009218**

(87) Internationale Veröffentlichungsnummer:
**WO 2010/083870 (29.07.2010 Gazette 2010/30)**

(54) **NEUE HETEROCYCLISCHE VERBINDUNGEN ALS METAP-2 INHIBITOREN**

NOVEL HETEROCYCLIC COMPOUNDS FOR USE AS METAP-2 INHIBITORS

NOUVEAUX COMPOSÉS HÉTÉROCYCLIQUES COMME INHIBITEURS DE LA METAP-2

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **20.01.2009 DE 102009005193**

(43) Veröffentlichungstag der Anmeldung:
**26.10.2011 Patentblatt 2011/43**

(73) Patentinhaber: **Merck Patent GmbH**
**64293 Darmstadt (DE)**

(72) Erfinder:
- **HEINRICH, Timo**
**64823 Gross-Umstadt (DE)**
- **ZENKE, Frank**
**64291 Darmstadt (DE)**
- **KRIER, Mireille**
**64285 Darmstadt (DE)**
- **SCHIEMANN, Kai**
**64342 Seeheim-Jugenheim (DE)**

(56) Entgegenhaltungen:
**WO-A1-2007/017069**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 379 558 B1

## Beschreibung

Die Erfindung betrifft Verbindungen der Formel I

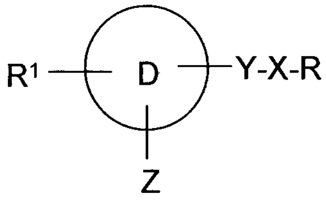

I

worin

| | |
|---|---|
| Ⓓ | Pyrrolidinyl, Imidazolinyl, Pyrazolyl, 2-Oxa-5-aza-bicyclo[2.2.1]hept-5-yl, Morpholinyl oder N, de |
| Z | Imidazopyrimidinyl, Triazolopyrimidinyl oder Pyrrolopyrimidinyl, wobei Z an einen Stickstoff des Restes D gebunden ist, |
| Y | $(CH_2)_n$ oder fehlt, |
| X | $O(CH_2)_r$, NH, NA, OC(=O), $NHSO_2$ oder fehlt, |
| $R^1$ | H, A oder COA, |
| R | Het, |
| Het | einen unsubstituierten oder ein-, zwei- oder dreifach durch Hal, A, $(CH_2)_mOR^6$, $(CH_2)_mN(R^6)_2$, $(CH_2)_mCN$, $(CH_2)_mCOOR^6$, $CONH(CH_2)_mCOOH$, $CONH(CH_2)_mHet^2$, $CO(CH_2)_mN(R^6)_2$, $CO(CH_2)_mNH(CH_2)_rCOOA$, $(CH_2)_rCONH(CH_2)_mAr^1$, $COCH[(CH_2)_mCONH_2]NH_2$, $COCH[(CH_2)_mCONH_2]NHCOOA$, $COCH[(CH_2)_mHet^2]NHCOOA$, $COCH(NHCOA)CH_2CONH_2$, $(CH_2)_mNHCOOA$, $COCH[(CH_2)_mCN]NHCOOA$, $COO(CH_2)_mCN$, $COO(CH_2)_mN(R^6)_2$, $CO(CH_2)_mCON(R^6)_2$, $COR^6$, $COHet^2$, $O(CH_2)_mAr^1$ und/oder =O (Carbonylsauerstoff) substituierten zweikernigen gesättigten, ungesättigten oder aromatischen Heterocyclus mit 1 bis 4 N-, und/oder O- und/oder S-Atomen, |
| $Het^2$ | Imidazolyl, Indolyl, Isoxazolyl, Pyridazinyl, Pyrazolyl, Benzimidazolyl, Pyridyl, Dibenzofuranyl, Carbazolyl, Benzofuranyl, Isoindolyl, Chinazolinyl, Chinoxalinyl, Pyrimidinyl, Indazolyl, Furyl, Thienyl, Pyrrolyl, Oxazolyl, Thiazolyl, Triazolyl, Tetrazolyl, Thiadiazol, Benzothiazolyl, Imidazo[1,2-a]pyridinyl oder 1,3-Benzodioxolyl, |
| $R^6$ | H oder Alkyl mit 1-6 C-Atomen, |
| $Ar^1$ | unsubstituiertes oder ein-, zwei- oder dreifach durch Hal und/oder $SO_2NH_2$ substituiertes Phenyl, |
| A | unverzweigtes oder verzweigtes Alkyl mit 1-10 C-Atomen, worin 1-7 H-Atome durch F, Cl, Br und/oder OH ersetzt sein können, oder cyclisches Alkyl mit 3-7 C-Atomen, |
| Hal | F, Cl, Br oder I, |
| m | 0, 1, 2, 3, 4, 5 oder 6, |
| n | 1 oder 2, |
| q | 0, 1, 2, 3 oder 4, |
| r | 0, 1 oder 2 |

bedeuten,
sowie ihre pharmazeutisch verwendbaren Salze, Tautomere und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen.

Insbesondere zeigen sie eine regulatorische, modulatorische und/oder inhibierende Wirkung auf Metallproteasen, vorzugsweise auf die Methionin-Amino-Peptidase (MetAP), besonders auf den Subtyp MetAP-2.

Sie können als Arzneimittel gegen Krebs aber auch als Arzneimittel, die den Fett-Stoffwechsel positiv beeinflussen, aber auch als Arzneimittel gegen Entzündungen verwendet werden.

Andere Purinderivate zur Krebsbekämpfung sind in der WO 2007/017069 offenbart.

In der WO 01/79157 sind substituierte Hydrazide und N-Alkoxyamide beschrieben, die MetAP-2 inhibitorische Aktivität aufweisen und zur Inhibierung von Angiogenese verwendet werden können, insbesondere zur Behandlung von Krankheiten, wie z.B. Krebs, deren Entwicklung von Angiogenese abhängt.
In der WO 02/081415 sind MetAP-2 Inhibitoren beschrieben, die zur Behandlung von Krebs, Hämangiom, proliferativer Retinopathie, rheumatoider Arthritis, atherosklerotischer Neovaskularisation, Psoriasis, okularer Neovaskularisation und

Fettleibigkeit verwendet werden können.
In der WO 2008/011114 sind Verbindungen als Angiogenese-Inhibitoren und MetAP-2-Inhibitoren beschrieben, die zur Behandlung von lymphoider Leukämie und Lymphom verwendet werden können.

Die Wirkung der erfindungsgemäßen Verbindungen gegen Krebs liegt besonders in ihrer Wirkung gegen Angiogenese. Angiogenese-Hemmung hat sich bei über 70 Krankheiten als hilfreich erwiesen, wie z. B. Eierstockkrebs (F. Spinella et al. J. Cardiovasc. Pharmacol. 2004, 44, S140), Brustkrebs (A. Morabito et al. Crit. Rev. Oncol./Hematol. 2004, 49, 91), Prostatakrebs (B. Nicholson et al. Cancer Metastas. Rev. 2001, 20, 297), diabetische Erblindung, Schuppenflechte und Makuladegeneration (E. Ng et al. Can. J. Ophthalmol. 2005, 23, 3706).

Proteasen regulieren viele unterschiedliche Zell-Prozesse, besonders die Modulation von Peptiden und Proteinen, besonders den Protein-Umsatz, die Protein-Reifung und Signalpeptid-Prozessierung, den Abbau von anormalen Proteinen sowie die In-/Aktivierung von regulatorischen Proteinen. Besonders die Amino-terminale Modifikation von naszierenden Polypeptiden stellt die häufigste Modulation dar. Aminoproteasen sind Metalloproteasen, die Aminosäuren vom ungeschützten N-Terminus von Peptiden oder Proteinen abspalten, was sowohl co- als auch posttranslatorisch erfolgen kann. Methionin Aminopeptidase (MetAP) spaltet terminales Methionin naszierender Peptide besonders, wenn die vorletzte Aminosäure klein und ungeladen ist (z. B. Gly, Ala, Ser, Thr, Val, Pro oder Cys).

Bei vielen Krankheitsprozessen steht die Angiogenese entweder ursächlich im Mittelpunkt der Erkrankung oder wirkt sich verschlimmernd auf die Progression der Erkrankung aus. Beispielsweise im Krebsgeschehen führt die Angiogenese dazu, dass der Tumor sich vergrößern und in andere Organe übertreten kann. Weitere Erkrankungen, bei denen Angiogenese eine wichtige Rolle spielt sind Psoriasis, Arthrose, Arteriosklerose sowie Augenerkrankungen wie diabetische Retinopathie, altersbedingte makulare Degeneration, Rubeosis iridis oder neovasculäres Glaukom, ferner bei Entzündungen. Die dieser Erfindung zugrunde liegenden Verbindungen der Formel I, Zusammensetzungen, die diese Verbindungen enthalten, sowie die beschriebenen Verfahren können somit zur Behandlung dieser Krankheiten eingesetzt werden.

Dementsprechend werden die erfindungsgemäßen Verbindungen oder ein pharmazeutisch unbedenkliches Salz davon für die Behandlung von Krebs verabreicht, einschließlich solider Karzinome, wie zum Beispiel Karzinome der Lungen, des Pankreas, der Schilddrüse, der Harnblase oder des Kolons, myeloische Erkrankungen (z. B. myeloische Leukämie) oder Adenome (z. B. villöses Kolonadenom).
Zu den Tumoren zählen weiterhin die Monozytenleukämie, Hirn-, Urogenital-, Lymphsystem-, Magen-, Kehlkopf- und Lungenkarzinom, darunter Lungenadenokarzinom und kleinzelliges Lungenkarzinom, Bauchspeicheldrüsen- und/oder Brustkarzinom.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen anticancerogene Wirkung aufweisen. Die erfindungsgemäßen Verbindungen werden an einen Patienten mit einer Erkrankung verabreicht, z. B. zur Inhibierung des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation/ Vitalität wird durch Verabreichung der erfindungsgemäßen Verbindungen vor Entwicklung der evidenten Krankheit erreicht, z. B. zur Verhinderung des Tumorwachstums. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in vitro können kultivierte Zellen aus einer Biopsieprobe verwendet werden. Die Menge nach der Behandlung zurückbleibenden Zellen werden dann bestimmt. Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern,

während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Es wurde gefunden, dass die erfindungsgemäßen Verbindungen eine spezifische Inhibierung der MetAP-2 bewirken. Die erfindungsgemäßen Verbindungen zeigen bevorzugt eine vorteilhafte biologische Aktivität, die in den, zum Beispiel hierin beschriebenen Tests nachweisbar ist. In derartigen Tests zeigen und bewirken die erfindungsgemäßen Verbindungen einen inhibierenden Effekt, der gewöhnlich durch $IC_{50}$-Werte in einem geeigneten Bereich, bevorzugt im mikromolaren Bereich und bevorzugter im nanomolaren Bereich dokumentiert wird.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemotherapien und -bestrahlungen wiederherzustellen.

Unter Verbindungen der Formel I versteht man auch die Hydrate und Solvate dieser Verbindungen, ferner pharmazeutisch verwendbare Derivate. Gegenstand der Erfindung sind auch die optisch aktiven Formen (Stereoisomeren), Salze, die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:

> verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I sowie ihrer pharmazeutisch verwendbaren Salze, Tautomeren und Stereoisomeren, dadurch gekennzeichnet, daß man

eine Verbindung der Formel II

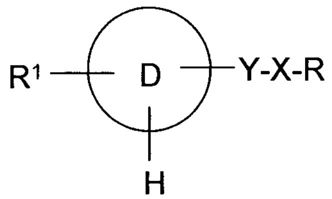

II

worin $R^1$, X, Y und R die in Anspruch 1 angegebenen Bedeutungen haben,

mit einer Verbindung der Formel III

Z-Cl III

worin Z die in Anspruch 1 angegebene Bedeutung hat,

umsetzt,

und/oder eine Base oder Säure der Formel I in eines ihrer Salze umwandelt.

Vor- und nachstehend haben die Reste R, X, Y, Z, $R^3$ und $R^4$ die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch

Pentyl, 1-, 2- oder 3-Methylbutyl, 1,1-, 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1,2,3,4,5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cycloalkyl bedeutet vorzugsweise Cyclopropyl, Cyclobutyl, Cylopentyl, Cyclohexyl oder Cycloheptyl.

$R^1$ bedeutet vorzugsweise H, A oder COA, ganz besonders bevorzugt H, Methyl oder Acetyl.

$R^6$ bedeutet vorzugsweise H, Methyl,Ethyl, Propyl oder Butyl.

$Ar^1$ bedeutet vorzugsweise unsubstituiertes oder ein-, zwei- oder dreifach durch Hal und/oder $SO_2NH_2$ substituiertes Phenyl.

X bedeutet vorzugsweise $O(CH_2)_r$, NH, NA, OC(=O), $NHSO_2$ oder fehlt.

Y bedeutet vorzugsweise $(CH_2)_n$ oder es fehlt.

R bedeutet vorzugsweise Het.

Het bedeutet, ungeachtetet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3-oder -5-yl, 1,3,4-Thiadiazol-2- oder -5-yl, 1,2,4-Thiadiazol-3- oder -5-yl, 1,2,3-Thiadiazol-4- oder-5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]-oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Die heterocyclischen Reste können auch teilweise oder vollständig hydriert sein.

Unsubstituiertes Het kann also z. B. auch bedeuten 2,3-Dihydro-2-, -3-, -4-oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4-oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4-oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3-oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet weiterhin vorzugsweise einen unsubstituierten oder ein-, zwei- oder dreifach durch Hal, A, $(CH_2)_mOR^6$, $(CH_2)_mN(R^6)_2$, $(CH_2)_mCN$, $(CH_2)_mCOOR^6$, $CONH(CH_2)_mCOOH$, $CONH(CH_2)_mHet^2$, $CO(CH_2)_mN(R^6)_2$, $CO(CH_2)_mNH(CH_2)_rCOOA$, $(CH_2)_rCONH(CH_2)_mAr^1$, $COCH[(CH_2)_mCONH_2]NH_2$, $COCH[(CH_2)mCONH_2]NHCOOA$, $COCH[(CH_2)_mHet^2]NHCOOA$, $COCH(NHCOA)CH_2CONH_2$, $(CH_2)_mNHCOOA$, $COCH[(CH_2)_mCN]NHCOOA$, $COO(CH_2)_mCN$, $COO(CH_2)_mN(R^6)_2$, $CO(CH_2)_mCON(R^6)_2$, $COR^6$, $COHet^2$, $O(CH_2)_mAr^1$ und/oder =O (Carbonylsauerstoff) substituierten zweikernigen gesättigten, ungesättigten oder aromatischen Heterocyclus mit 1 bis 4 N-, und/oder O- und/oder S-Atomen,

Het bedeutet besonders bevorzugt unsubstituiertes oder ein-, zwei- oder dreifach durch Hal, A, $(CH_2)_mOR^6$, $(CH_2)_mN(R^6)_2$, $(CH_2)_mCN$, $(CH_2)_mCOOR^6$, $CONH(CH_2)_mCOOH$, $CONH(CH_2)_mHet^2$, $CO(CH_2)_mN(R^6)_2$, $CO(CH_2)_mNH(CH_2)_rCOOA$, $(CH_2)_rCONH(CH_2)_mAr^1$, $COCH[(CH_2)_mCONH_2]NH_2$, $COCH[(CH_2)_mCONH_2]NHCOOA$, $COCH[(CH_2)_mHet^2]NHCOOA$, $COCH(NHCOA)CH_2CONH_2$, $(CH_2)_mNHCOOA$, $COCH[(CH_2)_mCN]NHCOOA$, $COO(CH_2)_mCN$, $COO(CH_2)_mN(R^6)_2$, $CO(CH_2)_mCON(R^6)_2$, $COR^6$, $COHet^2$, $O(CH_2)_mAr^1$ und/oder =O substituiertes Pyridazinyl, Pyrazolyl, Benzimidazolyl, Pyridyl, Dibenzofuranyl, Carbazolyl, Indolyl, Dihydro-Indolyl, Benzofuranyl, Dihydrobenzofuranyl, Piperazinyl, Morpholinyl, Chinolinyl, Isochinolinyl, Isoindolyl, Dihydrochinolinyl, Dihydroisochinolinyl, Tetrahydrochinolinyl, Tetrahydroisochinolinyl, Chinazolinyl, Chinoxalinyl, Phthalazinyl, Purinyl, Naphthyridinyl, Pyrimidinyl, Indazolyl, Furyl, Thienyl, Imidazolyl, Pyrrolyl, Oxazolyl, Isoxazolyl, Thiazolyl, Triazolyl, Tetrazolyl, Thiadiazol, Benzothiazolyl, Imidazo[1,2-a]pyridinyl, 1,3-Benzodioxolyl oder Benzoxazolyl.

Het bedeutet weiterhin vorzugsweise einen ein-, zwei- oder dreifach durch Hal, A, $(CH_2)_mOR^6$, $(CH_2)_mN(R^6)_2$, $(CH_2)_mCN$, $(CH_2)_mCOOR^6$, $CONH(CH_2)_mCOOH$, $CONH(CH_2)_mHet^2$, $CO(CH_2)_mN(R^6)_2$, $CO(CH_2)_mNH(CH_2)_rCOOA$, $(CH_2)_rCONH(CH_2)_mAr^1$, $COCH[(CH_2)_mCONH_2]NH_2$, $COCH[(CH_2)_mCONH_2]NHCOOA$, $COCH[(CH_2)_mHet^2]NHCOOA$,

$COCH(NHCOA)CH_2CONH_2$, $(CH_2)_mNHCOOA$, $COCH[(CH_2)_mCN]NHCOOA$, $COO(CH_2)_mCN$, $COO(CH_2)_mN(R^6)_2$, $CO(CH_2)_mCON(R^6)_2$, $COR^6$, $COHet^2$, $O(CH_2)_mAr^1$ und/oder =O substituiertes Chinolinyl, Isochinolinyl, Pyrazolyl oder Isoindolyl.

Cyc bedeutet vorzugsweise Cyclobutylen,Cyclopentylen oder Cyclohexylen.

$Het^2$ bedeutet vorzugsweise unsubstituiertes oder ein- oder zweifach durch A, $OR^6$, NHCOA und/oder =O (Carbonylsauerstoff) substituiertes Furyl, Thienyl, Pyrrolyl, Oxazolyl, Isoxazolyl, Thiazolyl, Imidazolyl, Pyridyl, Pyrimidinyl, Piperazinyl, Morpholinyl, Piperidinyl, Pyrrolidinyl, Azetidinyl, Dihydrofuranyl oder Tetrahydrofuranyl.

$Het^2$ bedeutet besonders bevorzugt Imidazolyl, Indolyl, Isoxazolyl, Pyridazinyl, Pyrazolyl, Benzimidazolyl, Pyridyl, Dibenzofuranyl, Carbazolyl, Benzofuranyl, Isoindolyl, Chinazolinyl, Chinoxalinyl, Pyrimidinyl, Indazolyl, Furyl, Thienyl, Pyrrolyl, Oxazolyl, Thiazolyl, Triazolyl, Tetrazolyl, Thiadiazol, Benzothiazolyl, Imidazo[1,2-a]pyridinyl oder 1,3-Benzodioxolyl.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind. Die Verbindungen der Formel I können ein oder mehrere chirale Zentren besitzen und daher in verschiedenen stereoisomeren Formen vorkommen. Die Formel I umschließt alle diese Formen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man Verbindungen der Formel II mit einer Verbindung der Formel III umsetzt. Die Verbindungen der Formel II und der Formel III sind in der Regel bekannt. Sind sie neu, so können sie aber nach an sich bekannten Methoden hergestellt werden.

Die Umsetzung erfolgt in einem inerten Lösungsmittel und erfolgt in der Regel in Gegenwart eines säurebindenden Mittels vorzugsweise einer organischen Base wie DIPEA, Triethylamin, Dimethylanilin, Pyridin oder Chinolin. Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -15° und 150°, normalerweise zwischen 40° und 130°, besonders bevorzugt zwischen 60° und 110°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder-monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt sind Glykolether, wie Ethylenglycolmonomethylether, THF, Dichlormethan und/oder DMF.

Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B.

Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden

Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie ($C_1$-$C_4$) Alkylhalogeniden, z.B. Methyl-, Ethyl-, Isopropyl- und tert.-Butylchlorid, -bromid und -iodid; Di($C_1$-$C_4$)Alkylsulfaten, z.B. Dimethyl-, Diethyl- und Diamylsulfat; ($C_{10}$-$C_{18}$)Alkyl-halogeniden, z.B. Decyl-, Dodecyl-, Lauryl-, Myristyl- und Stearylchlorid, -bromid und -iodid; sowie Aryl-($C_1$-$C_4$)Alkylhalogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt

werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und

Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von

(a) einer wirksamen Menge an einer Verbindung der Formel I und/oder ihrer pharmazeutisch verwendbaren Salze und Stereoisomere, einschließlich deren Mischungen in allen Verhältnissen,
und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze und Stereoisomere, einschließlich deren Mischungen in allen Verhältnissen,
und einer wirksamen Menge eines weiteren Arzneimittelwirkstoffs gelöst oder in lyophilisierter Form vorliegt.

Gegenstand der Erfindung sind auch Arzneimittel, enthaltend mindestens eine Verbindung nach Anspruch 9 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

VERWENDUNG

Die vorliegenden Verbindungen eignen sich als pharmazeutische Wirkstoffe für Säugetiere, insbesondere für den Menschen, bei der Behandlung und Bekämpfung von Krankheiten. Zu diesen Krankheiten zählen die Proliferation von Tumorzellen, die pathologische Gefäßneubildung (oder Angiogenese), die das Wachstum fester Tumoren fördert, die Gefäß neubildung im Auge (diabetische Retinopathie, altersbedingte Makula-Degeneration und dergleichen) sowie Entzündung (Schuppenflechte, rheumatoide Arthritis und dergleichen), sowie proliferative Erkrankungen der Mesangiumzellen.

Die vorliegende Erfindung umfasst die Verwendung der Verbindungen der Formel I und/oder ihre physiologisch unbedenklichen Salze und Solvate zur Herstellung eines Arzneimittels zur Behandlung oder Vorbeugung von Tumoren, Tumorerkrankungen und/oder Tumormetastasen.
Die Tumorerkrankung ist vorzugsweise ausgewählt aus der Gruppe Tumor des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom.

Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung von Osteoporose, Diabetes und Fettleibigkeit.

Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die angiogene Erkrankung ist vorzugsweise ausgewählt aus der Gruppe diabetische Retinopathie, Arthritis, Krebs, Psoriasis, Kaposi Sarkom, Hemangioma, myocardiale Angiogenesis, atherosklerotische Plaque-Neovaskularisation, angiogene Augenerkrankungen, choroidale Neovaskularisation, retrolentale Fibroplasie, makulare Degeneration, corneale Transplantatabstossung, Rubeosis iridis, neurosculares Glaukom, Oster Webber Syndrom.

Die proliferative Erkrankung der Mesangiumzellen ist vorzugsweise ausgewählt aus der Gruppe

Glomerulonephritis, diabetische Nephropathie, maligne Nephrosclerose, thrombotisches Mikroangiopathie-Syndrom, Transplantatabstossung, Glomerulopathie.

Die Verwendung von Verbindungen der Formel I und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Die inflammatorische Erkrankung ist vorzugsweise ausgewählt aus der Gruppe entzündliche Darmerkrankung, Arthritis, Atherosclersose, Asthma, Allergien, entzündliche Nierenerkrankungen, multiple Sklerose, chronisch obstruktive Lungenerkrankung, entzündliche Hauterkrankungen, Pardontalerkrankungen, Psoriasis,
durch T-Zellen - vermittelte Immunerkrankung.

Die entzündliche Darmerkrankung ist vorzugsweise ausgewählt aus der Gruppe

ulcerative Colitis, Morbus Crohn, unbestimmte Colitis.

Die durch T-Zellen - vermittelte Immunerkrankung ist vorzugsweise ausgewählt aus der Gruppe

allergische Encephalomyelitis, allergische Neuritis, Transplantatabstossung, Graft-versus-Host-Reaktion, Myocarditis, Thyroiditis, Nephritis, systemischer Lupus erythematodes, insulinabhängiger Diabetes mellitus.

Die Arthritis-Erkrankung ist vorzugsweise ausgewählt aus der Gruppe rheumatoide Arthritis, Osteoarthritis, Caplan Syndrom, Felty Syndrom, Sjogren Syndrom, Spondylitis ankylosans, Morbus Still, Chondrocalcinosis, metabolische Arthritis, rheumatisches Fieber, Morbus Reiter, Wissler Syndrom.

Die entzündliche Nierenerkrankung ist vorzugsweise ausgewählt aus der Gruppe

Glomerulonephritis, glomeruläre Verletzung, nephrotisches Syndrom, interstitielle Nephritis, Lupus nephritis, Goodpasture-Syndrom, Wegener-Granulomatose, Nierenvaskulitis, IgA-Nephropathie, idiopatische glomeruläre Erkrankung.

Die entzündliche Hauterkrankung ist vorzugsweise ausgewählt aus der Gruppe

Psoriasis, atopische Dermatitis, Kontaktempfindlichkeit, Akne.

Ebenfalls umfasst ist die Verwendung der Verbindungen der Formel I und/oder ihre physiologisch unbedenklichen Salze und Solvate zur Herstellung eines Arzneimittels zur Behandlung oder Vorbeugung einer Krankheit bzw. eines Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden. Die vorliegende Erfindung umfasst auch die Verwendung Verbindungen der Formel I und/oder ihre physiologisch unbedenklichen Salze und Solvate zur Herstellung eines Arzneimittels zur Behandlung oder Vorbeugung von Retina-Vaskularisierung.

Ebenfalls umfasst ist die Verwendung der Verbindungen der Formel I und/oder ihre physiologisch unbedenklichen Salze zur Herstellung eines Arzneimittels zur Behandlung und/oder Bekämpfung einer durch Tumore bedingten Krankheit bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Die offenbarten Verbindungen der Formel I können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die Verbindungen der Formel I können auch gemeinsam mit anderen gut bekannten Therapeutika, die aufgrund

ihrer jeweiligen Eignung für das behandelte Leiden ausgewählt werden, verabreicht werden.

Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll. "Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5$\alpha$-Reduktase-Hemmer, Nilutamid, Flutamid, Bicalutamid, Liarozol und Abirateron-acetat. "Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, $\alpha$-Difluormethylornithin, ILX23-7553, trans-N-(4'-Hydroxyphenyl)retinamid und N-4-Carboxyphenylretinamid. "Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.

Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.

Zu den Mikrotubulin-Hemmern zählen zum Beispiel Paclitaxel, Vindesin-sulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797. Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]-pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxy-phenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropyl-amino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxan-then-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.

Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]gfycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

**Wirkungsnachweis von pharmakologischen Inhibitoren auf die Proliferation/Vitalität von Tumorzellen *in vitro***

## 1.0 Hintergrund

In der vorliegenden Versuchsbeschreibung wird die Hemmung der Tumorzellproliferation/ Tumorzellvitalität durch Wirkstoffe beschrieben.
Die Zellen werden in geeigneter Zelldichte in Mikrotiterplatten (96-well Format) ausgesät und die Testsubstanzen werden in Form einer Konzentrationreihe zugegeben. Nach vier weiteren Tagen der Kultivierung in serumhaltigem Medium kann die Tumorzellproliferation/ Tumorzellvitalität mittels eines Alamarblue-Testsystem bestimmt werden.

## 2.0 Versuchsdurchführung

### 2.1 Zellkultur

Beispielsweise käuflich erhältliche Colon-Carcinom-Zelllinien, Zelllinien des Eierstocks, Zellinien der Prostata oder Zelllinien der Brust etc.
Die Zellen werden in Medium kultiviert. In Abständen von mehreren Tagen werden die Zellen mit Hilfe von Trypsin-Lösung von den Kulturschalen abgelöst und in geeigneten Verdünnung in frischem Medium ausgesät. Die Zellen werden bei 37° Celsius und 10% $CO_2$ kultiviert.

### 2.2. Aussaat der Zellen

Eine definierte Zellzahl (z.B. 2000 Zellen) werden pro Kultur/ well in einem Volumen von 180μl Kulturmedium in Mikrotiterplatten (96 well Zellkulturplatten) mit einer Mehrkanalpipette ausgesät. Die Zellen werden anschließend in einem CO2-Brutschrank (37°C und 10% CO2) kultiviert.

### 2.3. Zugabe der Testsubstanzen

Die Testsubstanzen werden beispielsweise in DMSO gelöst und anschließend in entsprechender Konzentration (gegebenenfalls einer Verdünnungsreihe) im Zellkulturmedium eingesetzt. Die Verdünnungs-stufen können je nach Effizienz der Wirkstoffe und gewünschter Spreizung der Konzentrationen angepasst werden. Die Testsubstanzen werden in entsprechenden Konzentrationen mit Zellkulturmedium versetzt. Die Zugabe der Testsubstanzen zu den Zellen kann am selben Tag wie die Aussat der Zellen erfolgen. Dazu wird aus der Vorverdünnungsplatte jeweils 20μl Substanzlösung in die Kulturen/wells gegeben. Die Zellen werden für weitere 4 Tage bei 37°Celsius und 10% $CO_2$ kultiviert.

### 2.4. Messung der Farbreaktion

Pro well werden jeweils 20μl AlamarBlue Reagenz gegeben und die Microtiterplatten werden beispielsweise für weitere sieben Stunden in einem CO2-Brutschrank (bei 37°C und 10% CO2) inkubiert. Die Platten werden an einem Reader mit einem Fluoreszenzfilter bei einer Wellenlänge von 540nm gemessen. Die Platten können direkt vor der Messung leicht geschüttelt werden.

## 3. Auswertung

Der Extinktionswert der Mediumkontrolle (keine Verwendung von Zellen und Testsubstanzen) wird von allen anderen Extinktionswerten subtrahiert. Die Kontrollen (Zellen ohne Testsubstanz) werden gleich 100 Prozent gesetzt und alle anderen Extinktionswerte hierzu in Beziehung gesetzt (beispielsweise in % der Kontrolle) ausgedrückt:

Rechnung:

$$\frac{100 * (\text{Wert mit Zellen und Testsubstanz - Wert der Mediumkontrolle})}{(\text{Wert mit Zellen - Wert der Mediumkontrolle})}$$

Die Bestimmung von $IC_{50}$ Werten (50%ige Hemmung) erfolgt mit Hilfe von Statistikprogrammen wie z.B. RS1. $IC_{50}$-Daten erfindungsgemäßer Verbindungen sind in Tabelle 1 angegeben.

| Material | Best. Nr. | Hersteller |
|---|---|---|
| Mikrotiterplatten für die Zellkultur (Nunclon Surface 96well Plate) | 167008 | Nunc |
| DMEM | P04-03550 | Pan Biotech |
| PBS (10x) Dulbecco | 14200-067 | Gibco |
| 96well Platten (Polypropylen) | | 267334 Nunc |
| AlamarBlue™ | BUF012B | Serotec |
| FCS | 1302 | Pan Biotech GmbH |
| Trypsin/EDTA Solution 10x | L 2153 | Biochrom AG |
| 75cm$^2$ Kulturflaschen | 353136 | BD Falcon |
| A2780 | 93112519 | ECACC |
| Colo205 | CCL222 | ATCC |
| MCF7 | HTB22 | ATCC |
| PC3 | CRL-1435 | ATCC |

**Bestimmung der Proliferationshemmung durch Inhibitoren der Methioninaminopeptidase 2 im BrdU Proliferationstest (zellulärer Assay)**

Die Hemmung der Proliferation wird durch Inkorporation von Bromodesoxyuridin (BrdU) in humanen Endothelzellen aus der Nabelschnur (HUVEC, PromoCell, C-12200) bestimmt. Die HUVEC werden bei 37°C und 5% $CO_2$ in Basalmedium (PromoCell, C-22200) mit Supplementmix (PromoCell, C-39225) kultiviert. Nach Ablösung der Zellen mittels Trypsin/EDTA wird die Lebendzellzahl bestimmt und die Zellen in einer Dichte von 1000 Zellen pro Kavität in einem Gesamtvolumen von 175 μl ausgesät (Kavitäten werden zuvor entweder mit supplementiertem Kulturmedium für 1-2 Stunden bei 37°C oder mit 1,5% Gelatine für 0,5 - 2 Stunden bei 37°C beschichtet). Nach 24 stündiger Kultivierung werden die Testsubstanzen in verschiedenen Konzentrationen (z.B. finale Konzentrationen 30 μM bis 0,03 nM in 10-fach Verdünnungsschritten) und einem Volumen von 25 μl hinzugegeben. Die DMSO Konzentration wird mit 0,3% konstant gehalten. Nach insgesamt 48 oder 72 stündiger Kultivierung werden 20 μl Bromdesoxyuridin (Roche, # 11647229001 1:1000 verdünnt in Kulturmedium, Endkonzentration 10μM) hinzugegeben und für weitere 20 bis 24 Stunden kultiviert. Nach insgesamt 72 bzw. 96 Stunden Inkubation mit Testsubstanzen wird das Kulturmedium entfernt und ein immunhistochemischer Nachweis zur Detektion der BrdU-Inkorporation durchgeführt (BrdU ELISA, Roche, # 11647229001). Hierzu werden die Zellen für 30 min bei Raumtemperatur mit einem Fixativ behandelt und anschließend mit einem Peroxidase-markiertem anti-BrdU Antikörper (1:100 verdünnt in Antikörperverdünnungspuffer) für 60 min bei Raumtemperatur inkubiert. Nach dreimaligem Waschen mit 1-fach konzentriertem DPBS-Puffer (Gibco, # 14200) wird die enzymatische Umsetzung in TMB-Substratlösung initiiert. Die Farbentwicklung wird nach 15 min durch Zugabe von 25 μl einer 1 M Schwefelsäurelösung abgestoppt. Eine Bestimmung der optischen Dichte erfolgt innerhalb von 5 min durch Messung bei einer Wellenlänge von 450 nM. Als Kontrollen dienen Kavitäten mit DMSO-behandelten Zellen (100% Kontrolle) oder leere Kavitäten (Leerwert). Die Sensitivität dieses Tests gegenüber Inhibitoren der Methioninaminopeptidase wird durch Verwendung des Inhibitors Fumagillin überprüft und bestätigt.

**MetAP-2 Aktivitätsmessung**

Die MetAP-2 Aktivität wird durch eine Kopplung von enzymatischen Reaktionen nachgewiesen. Das Tripeptid Met-Arg-Ser (MAS) wird als Substrat eingesetzt. Das freigesetzte Methionin wird zunächst durch die L-Amino-oxidase (AAO) zu $Met_{ox}$ und $H_2O_2$ umgesetzt. Im zweiten Schritt katalysiert die Peroxidase (POD) mt Hilfe des $H_2O_2$ die Oxidation des Leukofarbstoffs Dianisidin zu $Dianisidin_{ox}$, dessen Zunahme photometrisch bei 450 nm detektiert wird.

MetAP-2 Aktivität kann als Kinetik kontinuierlich aufgezeichnet werden. Das Reaktionsschema verdeutlicht, dass pro mol Methionen ein mol $Dianisidin_{ox}$ gebildet wird. Die MetAP-2 Enzymaktivität lässt sich deshalb direkt als Δ Absorption pro Zeiteinheit berechnen. Eine Qualifizierung der MetAP-2 Aktivität (mol Met/Zeiteinheit) ist mit Hilfe des $Dianisidin_{ox}$-Extinktionskoeffizienten möglich.
Die Extinktionsänderung pro Zeiteinheit wird graphisch dargestellt und eine Steigungsberechnung im visuell linearen Bereich der Reaktion durchgeführt. Die Aktivitäten der Verbindungen sind in Tabelle 1 zusammengefasst.

Löslichkeitsmessung

Bestimmung nach "Shake flask solubility measurement" Eluentenherstellung:

| | |
|---|---|
| Eluent A: | 2 ml Diethylamin, zur Synthese +<br>1000 ml Methanol, LiChrosolv |
| Eluent B: | 5 g Ammoniumacetat, zur Analyse +<br>5 ml Methanol, LiChrosolv +<br>995 ml Reinstwasser |

Probenlösungsmittel:

Puffer: 3,954 g Natriumdihydrogenphosphat-Monohydrat + 6,024 g Natriumchlorid + 950 ml Reinstwasser mit 0,1 M NaOH oder 0,1 M HCl wird der pH-Wert eingestellt.

Probenvorbereitung:

Die Proben werden bei 37°C und 450 rpm 24 h lang geschüttelt.
Nach ca. 7h wird der pH Wert der Proben überprüft und gegebenenfalls nachgestellt.
Es wird auch kontrolliert, ob die Probe noch im Überschuss vorhanden ist.
Kurz vor Ende der 24h-Schüttelzeit werden die Proben nochmals auf pH-Wert und auf einen Niederschlag überprüft.
Reinstwasser Anlage: MilliQ Gradient, Millipore, Gerät: F3PN37462D
Schüttler: TiMix control, Bühler
Inkubationshaube: TH 15 Bühler
pH Meter: 766 Calimatic Knick Gerät: pH 1
pH Elektrode: InLab 423 Mettler
APCI-MS (atmospheric pressure chemical ionization - mass spectrometry) $(M+H)^+$.
Die Charakterisierung der Substanzen erfolgt in der Regel über HPLC-MS. Hier ist die verwendete Säule und der Gradient angegeben. Die berichtete Retentionszeit bezieht sich auf die MS-Spur (Retention in der UV oder ELSD Spur kann in der dritten Nachkommastelle abweichen).
HPLC: Säule: Chromolite Performance RP18-e 50-4,6mm
Gradient: ACN/$H_2O$ mit 0,04/0,05% Ameisensäure
Methode: Polar Chromolith/Chromolith (extended)
Fluß: 2,4mL/min
[M+H]

$^\$$Agilent Anlage

Chromolite Performance RP18-e 50-4,6mm
Gradient: ACN/H2O mit 0,04/0,05% Ameisensäure
Methode: Polar
Fluß: 2,4mL/min
Die Aufnahme der NMR-Spektren erfolgt in DMSO-$d_6$ und in DMSO-$d_6$ + TFA-$d_1$. Die angebenen Daten beziehen sich auf die DMSO-$d_6$ - Spektren.
Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

F.: Schmelzpunkt

Massenspektrometrie (MS):
EI (Elektronenstoß-Ionisation) $M^+$
FAB (Fast Atom Bombardment) $(M+H)^+$
ESI (Electrospray Ionization) $(M+H)^+$

APCI-MS (atmospheric pressure chemical ionization - mass spectrometry) $(M+H)^+$.

Syntheseschemata zur Herstellung einiger erfindungsgemäßer Verbindungen:

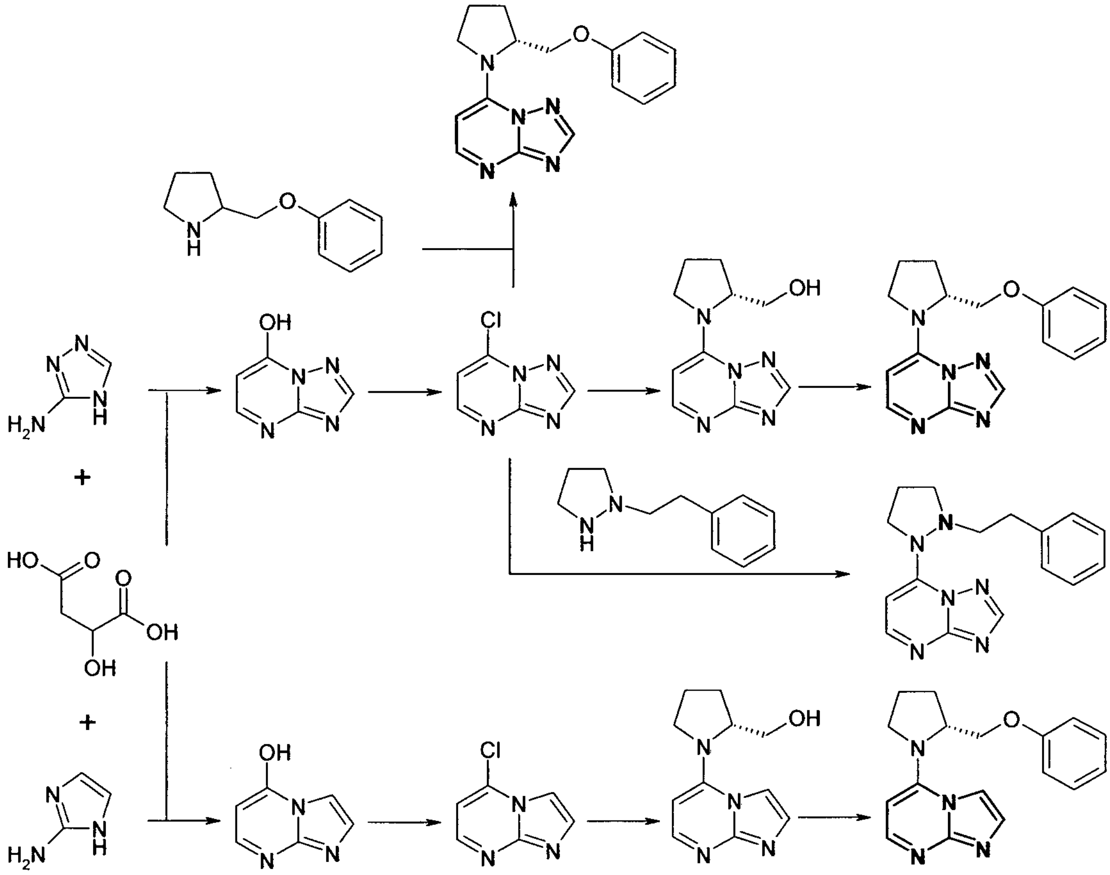

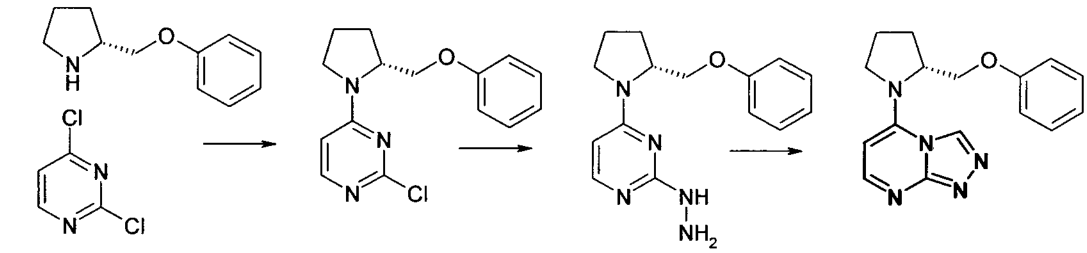

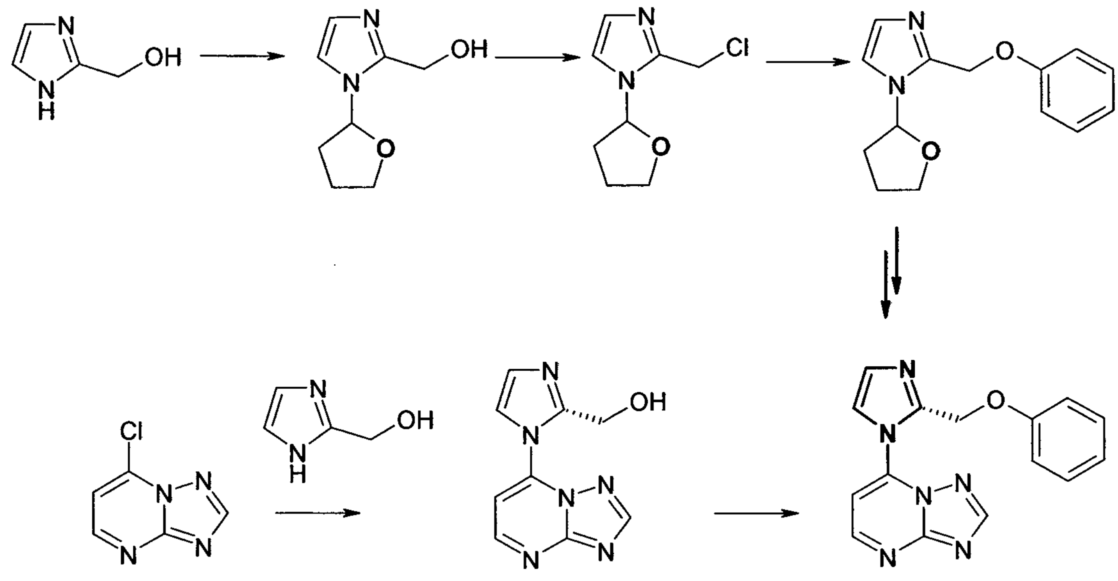

Beispiel 1 (Referenz)

Die Herstellung von 7-[(R)-2-(Naphthalin-1-yloxymethyf)-pyrrolidin-1-yl]-[1,2,4]triazolo[1,5-a]pyrimidin ("A1") erfolgt analog nachstehendem Schema

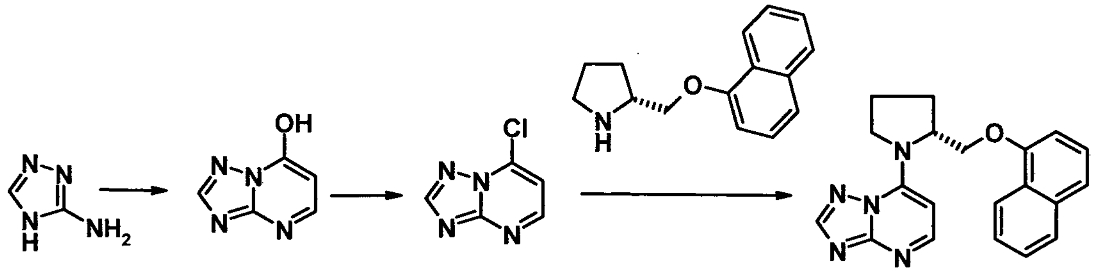

250 ml konz. Schwefelsäure werden auf 0°C abgekühlt und mit 90 g racemischer Äpfelsäure so versetzt, dass die Temperatur 10°C nicht überschreitet. Nachfolgend werden in analoger Weise 54 g 3-Amino-1,2,4-Triazol eingetragen. Nach 12 h bei RT wird die Suspension für eine Stunde auf 100°C erwärmt, wobei eine klare Lösung entsteht.

Zur Aufarbeitung lässt man die Reaktionslösung auf RT abkühlen und gießt den Ansatz auf 300 ml Eiswasser. Mit konzentrierter NaOH-Lösung wird ein pH-Wert von 6 eingestellt. Die dabei ausfallenden Kristalle werden abgesaugt, mit Wasser gewaschen und im Vakuum bei 100°C bis zur Massenkonstanz getrocknet. Das erhaltene Produkt [1,2,4]Triazolo[1,5-a]pyrimidin-7-ol schmilzt bei 290°C; HPLC: 0.475 Min; MS: [273.1]
(Yasuo Makisumi, Hideo Kano Bull Soc. Chem. Japan 1959, 8, 907; F. 286 - 287°C).

5.4 g [1,2,4]Triazolo[1,5-a]pyrimidin-7-ol aus dem ersten Syntheseschritt werden unter intensivem Rühren in 20 ml Phosphoroxychlorid eingetragen. Nach Vervollständigung wird noch für 1.5 h zum Rückfluß erwärmt. Zur Aufarbeitung wird der erkaltete Ansatz portionsweise in 100 ml Eiswasser eingerührt und nachfolgend mit konz. NaOH-Lösung auf pH 12 gebracht. Die wässrige Phase wird mit Ethylacetat extrahiert. Die vereinigten organischen Phasen werden über Natriumsulfat getrocknet und das Lösungsmittel im Vakuum entfernt. Das erhaltene 7-Chlor-[1,2,4]triazolo[1,5-a]pyrimidin wird ohne weitere Aufreinigung in der folgenden Reaktion eingesetzt; F. 190° [Lit. 186 - 187°C];
HPLC: 0.920 Min; MS: [157.0].

400 mg (R)-2-(Naphthalin-1-yloxymethyl)-pyrrolidin, 500 mg des oben hergestellten 7-Chlor-[1,2,4]triazolo[1,5-a]pyrimidin und 650 mg N-Ethyldiisopropylamin werden in 40 ml 1-Butanol für 3 h in der Mikrowelle auf 130°C erhitzt. Nachfolgend wird das Butanol im Vakuum entfernt, der Rückstand in Essigester aufgenommen, mit Wasser durchmischt, mit 1 N NaOH-Lösung auf pH 12 gestellt, Phasen getrennt, die organische Phase über Natriumsulfat getrocknet, eingeengt und über Kieselgel chromatographiert. Man erhält 640 mg "A1"; F. 74 - 75°;
HPLC: 2.386 min; MS: [346.1];
$^1$H NMR (500 MHz, $d_6$-DMSO) $\delta$ [ppm] 8.43 (s, 1H), 8.31 (d, 1H, J = 5.7 Hz), 7.83 (d, 1H, J = 8.1 Hz), 7.71 (d, 1H, J = 8.1 Hz), 7.49 (ddd, 1H, J = 1.3 Hz, J = 6.9 Hz, J = 8.1 Hz), 7.44 (d, 1H, J = 8.2 Hz), 7.40 (ddd, 1H, J = 1.3 Hz, J = 6.9

Hz, J = 8.1 Hz), 7.35 (t, 1H, J = 7.9 Hz), 6.9 (d, 1H, J = 7.7 Hz), 6.43 (d, 1H, J = 5.7 Hz), 5.74 (br. m, 1H), 4.39 (dd, 1H, J = 5.2 Hz, J = 9.7 Hz), 4.20 (dd, 1H, J = 6.5 Hz, J = 9.7 Hz), 4.00 (br. m, 1H), 3.80 (br. m, 1H), 2.33 - 2.22 (m, 3H), 2.10 (m, 1H).

Beispiel 2 (Referenz)

Die Herstellung von 7-Pyrazol-1-yl-[1,2,4]triazolo[1,5-a]pyrimidin ("A2") erfolgt analog nachstehendem Schema

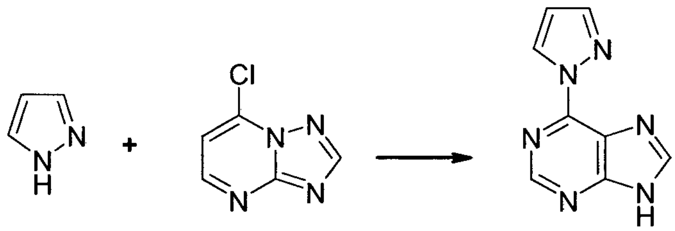

300 mg 7-Chlor-[1,2,4]triazolo[1,5-a]pyrimidin und 700 mg Pyrazol werden zusammen auf 120°C erhitzt, wobei eine Schmelze entsteht. Nach dem Abkühlen wird der Ansatz in Methanol teilweise gelöst, nach 30 Min. der unlösliche Rückstand abgetrennt und an der Luft getrocknet.
HPLC: 1.384 Min; MS: [187.1]; F.191 - 193°;
$^1$H NMR (500 MHz, $d_6$-DMSO) $\delta$ [ppm] 9.43 (d, 1H, 2.8 Hz), 8.95 (d, 1H, J = 5.0 Hz), 8.85 (s, 1H), 8.16 (d, 1H, J = 1.5 Hz), 7.81 (d, 1H, J = 5.0 Hz), 6.85 (dd, 1H, J = 1.6 J, = 2.8 Hz).

Beispiel 3 (Referenz)

Die Herstellung von 5-[(R)-2-(Naphthalin-1-yloxymethyl-pyrrolidin-1-yl]-imidazo[1,2-a]pyrimidin ("A3") erfolgt analog nachstehendem Schema

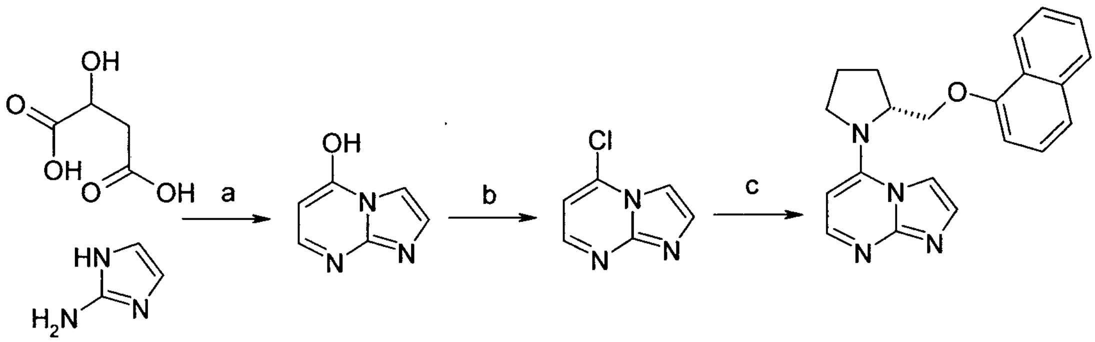


a) 9 g Äpfelsäure und 5.6 g 2-Aminoimidazol werden in 25 ml 0°C kalte rauchende Schwefelsäure gegeben. Diese Lösung wird für 12 h bei RT und eine Stunde am Rückfluß gerührt. Zur Aufarbeitung wird der Ansatz auf 100 g Eis gegeben und mit konz. NaOH-Lösung auf pH = 6 gestellt.

b) - c) analog Beispiel 1

Beispiel 4 (Referenz)

Die Herstellung von 5-[(R)-2-(Naphthalin-1-yloxymethyl)-pyrrolidin-1-yl]-[1,2,4]triazolo[4,3-a]pyrimidin ("A4") erfolgt analog nachstehendem Schema

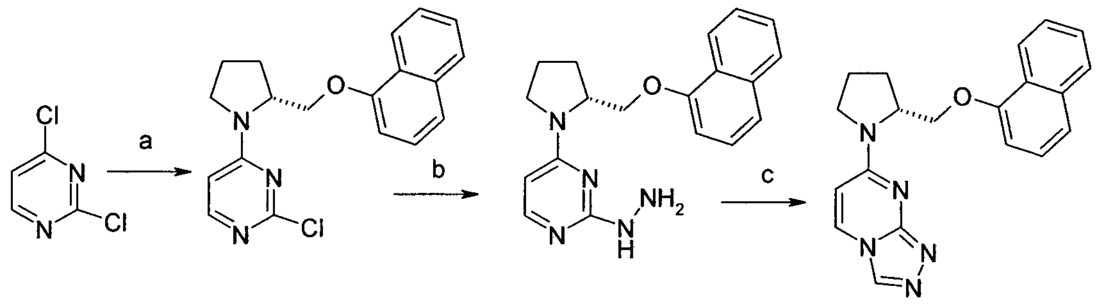


a) 327 mg 2,4-Dichlorpyrimidin werden in 20 ml Isopropanol gelöst und mit 0.8 ml N-Ethyldiisopropylamin und 500 mg (R)-2-(Naphthalin-1-yloxymethyl)-pyrrolidin versetzt. Diese Mischung wird für 6 h auf 80°C erwärmt. Der Ansatz wird nach dem Abkühlen auf 40 ml Wasser und 40 ml Essigsäureethylester gegossen. Nach der Phasentrennung wird die organische Phase getrocknet, eingeengt und an Kieselgel chromatographiert. Man erhält 480 mg 2-Chlor-4-[(R)-2-(naphthalin-1-yloxymethyl)-pyrrolidin1-yl]-pyrimidin als farbloses Öl;
HPLC: 2.831; MS [340.1].

b) 480 mg 2-Chlor-4-[(R)-2-(naphthalin-1-yloxymethyl)-pyrrolidin1-yl]-pyrimidin werden mit 5 g Hydrazin-Hydrat für 6 h auf 80°C erwärmt.
Man erhält 420 mg {4-[(R)-2-(Naphthalin-1-yloxymethyl)-pyrrolidin1-yl]-pyrimidin-2-yl}-hydrazin; HPLC: 1.751; MS [336.1].

c) 210 mg {4-[(R)-2-(Naphthalin-1-yloxymethyl)-pyrrolidin1-yl]-pyrimidin-2-yl}-hydrazin werden in 10 ml Triethylorthoformiat für 3 h auf 120°C erwärmt. Die ausfallenden Kristalle 5-[(R)-2-(Naphthalin-1-yloxymethyl)-pyrrolidin-1-yl]-[1,2,4]triazolo[4,3-a]pyrimidin werden abgesaugt und an der Luft getrocknet; HPLC: 1.846 Min; MS [346.2];
$^1$H NMR (500 MHz, $d_6$-DMSO) δ [ppm] 8.71 (s, 1H), 8.55 (d, 1H, J = 7.6 Hz), 8.14 (br. s, 1H), 7.86 (dd, 1H, J = 1.3 Hz, J= 7.6 Hz), 7.53 (dd, 1H, J = 1.3 Hz, J = 6.7 Hz), 7.49 (dd, 1H, J = 1.5 Hz, J = 8.9 Hz), 7.47 (d, 1H, J = 8.3 Hz), 7.39 (t, 1H, J = 7.9 Hz), 7.08 (br. s, 1H), 4.75 (br. s, 1H), 4.46 (br. s, 1H), 4.27 (br. s, 1H), 3.76 (br. m, 1H), 3.60 (br. s, 1H), 2.27 (br. m, 1H), 2.21 (br. m, 2H), 2.08 (br. m, 1H).

Beispiel 5

Die Herstellung von 5-((R)-1-[1,2,4]Triazolo[1,5-a]pyrimidin-7-yl-pyrrolidin-2-ylmethoxy)-chinolin ("A5") erfolgt analog nachstehendem Schema

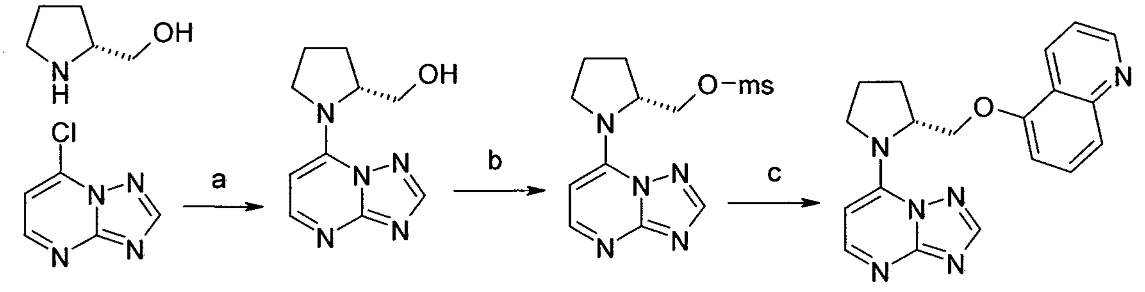


a) 1 g kommerziell erhältliches (D)-Prolinol, 1.5 g 7-Chlor-[1,2,4]triazolo[1,5-a]pyrimidin und 2.6 g N-Ethyldiisopropylamin werden in 60 ml 1-Butanol für 3 h in der Mikrowelle auf 130°C erhitzt. Nachfolgend wird das Butanol im Vakuum entfernt, der Rückstand in Essigester aufgenommen, mit Wasser durchmischt, mit 1 N NaOH-Lösung auf pH 12 gestellt, Phasen getrennt, die organische Phase über Natriumsulfat getrocknet, eingeengt und über Kieselgel chromatographiert. Man erhält 1.7 g ((R)-1-[1,2,4]Triazolo[1,5-a]pyrimidin-7-yl-pyrrolidin-2-yl)-methanol als beige Kristalle;
HPLC: 1.086 min; MS: [220.1].

b) 1.5 g ((R)-1-[1,2,4]Triazolo[1,5-a]pyrimidin-7-yl-pyrrolidin-2-yl)-methanol werden unter Rühren in 15 ml Dichlormethan gelöst und 1.9 ml Triethylamin hinzugefügt. Diese Lösung wird auf 0°C abgekühlt und tropfenweise mit einer Lösung von 0.8 ml Methansulfonsäurechlorid in 5 mL Dichlormethan versetzt. Nach vollständiger Zugabe lässt

man für 12 h auf RT erwärmen. Zur Aufarbeitung wird die Lösung auf gleiche Volumenanteile Wasser gegossen, die organische Phase mit Essigester erschöpfend extrahiert, die vereinigten organischen Phasen über Natriumsulfat getrocknet, abgesaugt, eingeengt und an Kieselgel chromatographiert. Man erhält 1.1 g Methansulfonsäure-((R)-1-[1,2,4]Triazolo[1,5-a]pyrimidin-7-yl-pyrrolidin-2-yl)-methylester als farbloses Öl;
HPLC: 1.424 Min; MS [298.1].

c) 250 mg Methansulfonsäure-((R)-1-[1,2,4]Triazolo[1,5-a]pyrimidin-7-yl-pyrrolidin-2-yl)-methylester, 120 mg 5-Hydroxychinolin und 1 g Cäsiumcarbonat werden in 20 ml DMF suspendiert und für 12 h bei 80°C gerührt. Nachfolgend wird das Lösungsmittel im Vakuum abgezogen, der Rückstand zwischen Essigester und Wasser verteilt, die organische Phase getrocknet und an Kieselgel chromatographisch gereinigt. Man erhält 90 mg 5-[(R)-1-[1,2,4]Triazolo[1,5-a]pyrimidin-7-yl-pyrrolidin-2-ylmethoxychinolin als beige Kristalle; HPLC: 1.312 Min; MS [347.1]; F. 93 - 95°; $^1$H NMR (500 MHz, $d_6$-DMSO) $\delta$ [ppm] 8.67 (d, 1H, J = 5.2 Hz), 8.42 (s, 1H), 8.31 (d, 1H, J = 5.6 Hz), 7.91 (d, 1H, J = 9.0 Hz), 7.71 (m, 2H), 7.47 (ddd, 1H, J = 1.0 Hz, J = 6.8 Hz, J = 7.8 Hz), 7.02 (d, 1H, J = 5.2 Hz), 6.43 (d, 1H, J = 5.6 Hz), 5.76 (br. m, 1H), 4.50 (dd, 1H, J = 5.2 Hz, J = 9.9 Hz), 4.32 (dd, 1H, J = 6.3 Hz, J = 9.9 Hz), 3.98 (m, 1H), 3.78 (m, 1H), 2.26 (m, 3H, 2.11 (m, 1H).

Beispiel 6 (Referenz)

Die Herstellung von 7-[2-(Naphthalin-1-yloxymethyl)-imidazol-1-yl]triazolo[1,5-a]pyrimidin ("A6") erfolgt analog nachstehendem Schema

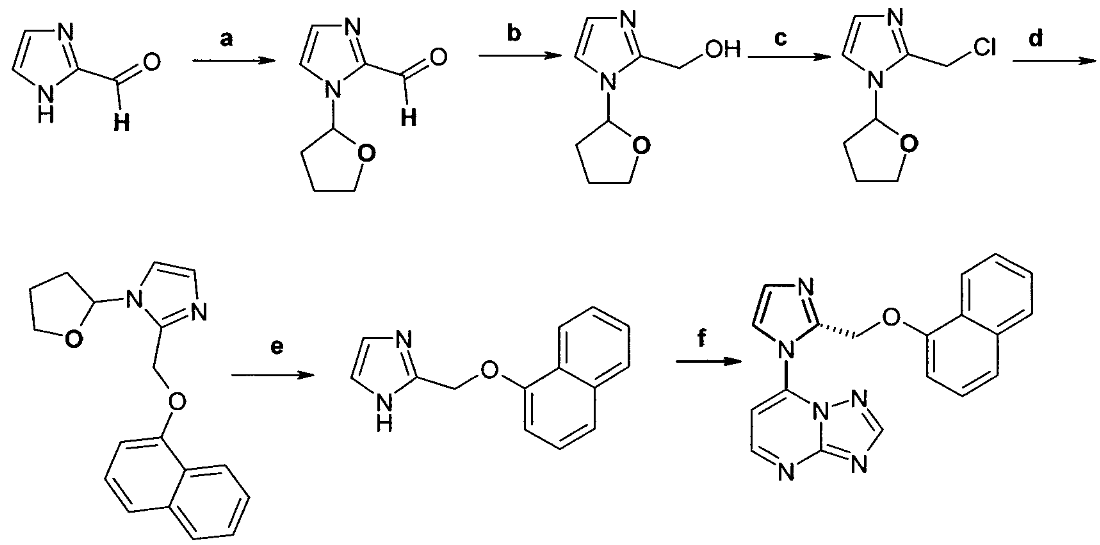


a) 5 g Imidazol-2-carboxaldehyd werden mit 7 g 2,3-Dihydrofuran und 0.8 g pToluolsulfonsäure in 100 ml THF für 12 h zum Rückfluß erhitzt. Nach dem Abkühlen wird das Lösungsmittel im Vakuum entfernt und der Rückstand zwischen Essigester und Wasser verteilt. Die organische Phase wird getrocknet, abfiltriert, eingeengt und an Kieselgel aufgereinigt. Man erhält 4.4 g eines gelben Öls, welches in der HPLC als Hydrat detektiert wird;
HPLC: 0.476; MS: [185.1].

b) 4.4 g [1-(Tetrahydrofuran-2-yl)-1H-imidazol-2-yl]-carboxaldehyd werden unter Stickstoff in 100 ml Methanol gelöst und portionsweise mit 1.9 g Natriumborhydrid versetzt. Nach 12 h ist die Reaktion vervollständigt und das Lösungsmittel wird im Vakuum entfernt. Der Rückstand wird zwischen Wasser und Essigester verteilt, die organische Phase getrocknet, abgesaugt und eingeengt. Das Produkt wird direkt weiter umgesetzt.

c) 2.8 g [1-(Tetrahydrofuran-2-yl)-1H-imidazol-2-yl]-methanol werden unter Stickstoff in 100 ml Dichlormethan gelöst und bei 0°C tropfenweise mit 2.4 ml Thionylchlorid versetzt. Nach beendeter Zugabe lässt man für 12h auf RT erwärmen. Der ausfallende Niederschlag 2-Chlormethyl)-1-(tetrahydrofuran-2-yl)-1H-imidazol Hydrochlorid wird abgesaugt, an der Luft getrocknet (1.6 g) und ohne weitere Aufreinigung in der folgenden Reaktion eingesetzt;
HPLC: 0.559; MS: [187.1].

d) 600 mg 2-Chlormethyl)-1-(tetrahydrofuran-2-yl)-1H-imidazol Hydrochlorid und 390 mg 1-Naphthol werden mit

1.2 g Kaliumcarbonat in 50 ml DMF bei 80°C für 12h gerührt. Der Ansatz wird zwischen gleichen Anteilen Essigester und Wasser verteilt, die organische Phase getrocknet, abfiltriert, bis zum Rückstand eingeengt und die erhaltenen 500 mg braunes Öl direkt weiter umgesetzt; HPLC: 1.672; MS: [295.1].

e) 500 mg 2-(Naphthilen-1-yloxymethyl)-1-(tetrahydrofuran-2-yl)-1H-imidazol werden mit 1.3 ml Trifluoressigsäure in 10 ml 1,2-Dichlorethan für 1h auf 80°C erwärmt. Die flüchtigen Bestandteile werden im Vakuum entfernt und man erhält 380 mg 2-(Naphthalin-1-yloxymethyl)-1H-imidazol welches ohne weitere Aufreinigung in der folgenden Reaktion eingesetzt wird.

f) analog letzte Stufe von Beispiel 1. Man erhält "A6".

Analog den Beispielen 1-6 werden die nachstehenden Verbindungen erhalten

| Verbindung Nr. | Struktur und/oder Name | F. [°C] | HPLC-MS; rt; [M+H+]$ |
|---|---|---|---|
| "A8" | 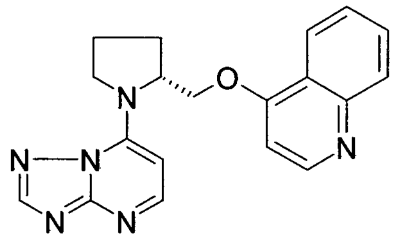 4-((R)-1-[1,2,4]Triazolo[1,5-a]pyrimidin-7-yl-pyrrolidin-2-ylmethoxy)-chinolin | 156-157 | 1.475 Min [347.1] |
| $^1$H NMR (500 MHz, $d_6$-DMSO) δ [ppm] 8.86 (dd, 1H, J = 1.7 Hz, J = 4.2 Hz), 8.42 (s, 1H), 8.31 (d, 1H, J = 5.6 Hz), 8.09 (d, 1H, J = 8.2 Hz), 7.58 (m, 2H), 7.42 (dd, 1H, J = 4.2 Hz, J = 8.4 Hz), 7.05 (d, 1H, J = 7.58 Hz), 6.42 (d, 1H, J = 5.7 Hz), 5.72 (br. m, 1H), 4.43 (dd, 1H, J = 5.1 Hz, J = 9.8 Hz), 4.24 (dd, 1H, J = 6.5 Hz, J = 9.8 Hz), 3.99 (br. m, 1H), 3.79 (br. m, 1H), 2.25 (m, 3H), 2.10 (m, 1H). | | | |
| "A9" | 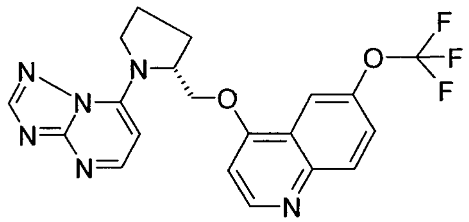 4-((R)-1-[1,2,4]Triazolo[1,5-a]pyrimidin-7-yl-pyrrolidin-2-ylmethoxy)-6-trifluormethoxy-chinolin | 77-78 | 1.804 Min [431.1] |
| $^1$H NMR (500 MHz, $d_6$-DMSO) δ [ppm] 8.75 (d, 1H, J = 5.1 Hz), 8.38 (s, 1H), 8.30 (d, 1H, J = 5.6 Hz), 8.06 (d, 1H, J = 9.1 Hz), 7.71 (dd, 1H, J = 2.2 Hz, J = 9.2Hz), 7.66 (s, 1H), 7.15 (d, 1H, J = 5.2 Hz), 6.44 (d, 1H, 5.6 Hz), 5.72 (br. m, 1H), 4.55 (dd, 1H, J = 5.2 Hz, J = 10.0 Hz), 4.37 (dd, 1H, J = 6.3 Hz, J = 10.0 Hz), 3.99 (br. m, 1H), 3.80 (br. m, 1H), 2.24 (m, 3H), 2.11 (m, 1H). | | | |
| "A10" | 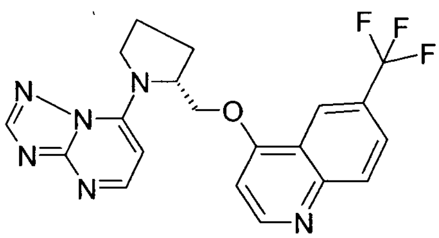 4-((R)-1-[1,2,4]Triazolo[1,5-a]pyrimidin-7-yl-pyrrolidin-2-ylmethoxy)-6-trifluormethyl-chinolin | 171-172 | 1.806 Min [415.1] |

(fortgesetzt)

| Verbindung Nr. | Struktur und/oder Name | F. [°C] | HPLC-MS; rt; [M+H+]$ |
|---|---|---|---|
| $^1H$ NMR (500 MHz, $d_6$-DMSO) δ [ppm] 8.85 (d, 1H, J = 5.2 Hz), 8.35 (s, 1H), 8.29 (d, 1H, J = 5.7 Hz), 8.18 (s, 1H), 8.12 (d, 1H, J = 8.8 Hz), 7.97 (dd, 1H, J = 2.0 Hz, J = 8.8Hz), 7.23 (d, 1H, J = 5.3 Hz), 6.46 (d, 1H, J = 5.7 Hz), 5.72 (br. m, 1H), 4.58 (dd, 1H, J = 5.5 Hz, J = 10.1 Hz), 4.41 (dd, 1H, J = 6.6 Hz, J = 10.1 Hz), 4.01 (br. m, 1H), 3.82 (br. m, 1H), 2.25 (m, 3H), 2.11 (m, 1H). | | | |
| "A11" | 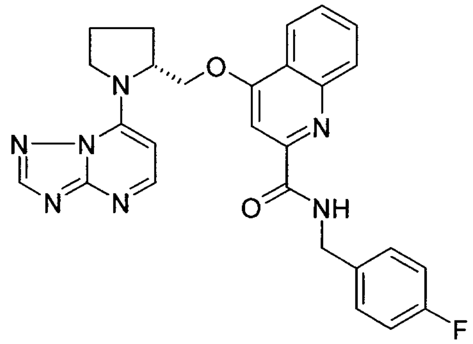 4-((R)-1-[1,2,4]Triazolo[1,5-a]pyrimidin-7-yl-pyrrolidin-2-ylmethoxy)-chinolin-2-carbonsäure-4-fluor-benzylamid | | 2.472 min [498.2] |
| $^1H$ NMR (500 MHz, $d_6$-DMSO) δ [ppm] 9.39 (t, 1H, J = 6.4 Hz), 8.48 (s, 1H), 8.31 (d, 1H, J = 5.6 Hz), 8.03 (d, 1H, J = 8.40 Hz), 7.81 (ddd, J = 1.2 Hz, J = 7.0 Hz, J = 8.3 Hz), 7.75 (d, 1H, J= 8.5 Hz), 7.73 (s, 1H), 7.57 (ddd, 1H, J = 0.8 Hz, J = 7.2 Hz, J = 7.9 Hz), 7.39 (dd, 2H, J = 5.6 Hz, J = 8.5 Hz), 7.14 (t, 2H, J = 8.9 Hz), 6.42 (d, 1H, J = 5.8 Hz), 5.79 (br. m, 1H), 4.64 (dd, 1H, J = 5.5 Hz, J = 10.0 Hz), 4.52 (d, 2H, J = 6.4 Hz), 4.43 (dd, 1H, J = 6.7 Hz, J = 10.0 Hz), 3.97 (br. m, 1H), 3.76 (br. m, 1H), 2.25 (m, 3H), 2.11 (m, 1H). | | | |
| "A12" | 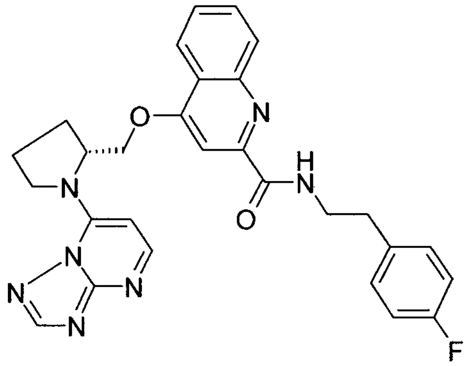 4-((R)-1-[1,2,4]Triazolo[1,5-a]pyrimidin-7-yl-pyrrolidin-2-ylmethoxy)-chinolin-2-carbonsäure-[2-(4-fluor-phenyl)-ethyl]-amid | | 2.537 min [513.2] |
| $^1H$ NMR (500 MHz, $d_6$-DMSO) δ [ppm] 8.87 (t, 1H, J = 6.1 Hz), 8.47 (s, 1H), 8.31 (d, 1H, J = 5.4 Hz), 8.00 (d, 1H, J = 8.3 Hz), 7.81 (ddd, 1H, J = 1.4 Hz, J = 6.9 Hz, J = 8.3 Hz), 7.75 (d, 1H, J = 8.2 Hz), 7.71 (s, 1H), 7.56 (ddd, 1H, J = 1.1 Hz, J = 6.9 Hz, J= 8.2 Hz), 7.29 (dd, 2H, J = 5.6 Hz, J = 8.5 Hz), 7.10 (t, 2H, J = 8.9 Hz), 6.42 (d, 1H, J = 5.7 Hz), 5.78 (br. m, 1H), 4.64 (dd, 1H; J = 5.5 Hz, J = 10.2 Hz), 4.43 (dd, 1H, J = 6.7 Hz, J = 10.2 Hz), 3.97 (br. m, 1H), 3.76 (br. m, 1H), 3.57 (q, 2H, J = 6.5 Hz), 2.89 (t, 2H, J = 7.5 Hz), 2.26 (m, 3H), 2.11 (m, 1H). | | | |

(fortgesetzt)

| Verbindung Nr. | Struktur und/oder Name | F. [°C] | HPLC-MS; rt; [M+H+]$ |
|---|---|---|---|
| "A13" | 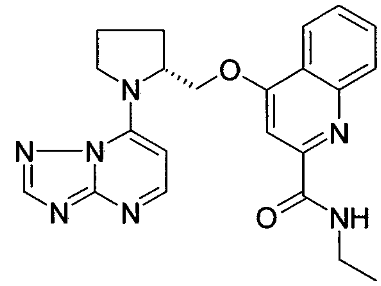 4-((R)-1-[1,2,4]Triazolo[1,5-a]pyrimidin-7-yl-pyrrolidin-2-ylmethoxy)-chinolin-2-carbonsäure-ethylamid | | 2.013 Min [418.1] |
| $^1$H NMR (500 MHz, $d_6$-DMSO) δ [ppm] 8.83 (t, 1H, J = 6.0 Hz), 8.48 (s, 1H), 8.31 (d, 1H, J = 5.7 Hz), 8.02 (d, 1H, J = 8.4 Hz), 7.80 (ddd, 1H, J = 1.3 Hz, J = 6.9 Hz, 8.3 Hz), 7.75 (d, 1H, J = 8.2 Hz), 7.72 (s, 1H), 7.56 (ddd, 1H, J = 0.9 Hz, J = 7.1 Hz, J = 8.0 Hz), 6.42 (d, 1H, J = 5.7 Hz), 5.78 (br. m, 1H), 4.64 (dd, 1H, J = 5.4 Hz, J = 10.0 Hz), 4.43 (dd, 1H, J = 6.7 Hz, J = 10.0 Hz), 3.97 (br. m, 1H), 3.77 (br. m, 1H), 3.37 (q, 2H, J = 7.2 Hz), 2.26 (m, 3H), 2.11 (m, 1H), 1.16 (t, 3H, J =7.2 Hz). | | | |
| "A16" | 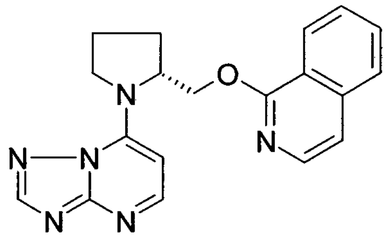 1-((R)-1-[1,2,4]Triazolo[1,5-a]pyrimidin-7-yl-pyrrolidin-2-ylmethoxy)-isochinolin | 72-74 | 2.195 min [347.1] |
| $^1$H NMR (500 MHz, $d_6$-DMSO) δ [ppm] 8.33 (s, 1H), 8.27 (d, 1H, J = 5.7 Hz), 7.87 (d, 1H, J = 5.8 Hz), 7.85 (d, 1H, J = 8.5 Hz), 7.72 (m, 2H), 7.52 (ddd, 1H, J = 1.0 Hz, J = 7.1 Hz, J =8.2 Hz), 7.34 (d, 1H, J = 5.7 Hz), 6.43 (d, 1H, 5.7 Hz), 5.64 (br. s, 1H), 4.64 (d, 2H, J = 5.5 Hz), 3.98 (br. s, 1H), 3.80 (br. m, 1H), 2.29 - 2.18 (m, 3H), 2.08 (m, 1H) | | | |
| "A17" | 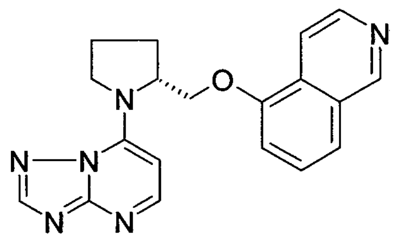 5-((R)-1-[1,2,4]Triazolo[1,5-a]pyrimidin-7-yl-pyrrolidin-2-ylmethoxy)-isochinolin | 158-159 | 1.408 min [347.1] |
| $^1$H NMR (500 MHz, $d_6$-DMSO) δ [ppm] 9.24 (s, 1H), 8.41 (s. 1H), 8.31 (d, 1H, J = 5.7 Hz), 7.64 (d, 1H, J = 8.2 Hz), 7.54 (d, 1H; J = 8.2 Hz), 7.53 (d, 1H, J )= 11.1 Hz), 7.23 (d, 1H, J = 7.6 Hz), 6.43 (d, 1H, J = 5.7 Hz), 5.72 (br. s, 1H), 4.44 (dd, 1H, J = 5.1 Hz, J = 9.8 Hz), 4.23 (dd, 1H, J = 6.4 Hz, J = 9.8 Hz), 4.00 (br. s, 1H), 3.79 (br. m, 1H), 2.29 (m, 3H), 2.11 (m, 1H) | | | |

(fortgesetzt)

| Verbindung Nr. | Struktur und/oder Name | F. [°C] | HPLC-MS; rt; [M+H+]$ |
|---|---|---|---|
| "A18" | 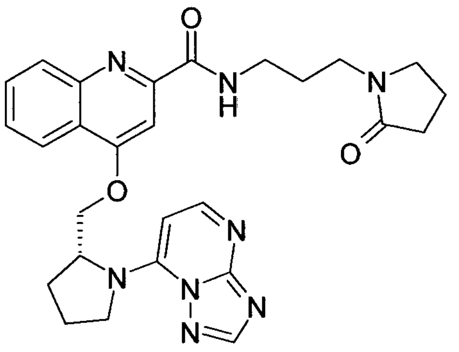 4-((R)-1-[1,2,4]Triazolo[1,5-a]pyrimidin-7-yl-pyrrolidin-2-ylmethoxy)-chinolin-2-carbonsäure-[3-(2-oxo-pyrrolidin-1-yl)-propyl]-amid | | 1.845min [515.2] |
| "A19" | 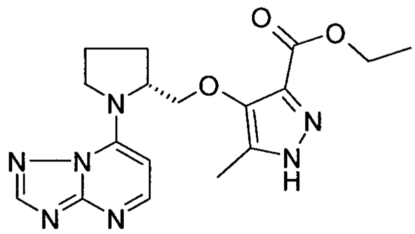 5-Methyl-4-((R)-1-[1,2,4]triazolo[1,5-a]pyrimidin-7-yl-pyrrolidin-2-ylmethoxy)-1H-pyrazol-3-carbonsäure-ethylester | | 1.666 min [372.2] |
| "A22" | 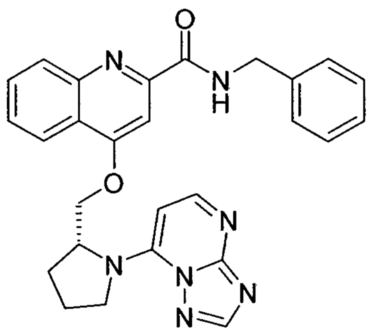 4-((R)-1-[1,2,4]Triazolo[1,5-a]pyrimidin-7-yl-pyrrolidin-2-ylmethoxy)-chinolin-2-carbonsäure-benzylamid | | 2.379 min [480.2] |
| "A23" | 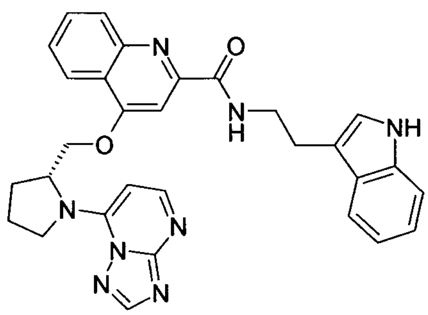 4-((R)-1-[1,2,4]Triazolo[1,5-a]pyrimidin-7-yl-pyrrolidin-2-ylmethoxy)-chinolin-2-carbonsäure-[2-(1H-indol-3-yl)-ethyl]-amid | | 2.336 min [533.2] |

(fortgesetzt)

| Verbindung Nr. | Struktur und/oder Name | F. [°C] | HPLC-MS; rt; [M+H+]$ |
|---|---|---|---|
| "A24" | 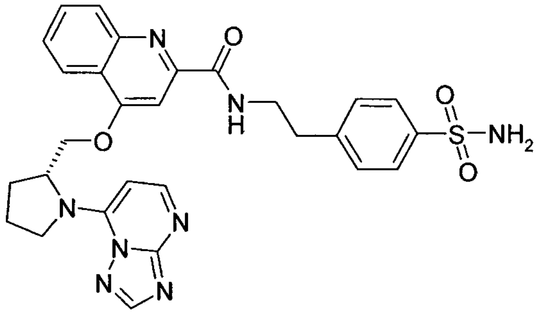<br>4-((R)-1-[1,2,4]Triazolo[1,5-a]pyrimidin-7-yl-pyrrolidin-2-ylmethoxy)-chinolin-2-carbonsäure-[2-(4-sulfamoyl-phenyl)-ethyl]-amid | | |
| "A25" | 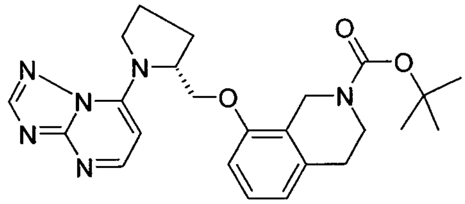<br>8-((R)-1-[1,2,4]Triazolo[1,5-a]pyrimidin-7-yl-pyrrolidin-2-ylmethoxy)-3,4-dihydro-1H-isochinolin-2-carbonsäure-tert.-butylester | | 2.492 min [451.2] |
| $^1$H NMR (500 MHz, $d_6$-DMSO) δ [ppm] 8.39 (s, 1H), 8.29 (d, 1H, J = 5.6 Hz), 7.07 (t, 1H, J = 7.8 Hz), 6.78 (br. d, 1H, J = 7.7 Hz), 6.72 (d, 1H, J = 7.4 Hz), 6.37 (br. d, 1H, J = 5.5 Hz), 5.49 (br. m, 1H), 4.34 (m, 2H), 4.25 (m, 1H), 4.10 (m, 1H), 3.97 (m, 1H), 3.81 (m, 1H), 3.50, m 2H), 2.71 (m, 2H), 2.25 - 2.18 m, 3H), 2.07 (m, 1H), 1.44 (s, 9H) | | | |
| "A26" | 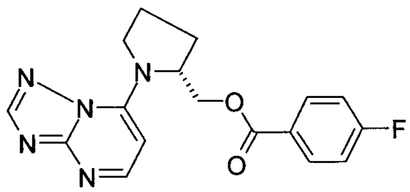<br>4-Fluor-benzoesäure-(R)-1-[1,2,4]triazolo[1,5-a]pyrimidin-7-yl-pyrrolidin-2-yl-methylester | | 2.020 Min [342.1] |
| "A27" | 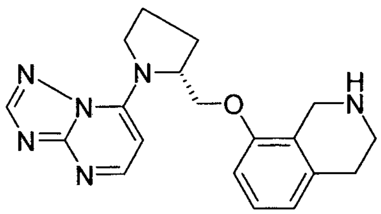<br>8-((R)-1-[1,2,4]Triazolo[1,5-a]pyrimidin-7-yl-pyrrolidin-2-ylmethoxy)-1,2,3,4-tetrahydroisochinolin | | 1.302 Min [351.2] |
| $^1$H NMR (500 MHz, $d_6$-DMSO) δ [ppm] 8.40 (s, 1H), 8.29 (d, 1H, J = 5.3 Hz), 6.98 (t, 1H, J = 7.8 Hz), 6.68 (d, 1H, J = 8.1 Hz), 6.62 (d, 1H, J = 7.5 Hz), 6.38 (d, 1H, J = 5.6 Hz), 5.54 (br. m, 1H), 4.16 (dd, 1H, J = 4.6 Hz, J = 9.8 Hz), 3.99 (dd, 1H, J = 5.9 Hz, J = 9.8 Hz), 3.94 (br. m, 1H), 3.77 (br. m, 1H), 3.50 (s, 2H), 2.83 (t, 2H, J = 5.7 Hz), 2.59 (t, 2H, J = 5.5 Hz), 2.23 - 2.17 (m, 2H), 2.15 - 2.10 (m, 1H), 2.05 (m, 1H) | | | |

(fortgesetzt)

| Verbindung Nr. | Struktur und/oder Name | F. [°C] | HPLC-MS; rt; [M+H+]$ |
|---|---|---|---|
| "A28" | 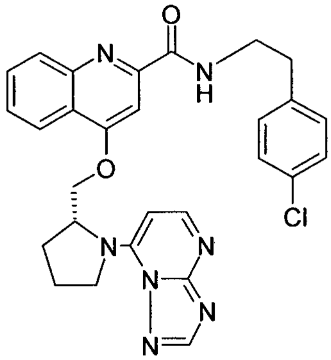 4-((R)-1-[1,2,4]Triazolo[1,5-a]pyrimidin-7-yl-pyrrolidin-2-ylmethoxy)-chinolin-2-carbonsäure-[2-(4-chlor-phenyl)-ethyl]-amid | | |
| "A29" | 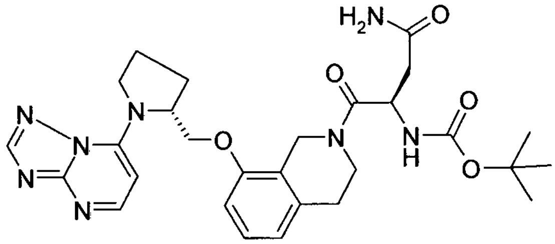 {(R)-1-Carbamoylmethyl-2-oxo-2-[8-((R)-1-[1,2,4]triazolo[1,5-a]pyrimidin-7-yl-pyrrolidin-2-ylmethoxy)-3,4-dihydro-1H-isochinolin-2-yl]-ethyl}-carbaminsäure-tert.-butylester | | 1.939 min [565.3] |
| "A30" | 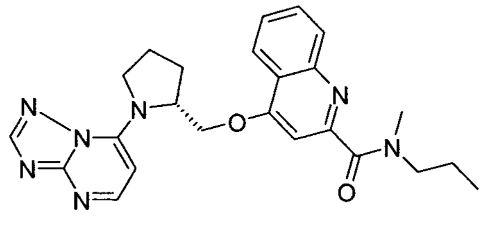 4-((R)-1-[1,2,4]Triazolo[1,5-a]pyrimidin-7-yl-pyrrolidin-2-ylmethoxy)-chinolin-2-carbonsäure-ethyl-propyl-amid | | |
| "A31" | 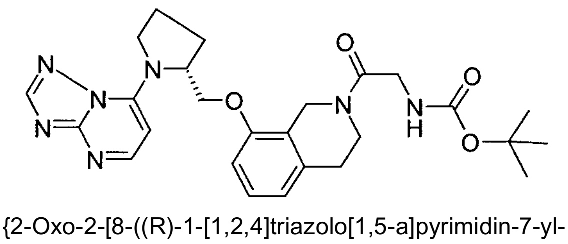 {2-Oxo-2-[8-((R)-1-[1,2,4]triazolo[1,5-a]pyrimidin-7-yl-pyrrolidin-2-ylmethoxy)-3,4-dihydro-1H-isochinolin-2-yl]-ethyl}-carbaminsäure-tert.-butylester | | 2.155 min [508.3] |

(fortgesetzt)

| Verbindung Nr. | Struktur und/oder Name | F. [°C] | HPLC-MS; rt; [M+H+]$ |
|---|---|---|---|
| "A32" | 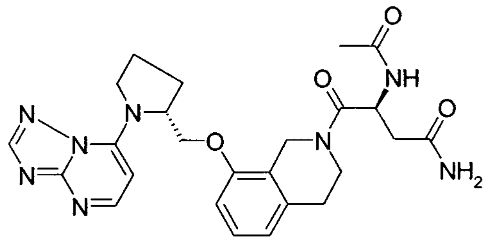 (S)-3-Acetylamino-4-oxo-4-[8-((R)-1-[1,2,4]triazolo[1,5-a]pyrimidin-7-yl-pyrrolidin-2-ylmethoxy)-3,4-dihydro-1H-isochinolin-2-yl]-butyramid Formiat | | 1.583 Min [507.2] |
| "A33" | 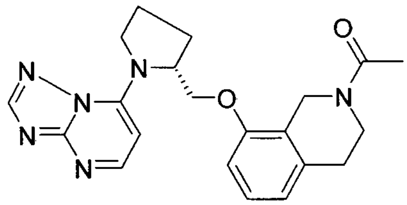 1-[8-((R)-1-[1,2,4]Triazolo[1,5-a]pyrimidin-7-yl-pyrrolidin-2-ylmethoxy)-3,4-dihydro-1H-isochinolin-2-yl]-ethanon | | 1.870 min [393.2] |
| "A34" | 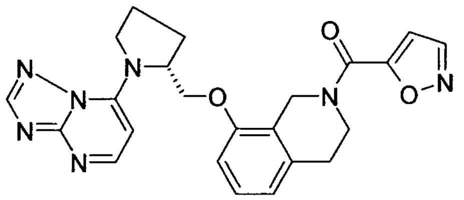 Isoxazol-5-yl-[8-((R)-1-[1,2,4]triazolo[1,5-a]pyrimidin-7-yl-pyrrolidin-2-ylmethoxy)-3,4-dihydro-1H-isochinolin-2-yl]-methanon | | 1.982 min [446.2] |
| $^1$H NMR (500 MHz, $d_6$-DMSO) δ [ppm] 8.81 (d, 1H, J = 31 Hz), 8.39 (d, 1H, J = 9.6 Hz), 8.29 (dd, 1H, J = 5.7 Hz, J = 15.0 Hz), 7.12 (m, 1H), 7.00 (d, 1H, J = 5.7 Hz), 6.87 (d, 1H, J = 8.1 Hz), 6.77 (d, 1H, J = 7.7 Hz) | | | |
| "A35" | 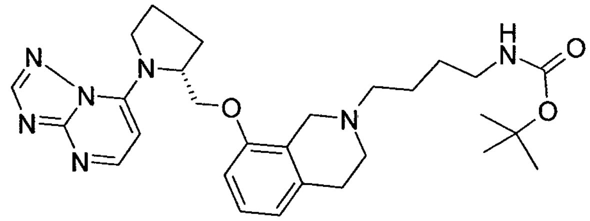 {4-[8-((R)-1-[1,2,4]Triazolo[1,5-a]pyrimidin-7-yl-pyrrolidin-2-ylmethoxy)-3,4-dihydro-1H-isochinolin-2-yl]-butyl} -carbaminsäure-tert.-butylester | | 1.665 min [522.3] |
| $^1$H NMR (500 MHz, $d_6$-DMSO) δ [ppm] 8.40 (s, 1H), 8.30 (d, 1H, J = 5.7 Hz), 7.01 (dd, 1H, J = 7.7 Hz, J = 8.2 Hz), 6.82 (br. m, 1H), 6.70 (d, 1H, J = 8.2 Hz), 6.65 (d, 1H, J = 7.7 Hz), 6.39 (d, 1H, J = 5.7 Hz), 5.62 (br. m, 1H), 4.13 (dd, 1H, J = 5.4 Hz, J = 9.8 Hz), 4.02 (dd, 1H, J = 5.6 Hz, J = 9.8 Hz), 3.94 (br. m, 1H), 3.79 (br. m, 1H), 3.04 (m, 2H), 2.95 (m, 2H), 2.69 (m, 2H), 2.34 (m, 2H), 2.20 (m, 2H), 2.10 (m, 1H), 2.06 (m, 1H), 1.37 (s, 9H) | | | |

(fortgesetzt)

| Verbindung Nr. | Struktur und/oder Name | F. [°C] | HPLC-MS; rt; [M+H+]$ |
|---|---|---|---|
| "A36" | 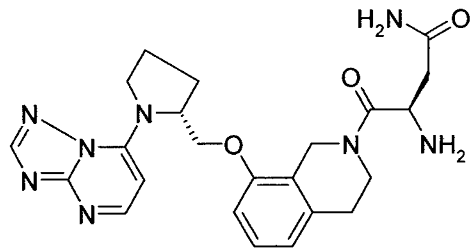 (R)-3-Amino-4-oxo-4-[8-((R)-1-[1,2,4]triazolo[1,5-a]pyrimidin-7-yl-pyrrolidin-2-ylmethoxy)-3,4-dihydro-1H-isochuinolin-2-yl]-butyramid | | 1.389 min [465.2] |
| "A37" | 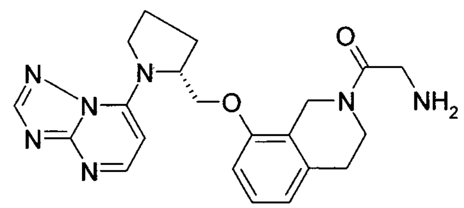 2-Amino-1-[8-((R)-1-[1,2,4]triazolo[1,5-a]pyrimidin-7-yl-pyrrolidin-2-ylmethoxy)-3,4-dihydro-1H-isochinolin-2-yl]-ethanon | | 1.419 min [408.2] |
| "A38" | 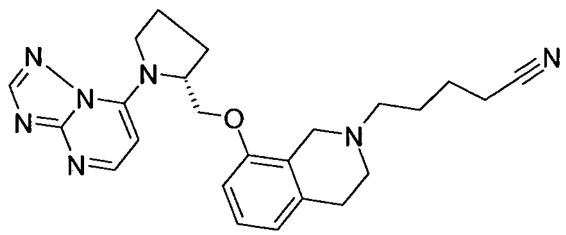 5-[8-((R)-1-[1,2,4]Triazolo[1,5-a]pyrimidin-7-yl-pyrrolidin-2-ylmethoxy)-3,4-dihydro-1H-isochinolin-2-yl]-pentannitril | | 1.422 min [432.25] |
| $^1$H NMR (500 MHz, $d_6$-DMSO) δ [ppm] 8.41 (s, 1H), 8.31 (d, 1H, J = 5.5 Hz), 7.01 (dd, 1H, J = 7.6 Hz J = 8.0 Hz), 6.70 (d, 1H, J = 8.0 Hz), 6.65 (d, 1H, J = 7.6 Hz), 6.39 (d, 1H, J = 5.5 Hz), 5.67 (br. m, 1H), 4.11 (dd, 1H, J = 5.9 Hz, J = 9.8 Hz), 4.03 (dd, 1H, J = 5.6 Hz, J = 9.8 Hz), 3.94 (br. m, 1H), 3.77 (br. m, 1H), 2.98 (m, 2H), 2.69 (m, 2H), 2.56 (m, 2H), 2.36 (m, 2H), 2.20 (m, 2H), 2.07 (m, 2H), 1.61 (m, 2H), 1.53 (m, 2H) | | | |
| "A39" | 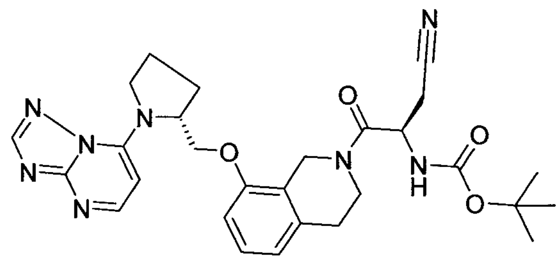 {(R)-2-Cyan-1-[8-((R)-1-[1,2,4]triazolo[1,5-a]pyrimidin-7-yl-pyrrolidin-2-ylmethoxy)-3,4-dihydro-1H-isochinolin-2-carbonyl]-ethyl}-carbaminsäure-tert.-butylester | | 2.177 min [547.3] |

(fortgesetzt)

| Verbindung Nr. | Struktur und/oder Name | F. [°C] | HPLC-MS; rt; [M+H+]$ |
|---|---|---|---|
| "A40" | 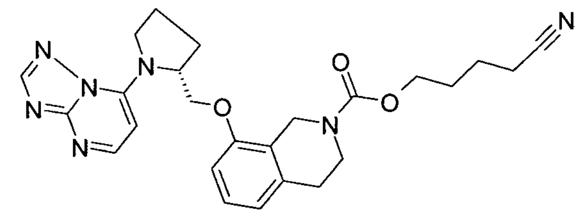 8-((R)-1-[1,2,4]Triazolo[1,5-a]pyrimidin-7-yl-pyrrolidin-2-ylmethoxy)-3,4-dihydro-1H-isochinolin-2-carbonsäure-4-cyan-butyl ester | | 2.138 min [476.2] |
| $^1H$ NMR (500 MHz, $d_6$-DMSO) δ [ppm] 8.39 (s, 1H), 8.23 (d, 1H, J = 5.7 Hz), 7.08 (dd, 1H, J = 7.6 Hz, J =8.0 Hz), 6.80 (d, 1H, J = 8.0), 6.73 (d, 1H, J = 7.6 Hz), 6.38 (d, 1H, J = 5.7 Hz), 5.53 (br. m, 1H), 4.31 (m, 2H), 4.25 (m, 1H), 4.08 (m, 3H), 3.96 (m, 1H), 3.80 (m, 1H), 3.54 (m, 2H), 2.72 (m, 2H), 2.55 (m, 2H), 2.23 (m, 2H), 2.16 (m, 1H), 2.08 (m, 1H), 1.68 (m, 4H) | | | |
| "A41" | 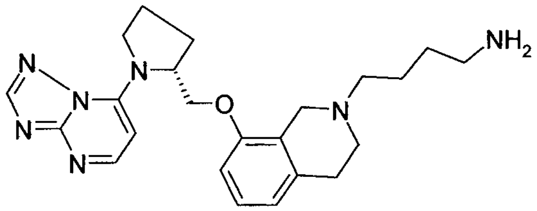 4-[8-((R)-1-[1,2,4]Triazolo[1,5-a]pyrimidin-7-yl-pyrrolidin-2-ylmethoxy)-3,4-dihydro-1H-isochinolin-2-yl]-butylamin | | 1.123 min [422.25] |
| "A42" | 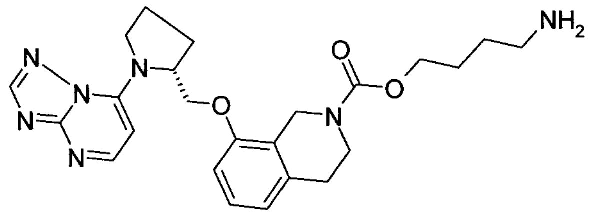 8-((R)-1-[1,2,4]Triazolo[1,5-a]pyrimidin-7-yl-pyrrolidin-2-ylmethoxy)-3,4-dihydro-1H-isochinolin-2-carbonsäure-4-amino-butylester | | 1.604 min [466.2] |
| "A43" | 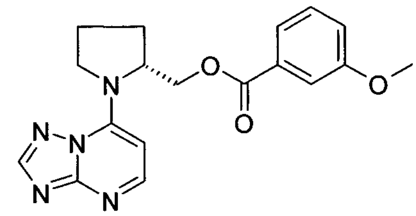 3-Methoxy-benzoesäure-(R)-1-[1,2,4]triazolo[1,5-a]pyrimidin-7-yl-pyrrolidin-2-yl-methylester | | 1.966 min [354.2] |
| $^1H$ NMR (500 MHz, $d_6$-DMSO) δ [ppm] 8.41 (s, 1H), 8.36 (d, J = 5.6, 1H), 7.42 (t, J = 7.9, 1H), 7.32 (d, J = 7.7, 1H), 7.29 - 7.27 (m, 1H), 7.25 (ddd, J = 8.2, 2.7, 0.8, 1H), 6.47 (d, J = 5.6, 1H), 5.62 (m, 1H), 4.55 (dd, J = 11.3, 5.6, 1H), 4.47 (dd, J = 11.3, 5.5, 1H), 3.99 (m, 1H), 3.83 (d, J = 10.1, 5H), 3.35 (d, J = 11.9, 4H), 2.35 - 2.18 (m, 2H), 2.18 - 2.04 (m, 2H) | | | |

(fortgesetzt)

| Verbindung Nr. | Struktur und/oder Name | F. [°C] | HPLC-MS; rt; $[M+H^+]^\$$ |
|---|---|---|---|
| "A44" | 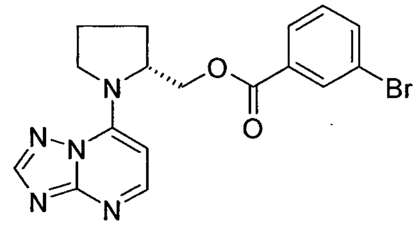 3-Brom-benzoesäure-(R)-1-[1,2,4]triazolo[1,5-a]pyrimidin-7-yl-pyrrolidin-2-yl-methylester | | 2.156 min [402.2 + 404.2] |
| $^1$H NMR (500 MHz, $d_6$-DMSO) δ [ppm] 8.34 (s, 1H), 8.31 (d, J = 5.6, 1H), 7.87 - 7.81 (d, J = 8, 1H), 7.79 (s, 1H), 7.67 (d, J = 7.8, 1H), 7.42 (t, J = 7.9, 1H), 6.42 (d, J = 5.6, 1H), 5.58 (br. m, 1H), 4.51 (dd, J = 11.3, 5.7, 1H), 4.42 (dd, J = 11.3, 5.8, 1H), 3.95 (br. m, 1H), 3.77 (br. m, 1H), 2.32 - 2.13 (m, 2H), 2.13 - 1.98 (m, 2H) | | | |
| "A45" | 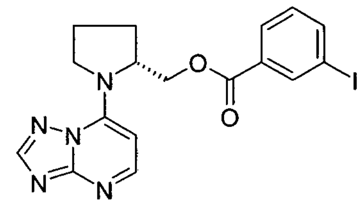 3-Iod-benzoesäure-(R)-1-[1,2,4]triazolo[1,5-a]pyrimidin-7-yl-pyrrolidin-2-yl-methylester | | 2.217 min [450.0] |
| $^1$H NMR (500 MHz, $d_6$-DMSO) δ [ppm] 8.36 (d, J = 5.6, 1H), 8.07 - 8.00 (m, 2H), 7.73 (d, J = 7.8, 1H), 7.31 (t, J = 7.9, 1H), 6.47 (d, J = 5.7, 1H), 5.61 (s, 1H), 4.55 (dd, J = 11.3, 5.7, 1H), 4.46 (dd, J = 11.3, 5.8, 1H), 4.00 (s, 1H), 3.82 (t, J = 8.9, 1H), 2.35 - 2.16 (m, 2H), 2.16 - 2.04 (m, 2H) | | | |
| "A46" | 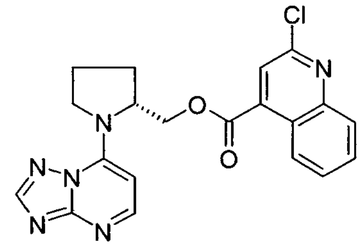 2-Chlor-chinolin-4-carbonsäure-(R)-1-[1,2,4]triazolo[1,5-a]pyrimidin-7-yl-pyrrolidin-2-yl-methylester | | 2.123 min [409.2] |
| $^1$H NMR (500 MHz, $d_6$-DMSO) δ [ppm] 8.49 (d, J = 8.1, 1H), 8.32 (s, 1H), 8.30 (d, J = 5.6, 1H), 8.05 (d, J = 8.2, 1H), 7.94 - 7.88 (m, 1H), 7.75 (ddd, J = 8.3, 7.0, 1.2, 1H), 7.65 (s, 1H), 6.46 (d, J = 5.7, 1H), 5.57 (s, 1H), 4.66 (dd, J = 11.3, 5.8, 1H), 4.55 (dd, J = 11.3, 5.5, 1H), 4.00 (d, J = 12.4, 1H), 3.78 (dd, J = 17.1, 7.3, 1H), 2.31 - 2.20 (m, 1H), 2.19 - 2.10 (m, 2H), 2.10 - 1.99 (m, 1H) | | | |
| "A50" | 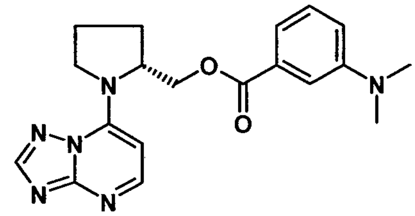 3-Dimethylamino-benzoesäure-(R)-1-[1,2,4]triazolo[1,5-a]pyrimidin-7-yl-pyrrolidin-2-yl-methylester | | 1.929 min [367.2] |

(fortgesetzt)

| Verbindung Nr. | Struktur und/oder Name | F. [°C] | HPLC-MS; rt; [M+H+]$ |
|---|---|---|---|
| "A52" | 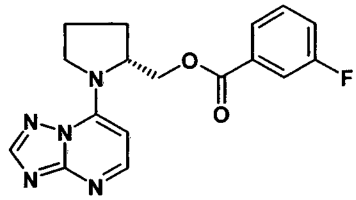 3-Fluor-benzoesäure-(R)-1-[1,2,4]triazolo[1,5-a]pyrimidin-7-yl-pyrrolidin-2-yl-methylester | | 1.968 min [342.2] |
| "A53" | 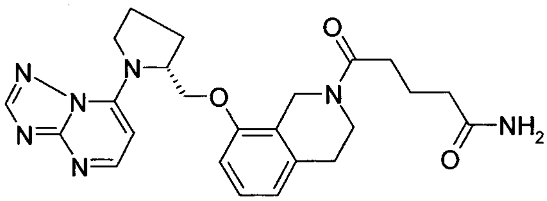 5-Oxo-5-[8-((R)-1-[1,2,4]triazolo[1,5-a]pyrimidin-7-yl-pyrrolidin-2-ylmethoxy)-3,4-dihydro-1H-isochinolin-2-yl]-pentansäure-amid | | 1.709 min [464.3] |
| "A59" | 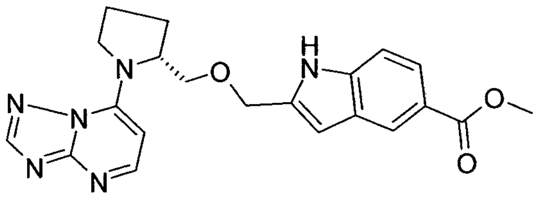 2-((R)-1-[1,2,4]Triazolo[1,5-a]pyrimidin-7-yl-pyrrolidin-2-ylmethoxymethyl)-1H-indol-5-carbonsäure-methylester | | 1.930 min [407.2] |
| "A61" | 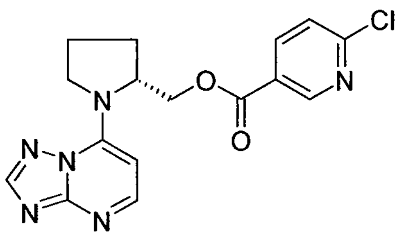 6-Chlor-nicotinsäure-(R)-1-[1,2,4]triazolo[1,5-a]pyrimidin-7-yl-pyrrolidin-2-yl-methylester | | 1.794 min [359.0] |
| $^1$H NMR (500 MHz, $d_6$-DMSO) δ [ppm] 8.62 (d, J = 2.1, 1H), 8.34 (s, 1H), 8.30 (d, J = 5.7, 1H), 8.05 (dd, J = 8.3, 2.4, 1H), 7.62 (dd, J = 8.3, 0.6, 1H), 6.41 (d, J = 5.7, 1H), 5.53 (br. m, 1H), 4.54 (dd, J = 11.3, 5.6, 1H), 4.44 (dd, J = 11.3, 5.7, 1H), 3.94 (br. m, 1H), 3.74 (br. m, 1H), 2.27 - 1.99 (m, 4H) | | | |
| "A63" | 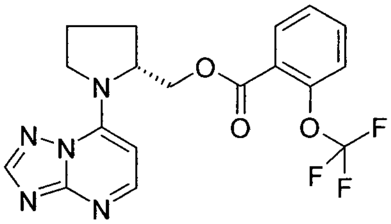 2-Trifluormethoxy-benzoesäure-(R)-1-[1,2,4]triazolo[1,5-a]pyrimidin-7-yl-pyrrolidin-2-yl-methylester | | 2.055 min [328.2] |

(fortgesetzt)

| Verbindung Nr. | Struktur und/oder Name | F. [°C] | HPLC-MS; rt; [M+H+]$ |
|---|---|---|---|
| $^1$H NMR (500 MHz, $d_6$-DMSO) δ [ppm] 8.35 (s, 1H), 8.30 (d, J = 5.6, 1H), 7.79 - 7.65 (m, 2H), 7.53 - 7.43 (m, 2H), 6.38 (d, J = 5.7, 1H), 5.49 (br. m, 1H), 4.52 - 4.39 (m, 2H), 3.92 (br. m, 1H), 3.82 - 3.63 (m, 1H), 2.26 - 1.96 (m, 4H) | | | |
| "A64" | 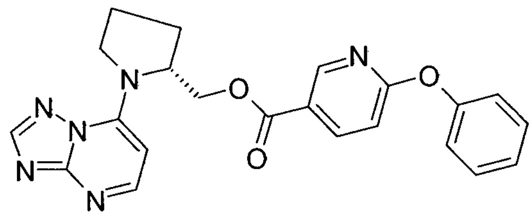 6-Phenoxy-nicotinsäure-(R)-1-[1,2,4]triazolo[1,5-a]pyrimidin-7-yl-pyrrolidin-2-yl-methylester | | 2.154 min [417.2] |
| $^1$H NMR (500 MHz, $d_6$-DMSO) δ [ppm] 8.48 (d, J = 1.8, 1H), 8.34 (s, 1H), 8.29 (d, J = 5.6, 1H), 8.05 (dd, J = 8.6, 2.1, 1H), 7.46 (m, 2H), 7.26 (m, 1H), 7.19 (d, J = 7.9, 2H), 7.05 (d, J = 8.6, 1H), 6.40 (d, J = 5.6, 1H), 5.52 (br. m, 1H), 4.49 (dd, J = 11.3, 5.5, 1H), 4.42 (dd, J = 11.3, 5.3, 1H), 3.89 (br. m, 1H), 3.74 (br. m, 1H), 2.23 - 2.02 (m, 4H) | | | |
| "A68" | 2-Iod-benzoesäure-(R)-1-[1,2,4]triazolo[1,5-a]pyrimidin-7-yl-pyrrolidin-2-yl-methylester | | 2.124 min [450.0] |
| $^1$H NMR (500 MHz, $d_6$-DMSO) δ [ppm] 8.40 (s, 1H), 8.32 (d, J = 5.6, 1H), 8.00 (dd, J = 7.9, 0.7, 1H), 7.54 - 7.39 (m, 2H), 7.27 (td, J = 7.7, 1.9, 1H), 6.41 (d, J = 5.7, 1H), 5.49 (br. m, 1H), 4.47 (dd, J = 10.0, 4.7, 1H), 4.44 (dd, J = 10.0, 3.8, 1H), 3.97 (br. m, 1H), 3.83 - 3.68 (m, 1H), 2.29 - 2.10 (m, 3H), 2.09 - 1.99 (m, 1H) | | | |
| "A69" | Pyridin-2-carbonsäure-(R)-1-[1,2,4]triazolo[1,5-a]pyrimidin-7-yl-pyrrolidin-2-yl-methylester | | 1.443 min [325.2] |
| $^1$H NMR (500 MHz, $d_6$-DMSO) δ [ppm] 8.62 (d, J = 4.6, 1H), 8.31 (s, 1H), 8.28 (d, J = 5.6, 1H), 7.92 (td, J = 7.7, 1.6, 1H), 7.81 (d, J = 7.8, 1H), 7.60 (dd, J = 7.1, 5.2, 1H), 6.38 (d, J = 5.6, 1H), 5.60 (br. m, 1H), 4.55 (dd, J = 11.3, 5.7, 1H), 4.42 (dd, J = 11.3, 5.4, 1H), 3.91 (br. m, 1H), 3.80 - 3.65 (m, 1H), 2.32 - 2.15 (m, 2H), 2.15 - 1.98 (m, 2H) | | | |
| "A70" | 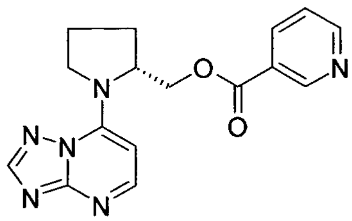 Nicotinsäure-(R)-1-[1,2,4]triazolo[1,5-a]pyrimidin-7-yl-pyrrolidin-2-yl-methylester | | 1.489 min [325.2] |
| $^1$H NMR (500 MHz, $d_6$-DMSO) δ [ppm] 8.85 (d, J = 1.7, 1H), 8.79 (dd, J = 4.8, 1.6, 1H), 8.35 (s, 1H), 8.31 (d, J = 5.6, 1H), 8.02 (dt, J = 7.9, 1.8, 1H), 7.50 (dd, J = 7.9, 4.9, 1H), 6.42 (d, J = 5.7, 1H), 5.58 (br. m, 1H), 4.53 (dd, J = 11.3, 5.7, 1H), 4.46 (dd, J = 11.3, 5.5, 1H), 3.96 (br. m, 1H), 3.76 (br. m, 1H), 2.30 - 1.97 (m, 4H) | | | |
| "A71" | Benzoesäure-(R)-1-[1,2,4]triazolo[1,5-a]pyrimidin-7-yl-pyrrolidin-2-yl-methylester | | 1.917 min [324.2] |
| "A72" | 3-Ethoxy-benzoesäure-(R)-1-[1,2,4]triazolo[1,5-a]pyrimidin-7-yl-pyrrolidin-2-yl-methylester | | 2.111 min [368.2] |
| $^1$H NMR (500 MHz, $d_6$-DMSO) δ [ppm] 8.36 (s, 1H), 8.31 (d, J = 5.6, 1H), 7.35 (t, J = 7.9, 1H), 7.25 (d, J = 7.7, 1H), 7.24 - 7.14 (m, 2H), 6.42 (d, J = 5.7, 1H), 5.56 (br. m, 1H), 4.49 (dd, J = 11.3, 5.7, 1H), 4.41 (dd, J = 11.3, 5.5, 1H), 4.02 (q, J = 7.0, 3H), 3.95 (br. m, 1H), 3.83 - 3.71 (m, 1H), 2.29 - 2.13 (m, 2H), 2.07 (m, 2H), 1.34 (t, J = 7.0, 4H) | | | |
| "A73" | 5-Chlor-2-fluor-benzoesäure-(R)-1-[1,2,4]triazolo[1,5-a]pyrimidin-7-yl-pyrrolidin-2-yl-methylester | | 2.086 min [376.0] |

(fortgesetzt)

| Verbindung Nr. | Struktur und/oder Name | F. [°C] | HPLC-MS; rt; [M+H$^+$]$^\$$ |
|---|---|---|---|
| $^1$H NMR (500 MHz, $d_6$-DMSO) δ [ppm] 8.34 (s, 1H), 8.31 (d, J = 5.6, 1H), 7.78 - 7.70 (m, 1H), 7.61 (dd, J = 6.1, 2.7, 1H), 7.43 - 7.30 (m, 1H), 6.41 (d, J = 5.7, 1H), 5.54 (br. m, 1H), 4.52 (dd, J = 11.3, 5.7, 1H), 4.42 (dd, J = 11.3, 5.5, 1H), 3.95 (br. m, 1H), 3.84 - 3.65 (m, 1H), 2.26 - 2.13 (m, 2H), 2.13 - 1.97 (m, 2H) | | | |
| "A74" | 3-Chlormethyl-benzoesäure-(R)-1-[1,2,4]triazolo[1,5-a]pyrimidin-7-yl-pyrrolidin-2-yl-methylester | | 2.059 min [372.0] |
| "A75" | 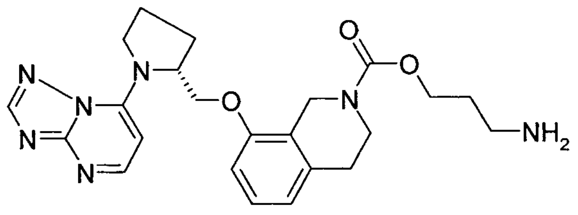 8-((R)-1-[1,2,4]Triazolo[1,5-a]pyrimidin-7-yl-pyrrolidin-2-ylmethoxy)-3,4-dihydro-1H-isochinolin-2-carbonsäure-3-amino-propyl ester | | 1.570 min [452.2] |
| "A76" | 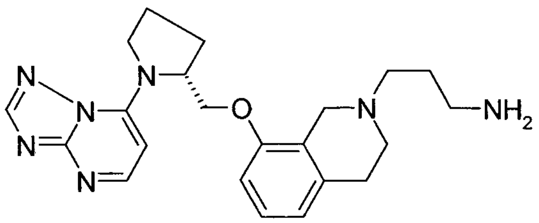 3-[8-((R)-1-[1,2,4]Triazolo[1,5-a]pyrimidin-7-yl-pyrrolidin-2-ylmethoxy)-3,4-dihydro-1H-isochinolin-2-yl]-propylamin | | 1.181 min [408.3] |
| "A77" | 7-[(R)-2-(Naphthalin-1-yloxymethyl)-pyrrolidin-1-yl]-[1,2,4]triazolo[1,5-a][1,3,5]triazin | | 2.450 min [347.1] |
| $^1$H NMR (500 MHz, $d_6$-DMSO) δ [ppm] 8.51 (s, 1H), 8.38 (s, 1H), 7.70 (d, J = 7.9, 1H), 7.46 - 7.31 (m, 4H), 7.26 (m, 1H), 6.85 (m, 1H), 5.80 (br. m, 1H), 5.00 (br. m, 1H), 4.50 (m, 1H) 4.41 (dd, J = 9.6, 3.4, 1H), 4.35 (br. m 1H), 2.25 (m, 3H), 2.07 (m, 1H) | | | |
| "A78" | 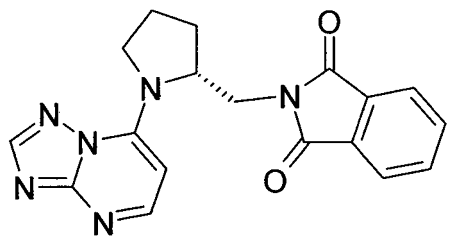 2-((R)-1-[1,2,4]Triazolo[1,5-a]pyrimidin-7-yl-pyrrolidin-2-ylmethyl)-isoindol-1,3-dion | | 1.697 min [349.2] |
| "A79" | 3-Cyan-benzoesäure-(R)-1-[1,2,4]triazolo[1,5-a]pyrimidin-7-yl-pyrrolidin-2-yl-methylester | | 1.829 min [349.2] |
| "A81" | 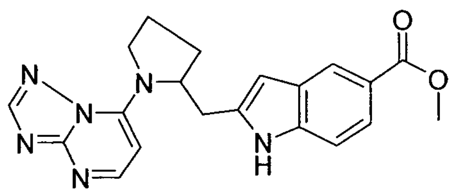 | | 2.023 min [377.2] |

(fortgesetzt)

| Verbindung Nr. | Struktur und/oder Name | F. [°C] | HPLC-MS; rt; [M+H+]$ |
|---|---|---|---|
| | 2-(1-[1,2,4]Triazolo[1,5-a]pyrimidin-7-yl-pyrrolidin-2-ylmethyl)-1H-indol-5-carbonsäuremethylester | | |
| $^1$H NMR (500 MHz, $d_6$-DMSO) δ [ppm] 11.42 (s, 1H), 8.44 (s, 1H), 8.30 (d, J = 5.7, 1H), 8.14 (d, J = 15.1, 1H), 7.68 (dd, J = 8.5, 1.5, 1H), 7.41 (d, J = 8.5, 1H), 6.49 - 6.33 (m, 2H), 5.24 (br. m, 1H), 4.01 (br. m, 1H), 3.83 (s, 3H), 3.23 (dd, J = 14.4, 3.2, 1H), 3.06 (dd, J = 14.4, 7.2, 2H), 2.93 (dd, J = 14.4, 9.7, 1H), 2.07 - 1.81 (m, 4H), 1.25 - 1.11 (m, 2H) | | | |
| "A82" | 3-Trifluormethoxy-benzoesäure-(R)-1-[1,2,4]triazolo[1,5-a]pyrimidin-7-yl-pyrrolidin-2-yl-methylester | | 2.251 min [408.2] |
| $^1$H NMR (500 MHz, $d_6$-DMSO) δ [ppm] 8.33 (s, 1H), 8.29 (d, J = 5.6, 1H), 7.69 (br. d, J = 7.6, 1H), 7.65 (br. d, J = 8.2, 1H), 7.61 (d, J = 7.8, 1H), 7.58 (m, 1H), 6.41 (d, J = 5.7, 1H), 5.58 (s, 1H), 4.51 (dd, J = 11.3, 5.7, 1H), 4.44 (dd, J = 11.3, 5.7, 1H), 3.95 (br. m, 1H), 3.75 (br. m, 1H), 2.27 - 2.00 (m, 4H) | | | |
| "A83" | 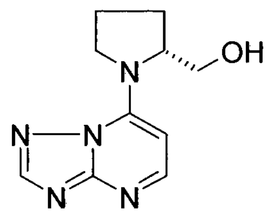 ((R)-1-[1,2,4]Triazolo[1,5-a]pyrimidin-7-yl-pyrrolidin-2-yl)-methanol | 168-170 | 1.094 min [220.1] |
| $^1$H NMR (500 MHz, $d_6$-DMSO) δ [ppm] 8.37 (s, 1H), 8.27 (t, J = 6.6, 1H), 6.30 (d, J = 5.7, 1H), 4.94 (s, 1H), 4.87 (t, J = 5.7, 1H), 3.96 (s, 1H), 3.79 (d, J = 6.3, 1H), 3.57 (ddd, J = 10.8, 5.4, 3.9, 1H), 3.45 (dt, J = 11.0, 6.3, 1H), 2.15 - 2.01 (m, 3H), 2.00 - 1.91 (m, 1H) | | | |
| "A93" | 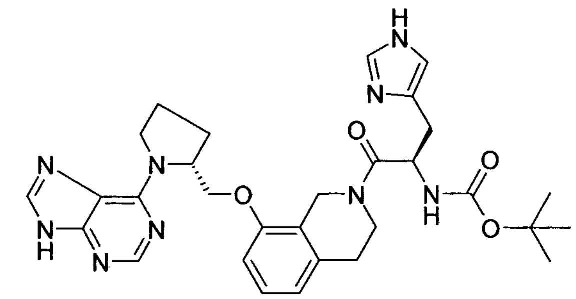 ((R)-1-(1H-Imidazol-4-ylmethyl)-2-oxo-2-{8-[(R)-1-(9H-purin-6-yl)-pyrrolidin-2-ylmethoxy]-3,4-dihydro-1H-isochinolin-2-yl}-ethyl)-carbaminsäure-tert.-butyl ester | | 1.587 min [588.3] |

Tabelle 1

| Inhibierung der MetAP-2 | | | |
|---|---|---|---|
| $IC_{50}$ von erfindungsgemäßen Verbindungen der Formel I | | | |
| Verbindung Nr. | $IC_{50}$ Enzym | Verbindung Nr. | $IC_{50}$ Enzym |
| "A5" | A | "A16" | A |
| "A8" | A | "A17" | A |
| "A9" | A | "A18" | A |
| "A10" | A | "A19" | A |
| "A11" | A | "A22" | A |

(fortgesetzt)

| Inhibierung der MetAP-2 | | | |
|---|---|---|---|
| $IC_{50}$ von erfindungsgemäßen Verbindungen der Formel I | | | |
| Verbindung Nr. | $IC_{50}$ Enzym | Verbindung Nr. | $IC_{50}$ Enzym |
| "A12" | A | "A23" | A |
| "A13" | A | "A24" | A |
| "A31" | A | "A25" | B |
| "A32" | A | "A26" | A |
| "A33" | A | "A27" | A |
| "A34" | A | "A28" | B |
| "A35" | A | "A29" | A |
| "A36" | A | "A30" | |
| "A37" | A | "A46" | A |
| "A38" | A | "A50" | A |
| "A39" | A | "A51" | A |
| "A40" | A | "A52" | A |
| "A41" | A | "A53" | A |
| "A42" | A | "A71" | A |
| "A43" | A | "A72" | A |
| "A44" | A | "A73" | A |
| "A45" | A | "A74" | A |
| "A61" | A | "A75" | A |
| "A63" | A | "A76" | A |
| "A64" | A | "A77" | B |
| "A68" | A | "A78" | B |
| "A69" | A | "A79" | A |
| "A70" | A | | |
| "A81" | A | | |
| "A82" | A | | |
| "A83" | A | | |
| $IC_{50}$: 10 nM - 1 µM = A<br>1 µM - 10 µM = B<br>> 10 µM = C | | | |

Die nachfolgenden Beispiele betreffen Arzneimittel:

**Beispiel A: Injektionsgläser**

Eine Lösung von 100 g eines Wirkstoffes der Formel I und 5 g Dinatriumhydrogenphosphat wird in 3 l zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \cdot 2\ H_2O$, 28,48 g $Na_2HPO_4 \cdot 12\ H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

**Patentansprüche**

1. Verbindungen der Formel I

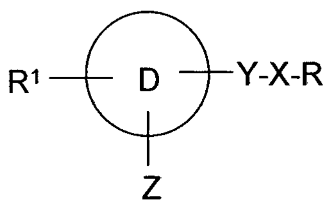

I

worin

Ⓓ Pyrrolidinyl, Imidazolinyl, Pyrazolyl, 2-Oxa-5-aza-bicyclo[2.2.1]hept-5-yl, Morpholinyl oder N,
Z Imidazopyrimidinyl, Triazolopyrimidinyl oder de Pyrrolopyrimidinyl, wobei Z an einen Stickstoff des Restes D gebunden ist,
Y $(CH_2)_n$ oder fehlt,
X $O(CH_2)_r$, NH, NA, OC(=O), $NHSO_2$ oder fehlt,
$R^1$ H, A oder COA,
R Het,
Het einen unsubstituierten oder ein-, zwei- oder dreifach durch Hal, A, $(CH_2)_mOR^6$, $(CH_2)_mN(R^6)_2$, $(CH_2)_mCN$,

$(CH_2)_mCOOR^6$, $CONH(CH_2)_mCOOH$, $CONH(CH_2)_mHet^2$, $CO(CH_2)_mN(R^6)_2$, $CO(CH_2)_mNH(CH_2)_rCOOA$, $(CH_2)_rCONH(CH_2)_mAr^1$, $COCH[(CH_2)_mCONH_2]NH_2$, $COCH[(CH_2)_mCONH_2]NHCOOA$, $COCH[(CH_2)_mHet^2]NHCOOA$, $COCH(NHCOA)CH_2CONH_2$, $(CH_2)_mNHCOOA$, $COCH[(CH_2)_mCN]NHCOOA$, $COO(CH_2)_mCN$, $COO(CH_2)_mN(R^6)_2$, $CO(CH_2)_mCON(R^6)_2$, $COR^6$, $COHet^2$, $O(CH_2)_mAr^1$ und/oder =O (Carbonylsauerstoff) substituierten zweikernigen gesättigten, ungesättigten oder aromatischen Heterocyclus mit 1 bis 4 N-, und/oder O- und/oder S-Atomen,

$Het^2$ Imidazolyl, Indolyl, Isoxazolyl, Pyridazinyl, Pyrazolyl, Benzimidazolyl, Pyridyl, Dibenzofuranyl, Carbazolyl, Benzofuranyl, Isoindolyl, Chinazolinyl, Chinoxalinyl, Pyrimidinyl, Indazolyl, Furyl, Thienyl, Pyrrolyl, Oxazolyl, Thiazolyl, Triazolyl, Tetrazolyl, Thiadiazol, Benzothiazolyl, Imidazo[1,2-*a*]pyridinyl oder 1,3-Benzodioxolyl,

$R^6$ H oder Alkyl mit 1-6 C-Atomen,

$Ar^1$ unsubstituiertes oder ein-, zwei- oder dreifach durch Hal und/oder $SO_2NH_2$ substituiertes Phenyl,

A unverzweigtes oder verzweigtes Alkyl mit 1-10 C-Atomen, worin 1-7 H-Atome durch F, Cl, Br und/oder OH ersetzt sein können, oder cyclisches Alkyl mit 3-7 C-Atomen,

Hal F, Cl, Br oder I,

m 0, 1, 2, 3, 4, 5 oder 6,

n 1 oder 2,

q 0, 1, 2, 3 oder 4,

r 0, 1 oder 2

bedeuten,
sowie ihre pharmazeutisch verwendbaren Salze, Tautomere und Stereoisomere, einschließlich deren Mischungen in allen Verhältnissen.

**2.** Verbindungen nach Anspruch 1, ausgewählt aus der Gruppe

| Verbindung Nr. | Name und/oder Struktur |
|---|---|
| "A5" | 5-((R)-1-[1,2,4]Triazolo[1,5-a]pyrimidin-7-yl-pyrrolidin-2-ylmethoxy)-chinolin |
| "A8" | 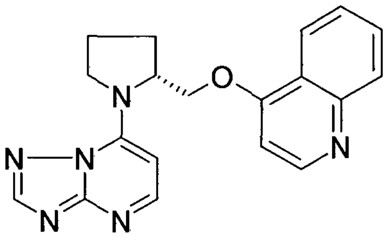 4-((R)-1-[1,2,4]Triazolo[1,5-a]pyrimidin-7-yl-pyrrolidin-2-ylmethoxy)-chinolin |
| "A9" | 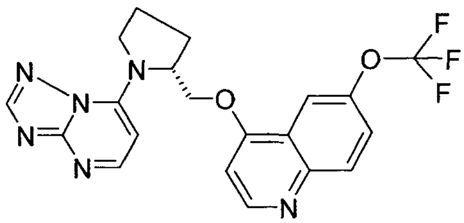 4-((R)-1-[1,2,4]Triazolo[1,5-a]pyrimidin-7-yl-pyrrolidin-2-ylmethoxy)-6-trifluormethoxy-chinolin |
| "A10" | 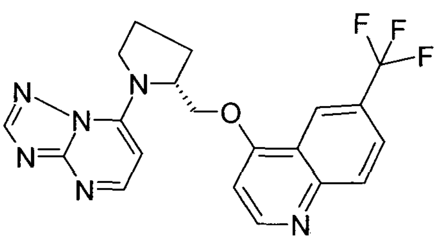 4-((R)-1-[1,2,4]Triazolo[1,5-a]pyrimidin-7-yl-pyrrolidin-2-ylmethoxy)-6-trifluormethyl-chinolin |

(fortgesetzt)

| Verbindung Nr. | Name und/oder Struktur |
|---|---|
| "A11" | 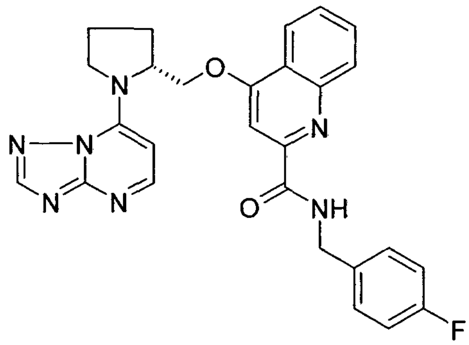<br>4-((R)-1-[1,2,4]Triazolo[1,5-a]pyrimidin-7-yl-pyrrolidin-2-ylmethoxy)-chinolin-2-carbonsäure-4-fluor-benzylamid |
| "A12" | 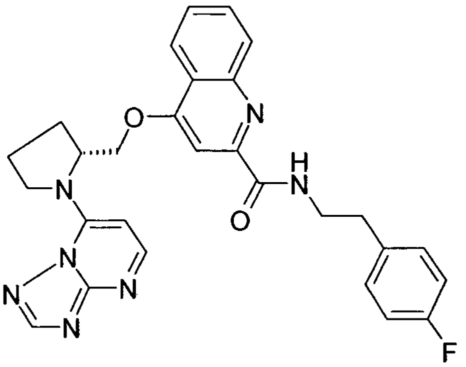<br>4-((R)-1-[1,2,4]Triazolo[1,5-a]pyrimidin-7-yl-pyrrolidin-2-ylmethoxy)-chinolin-2-carbonsäure-[2-(4-fluor-phenyl)-ethyl]-amid |
| "A13" | 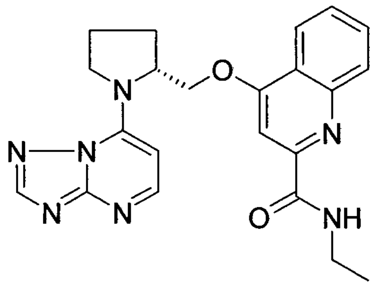<br>4-((R)-1-[1,2,4]Triazolo[1,5-a]pyrimidin-7-yl-pyrrolidin-2-ylmethoxy)-chinolin-2-carbonsäure-ethylamid |
| "A16" | 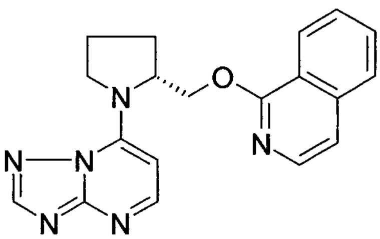<br>1-((R)-1-[1,2,4]Triazolo[1,5-a]pyrimidin-7-yl-pyrrolidin-2-ylmethoxy)-isochinolin |

(fortgesetzt)

| Verbindung Nr. | Name und/oder Struktur |
|---|---|
| "A17" | 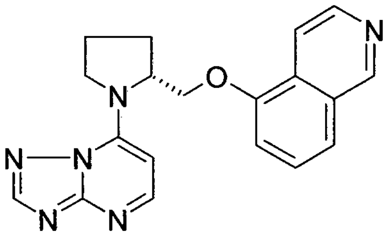 5-((R)-1-[1,2,4]Triazolo[1,5-a]pyrimidin-7-yl-pyrrolidin-2-ylmethoxy)-isochinolin |
| "A18" | 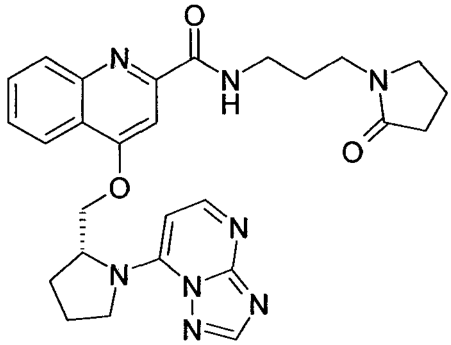 4-((R)-1-[1,2,4]Triazolo[1,5-a]pyrimidin-7-yl-pyrrolidin-2-ylmethoxy)-chinolin-2-carbonsäure-[3-(2-oxo-pyrrolidin-1-yl)-propyl]-amid |
| "A22" | 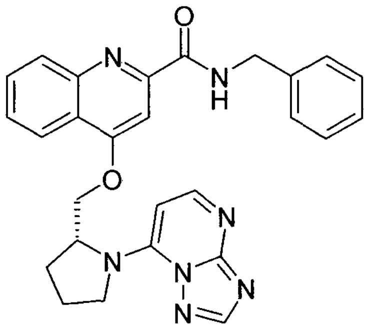 4-((R)-1-[1,2,4]Triazolo[1,5-a]pyrimidin-7-yl-pyrrolidin-2-ylmethoxy)-chinolin-2-carbonsäure-benzylamid |
| "A23" | 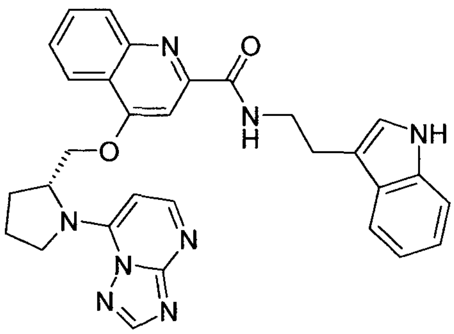 4-((R)-1-[1,2,4]Triazolo[1,5-a]pyrimidin-7-yl-pyrrolidin-2-ylmethoxy)-chinolin-2-carbonsäure-[2-(1H-indol-3-yl)-ethyl]-amid |

(fortgesetzt)

| Verbindung Nr. | Name und/oder Struktur |
|---|---|
| "A24" | 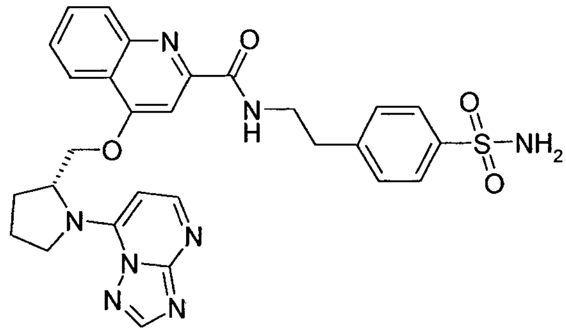 4-((R)-1-[1,2,4]Triazolo[1,5-a]pyrimidin-7-yl-pyrrolidin-2-ylmethoxy)-chinolin-2-carbonsäure-[2-(4-sulfamoyl-phenyl)-ethyl]-amid |
| "A25" | 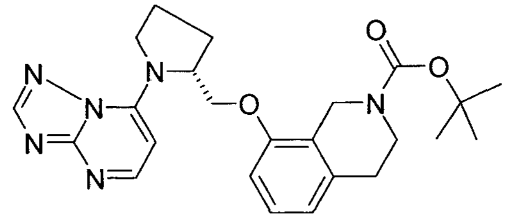 8-((R)-1-[1,2,4]Triazolo[1,5-a]pyrimidin-7-yl-pyrrolidin-2-ylmethoxy)-3,4-dihydro-1H-isochinolin-2-carbonsäure-tert.-butylester |
| "A27" | 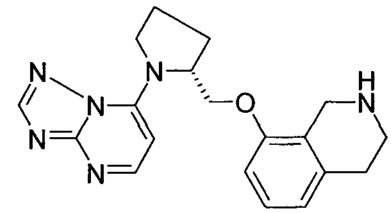 8-((R)-1-[1,2,4]Triazolo[1,5-a]pyrimidin-7-yl-pyrrolidin-2-ylmethoxy)-1,2,3,4-tetrahydro-isochinolin |
| "A28" | 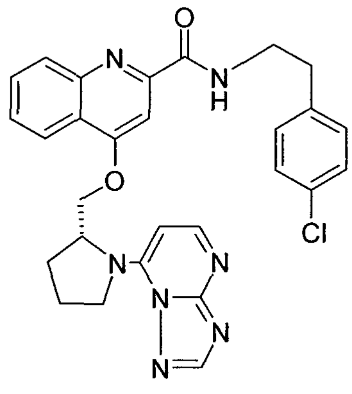 4-((R)-1-[1,2,4]Triazolo[1,5-a]pyrimidin-7-yl-pyrrolidin-2-ylmethoxy)-chinolin-2-carbonsäure-[2-(4-chlor-phenyl)-ethyl]-amid |

(fortgesetzt)

| Verbindung Nr. | Name und/oder Struktur |
|---|---|
| "A29" | 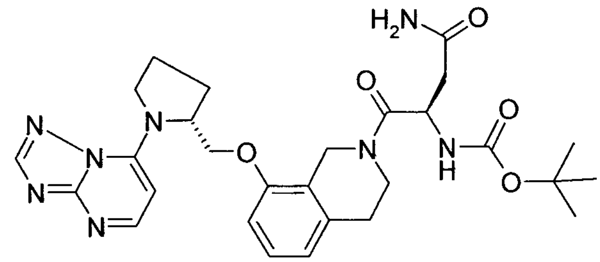<br>{(R)-1-Carbamoylmethyl-2-oxo-2-[8-((R)-1-[1,2,4]triazolo[1,5-a]pyrimidin-7-yl-pyrrolidin-2-ylmethoxy)-3,4-dihydro-1H-isochinolin-2-yl]-ethyl}-carbaminsäure-tert.-butylester |
| "A30" | 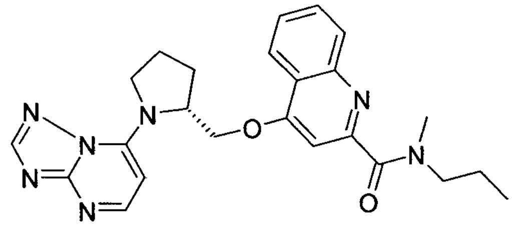<br>4-((R)-1-[1,2,4]Triazolo[1,5-a]pyrimidin-7-yl-pyrrolidin-2-ylmethoxy)-chinolin-2-carbonsäure-ethyl-propyl-amid |
| "A31" | 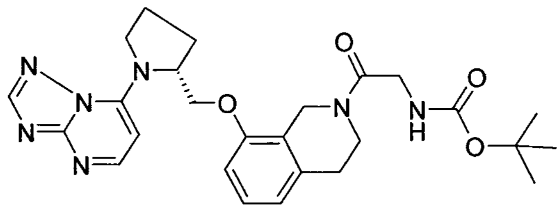<br>{2-Oxo-2-[8-((R)-1-[1,2,4]triazolo[1,5-a]pyrimidin-7-yl-pyrrolidin-2-ylmethoxy)-3,4-dihydro-1H-isochinolin-2-yl]-ethyl}-carbaminsäure-tert.-butylester |
| "A32" | 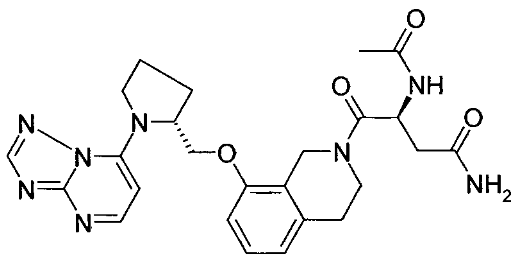<br>(S)-3-Acetylamino-4-oxo-4-[8-((R)-1-[1,2,4]triazolo[1,5-a]pyrimidin-7-yl-pyrrolidin-2-ylmethoxy)-3,4-dihydro-1H-isochinolin-2-yl]-butyramid |
| "A33" | 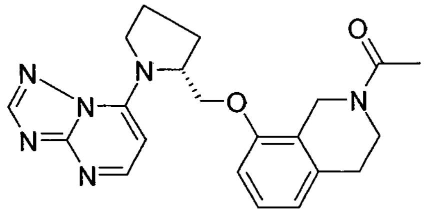<br>1-[8-((R)-1-[1,2,4]Triazolo[1,5-a]pyrimidin-7-yl-pyrrolidin-2-ylmethoxy)-3,4-dihydro-1H-isochinolin-2-yl]-ethanon |

(fortgesetzt)

| Verbindung Nr. | Name und/oder Struktur |
|---|---|
| "A34" | 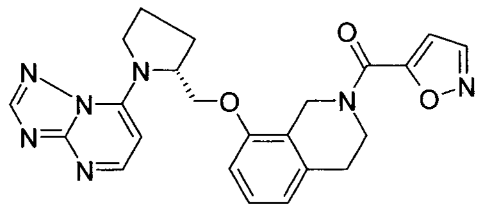<br>Isoxazol-5-yl-[8-((R)-1-[1,2,4]triazolo[1,5-a]pyrimidin-7-yl-pyrrolidin-2-ylmethoxy)-3,4-dihydro-1H-isochinolin-2-yl]-methanon |
| "A35" | 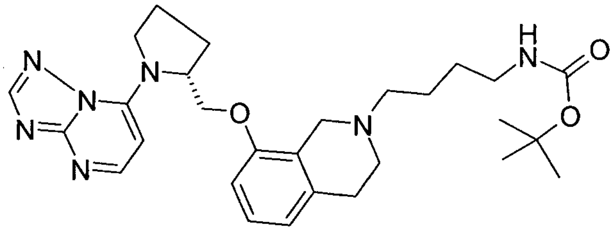<br>{4-[8-((R)-1-[1,2,4]Triazolo[1,5-a]pyrimidin-7-yl-pyrrolidin-2-ylmethoxy)-3,4-dihydro-1H-isochinolin-2-yl]-butyl}-carbaminsäure-tert.-butylester |
| "A36" | 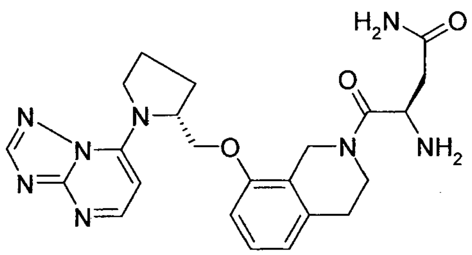<br>(R)-3-Amino-4-oxo-4-[8-((R)-1-[1,2,4]triazolo[1,5-a]pyrimidin-7-yl-pyrrolidin-2-ylmethoxy)-3,4-dihydro-1H-isochuinolin-2-yl]-butyramid |
| "A37" | 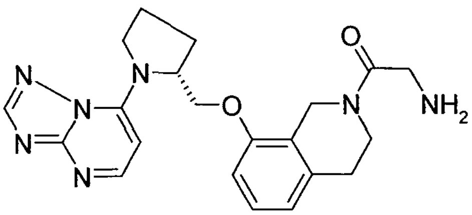<br>2-Amino-1-[8-((R)-1-[1,2,4]triazolo[1,5-a]pyrimidin-7-yl-pyrrolidin-2-ylmethoxy)-3,4-dihydro-1H-isochinolin-2-yl]-ethanon |
| "A38" | 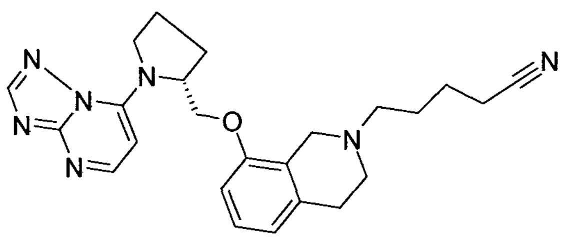<br>5-[8-((R)-1-[1,2,4]Triazolo[1,5-a]pyrimidin-7-yl-pyrrolidin-2-ylmethoxy)-3,4-dihydro-1H-isochinolin-2-yl]-pentannitril |

(fortgesetzt)

| Verbindung Nr. | Name und/oder Struktur |
|---|---|
| "A39" | 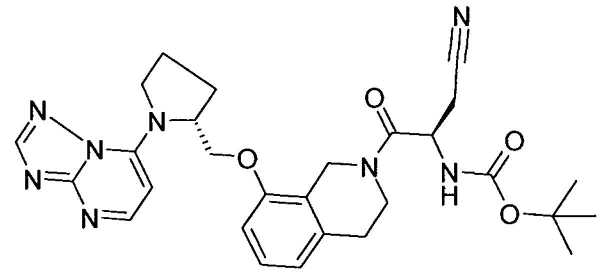<br>{(R)-2-Cyan-1-[8-((R)-1-[1,2,4]triazolo[1,5-a]pyrimidin-7-yl-pyrrolidin-2-ylmethoxy)-3,4-dihydro-1H-isochinolin-2-carbonyl]-ethyl}-carbaminsäure-tert.-butylester |
| "A40" | 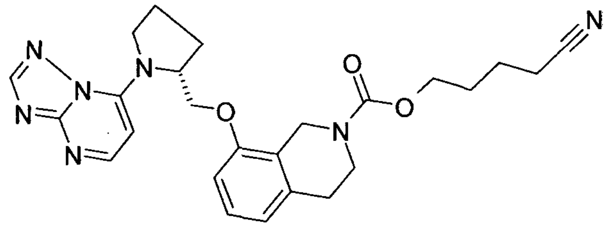<br>8-((R)-1-[1,2,4]Triazolo[1,5-a]pyrimidin-7-yl-pyrrolidin-2-ylmethoxy)-3,4-dihydro-1H-isochinolin-2-carbonsäure-4-cyan-butyl ester |
| "A41" | 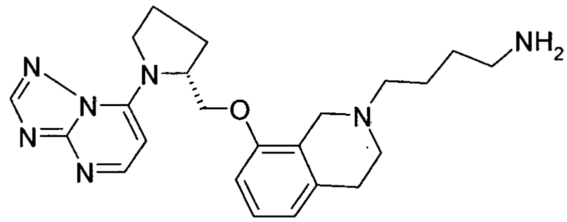<br>4-[8-((R)-1-[1,2,4]Triazolo[1,5-a]pyrimidin-7-yl-pyrrolidin-2-ylmethoxy)-3,4-dihydro-1H-isochinolin-2-yl]-butylamin |
| "A42" | 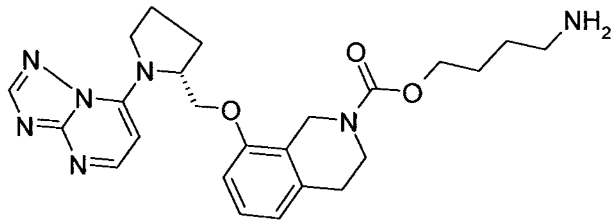<br>8-((R)-1-[1,2,4]Triazolo[1,5-a]pyrimidin-7-yl-pyrrolidin-2-ylmethoxy)-3,4-dihydro-1H-isochinolin-2-carbonsäure-4-amino-butylester |
| "A46" | 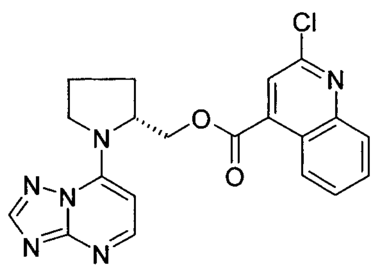<br>2-Chlor-chinolin-4-carbonsäure-(R)-1-[1,2,4]triazolo[1,5-a]pyrimidin-7-yl-pyrrolidin-2-yl-methylester |

(fortgesetzt)

| Verbindung Nr. | Name und/oder Struktur |
|---|---|
| "A53" | 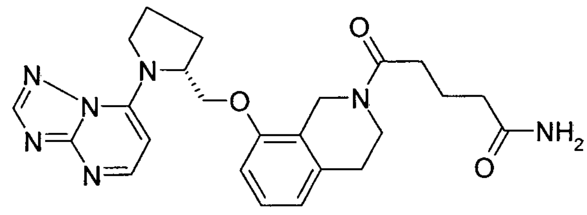 5-Oxo-5-[8-((R)-1-[1,2,4]triazolo[1,5-a]pyrimidin-7-yl-pyrrolidin-2-ylmethoxy)-3,4-dihydro-1H-isochinolin-2-yl]-pentansäure-amid |
| "A59" | 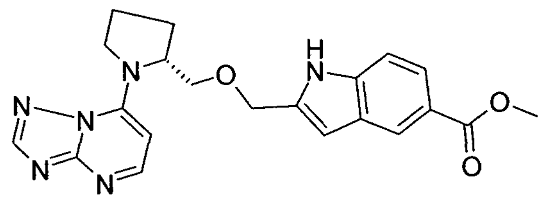 2-((R)-1-[1,2,4]Triazolo[1,5-a]pyrimidin-7-yl-pyrrolidin-2-ylmethoxymethyl)-1H-indol-5-carbonsäure-methylester |
| "A75" | 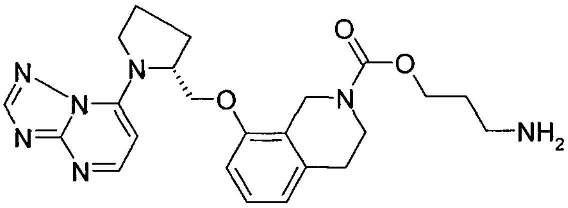 8-((R)-1-[1,2,4]Triazolo[1,5-a]pyrimidin-7-yl-pyrrolidin-2-ylmethoxy)-3,4-dihydro-1H-isochinolin-2-carbonsäure-3-amino-propyl ester |
| "A76" | 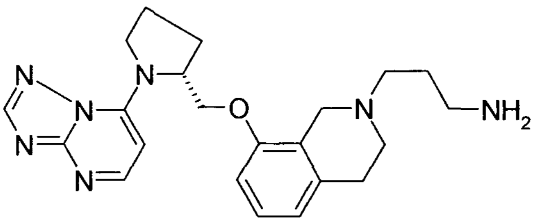 3-[8-((R)-1-[1,2,4]Triazolo[1,5-a]pyrimidin-7-yl-pyrrolidin-2-ylmethoxy)-3,4-dihydro-1H-isochinolin-2-yl]-propylamin |
| "A77" | 7-[(R)-2-(Naphthalin-1-yloxymethyl)-pyrrolidin-1-yl]-[1,2,4]triazolo[1,5-a][1,3,5]triazin |
| "A78" | 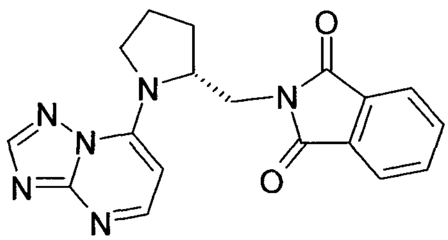 2-((R)-1-[1,2,4]Triazolo[1,5-a]pyrimidin-7-yl-pyrrolidin-2-ylmethyl)-isoindol-1,3-dion |

(fortgesetzt)

| Verbindung Nr. | Name und/oder Struktur |
|---|---|
| "A81" | 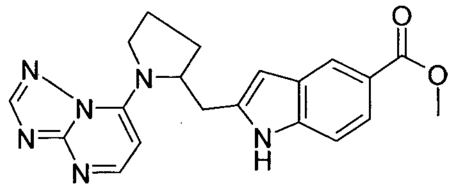 2-(1-[1,2,4]Triazolo[1,5-a]pyrimidin-7-yl-pyrrolidin-2-ylmethyl)-1 H-indol-5-carbonsäure-methylester |
| "A93" | 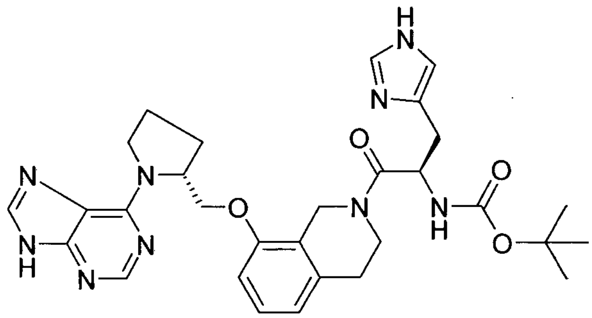 ((R)-1-(1H-Imidazol-4-ylmethyl)-2-oxo-2-{8-[(R)-1-(9H-purin-6-yl)-pyrrolidin-2-ylmethoxy]-3,4-dihydro-1H-isochinolin-2-yl}-ethyl)-carbaminsäure-tert.-butyl ester |

sowie ihre pharmazeutisch verwendbaren Salze, Tautomere und Stereoisomere, einschließlich deren Mischungen in allen Verhältnissen.

**3.** Verfahren zur Herstellung von Verbindungen der Formel I nach den Ansprüchen 1-2 sowie ihrer pharmazeutisch verwendbaren Salze, Tautomeren und Stereoisomeren, **dadurch gekennzeichnet, daß** man eine Verbindung der Formel II

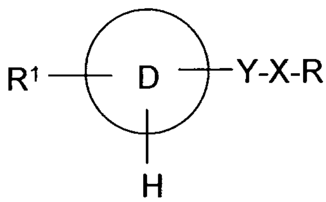

II

worin $R^1$, X, Y und R die in Anspruch 1 angegebenen Bedeutungen haben, mit einer Verbindung der Formel III

Z-Cl III

worin Z die in Anspruch 1 angegebene Bedeutung hat,
umsetzt,
und/oder
eine Base oder Säure der Formel I in eines ihrer Salze umwandelt.

**4.** Arzneimittel, enthaltend mindestens eine Verbindung der Formel I nach Anspruch 1-2 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

**5.** Verwendung von Verbindungen nach Anspruch 1-2 sowie ihrer pharmazeutisch verwendbaren Salze, Tautomeren und Stereoisomeren, einschließlich deren Mischungen in allen Verhältnissen, zur Herstellung eines Arzneimittels

zur Behandlung von Tumoren, Tumormetastasen, proliferativer Erkrankungen der Mesangiumzellen, Hämangiom, proliferativer Retinopathie, rheumatoider Arthritis, atherosklerotischer Neovaskularisation, Psoriasis, okularer Neovaskularisation, Osteoporose, Diabetes und Fettleibigkeit, lymphoider Leukämie und Lymphom.

**6.** Verwendung nach Anspruch 5, wobei die Tumorerkrankung ausgewählt ist aus der Gruppe des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Magens, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom,

**7.** Verwendung von Verbindungen der Formel I gemäß Anspruch 1-2 und/oder ihrer physiologisch unbedenklichen Salze zur Herstellung eines Arzneimittels zur Behandlung von Tumoren, wobei eine therapeutisch wirksame Menge einer Verbindung der Formel I in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

**8.** Verwendung von Verbindungen der Formel I gemäß Anspruch 1-2 und/oder ihrer physiologisch unbedenklichen Salze zur Herstellung eines Arzneimittels zur Behandlung von Tumoren wobei eine therapeutisch wirksame Menge einer Verbindung der Formel I in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-ProteaseHemmer, 9) Reverse-Transkriptase-Hemmer sowie 10) weiterer Angiogenese-Hemmer verabreicht wird.

**9.** Verbindungen nach Anspruch 1, ausgewählt aus der Gruppe

| Verbindung Nr. | Name und/oder Struktur |
|---|---|
| "A19" | 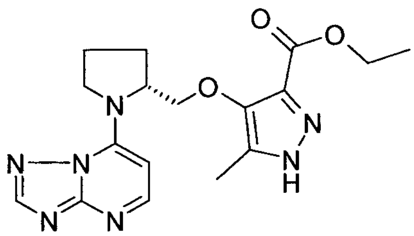 5-Methyl-4-((R)-1-[1,2,4]triazolo[1,5-a]pyrimidin-7-yl-pyrrolidin-2-ylmethoxy)-1H-pyrazol-3-carbonsäure-ethylester |
| "A26" | 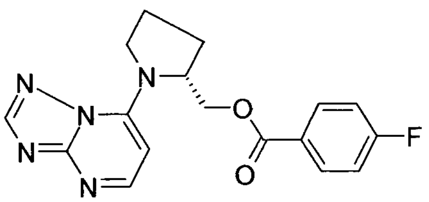 4-Fluor-benzoesäure-(R)-1-[1,2,4]triazolo[1,5-a]pyrimidin-7-yl-pyrrolidin-2-yl-methylester |
| "A43" | 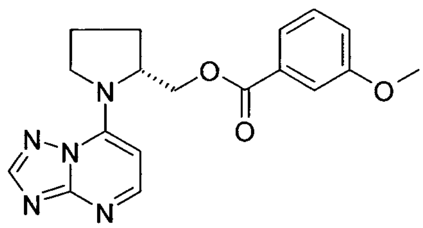 3-Methoxy-benzoesäure-(R)-1-[1,2,4]triazolo[1,5-a]pyrimidin-7-yl-pyrrolidin-2-yl-methylester |

(fortgesetzt)

| Verbindung Nr. | Name und/oder Struktur |
|---|---|
| "A44" | 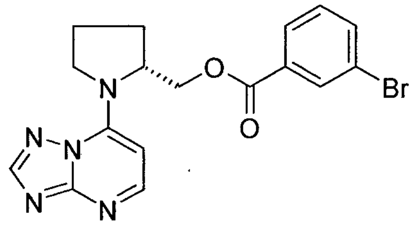 3-Brom-benzoesäure-(R)-1-[1,2,4]triazolo[1,5-a]pyrimidin-7-yl-pyrrolidin-2-yl-methylester |
| "A45" | 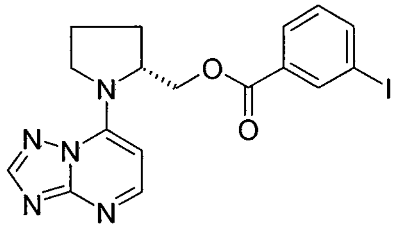 3-Iod-benzoesäure-(R)-1-[1,2,4]triazolo[1,5-a]pyrimidin-7-yl-pyrrolidin-2-yl-methylester |
| "A50" | 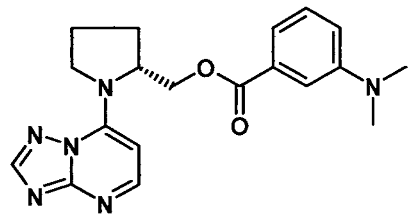 3-Dimethylamino-benzoesäure-(R)-1-[1,2,4]triazolo[1,5-a]pyrimidin-7-yl-pyrrolidin-2-yl-methylester |
| "A52" | 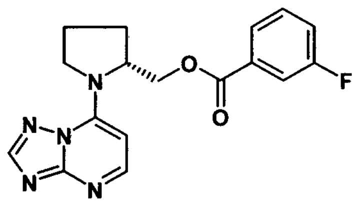 3-Fluor-benzoesäure-(R)-1-[1,2,4]triazolo[1,5-a]pyrimidin-7-yl-pyrrolidin-2-yl-methylester |
| "A61" | 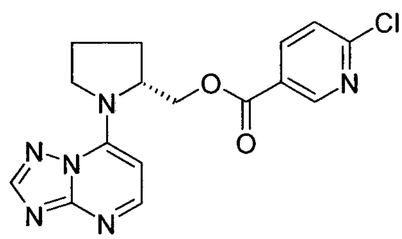 6-Chlor-nicotinsäure-(R)-1-[1,2,4]triazolo[1,5-a]pyrimidin-7-yl-pyrrolidin-2-yl-methylester |
| "A63" | 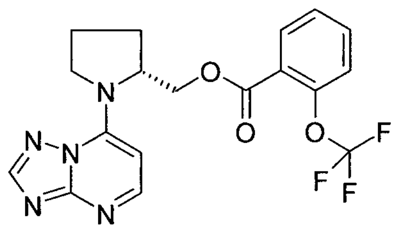 |

(fortgesetzt)

| Verbindung Nr. | Name und/oder Struktur |
|---|---|
| | 2-Trifluormethoxy-benzoesäure-(R)-1-[1,2,4]triazolo[1,5-a]pyrimidin-7-yl-pyrrolidin-2-yl-methylester |
| "A64" | 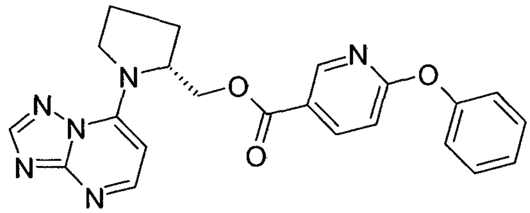 6-Phenoxy-nicotinsäure-(R)-1-[1,2,4]triazolo[1,5-a]pyrimidin-7-yl-pyrrolidin-2-yl-methylester |
| "A68" | 2-Iod-benzoesäure-(R)-1-[1,2,4]triazolo[1,5-a]pyrimidin-7-yl-pyrrolidin-2-yl-methylester |
| "A69" | Pyridin-2-carbonsäure-(R)-1-[1,2,4]triazolo[1,5-a]pyrimidin-7-yl-pyrrolidin-2-yl-methylester |
| "A70" | 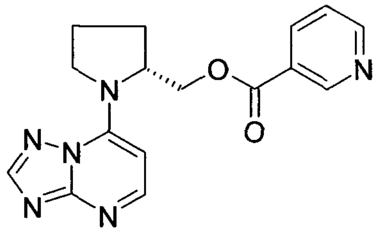 Nicotinsäure-(R)-1-[1,2,4]triazolo[1,5-a]pyrimidin-7-yl-pyrrolidin-2-yl-methylester |
| "A71" | Benzoesäure-(R)-1-[1,2,4]triazolo[1,5-a]pyrimidin-7-yl-pyrrolidin-2-yl-methylester |
| "A72" | 3-Ethoxy-benzoesäure-(R)-1-[1,2,4]triazolo[1,5-a]pyrimidin-7-yl-pyrrolidin-2-yl-methylester |
| "A73" | 5-Chlor-2-fluor-benzoesäure-(R)-1-[1,2,4]triazolo[1,5-a]pyrimidin-7-yl-pyrrolidin-2-yl-methylester |
| "A74" | 3-Chlormethyl-benzoesäure-(R)-1-[1,2,4]triazolo[1,5-a]pyrimidin-7-yl-pyrrolidin-2-yl-methylester |
| "A79" | 3-Cyan-benzoesäure-(R)-1-[1,2,4]triazolo[1,5-a]pyrimidin-7-yl-pyrrolidin-2-yl-methylester |
| "A82" | 3-Trifluormethoxy-benzoesäure-(R)-1-[1,2,4]triazolo[1,5-a]pyrimidin-7-yl-pyrrolidin-2-yl-methylester |
| "A83" | 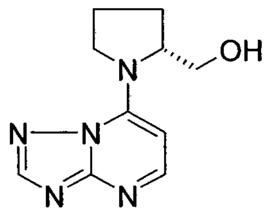 ((R)-1-[1,2,4]Triazolo[1,5-a]pyrimidin-7-yl-pyrrolidin-2-yl)-methanol |

sowie ihre pharmazeutisch verwendbaren Salze, Tautomere und Stereoisomere, einschließlich deren Mischungen in allen Verhältnissen.

**10.** Arzneimittel, enthaltend mindestens eine Verbindung nach Anspruch 9 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

## Claims

**1.** Compounds of the formula I

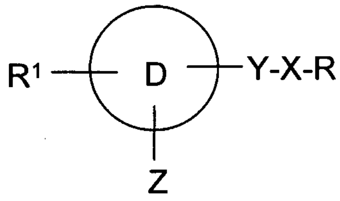

I

in which

p EMBED ISISServer ⓓ denotes pyrrolidinyl, imidazolinyl, bicyclo[2.2.1]hept-5-yl, morpholinyl or N,
Z denotes imidazopyrimidinyl, triazolopyrimidinyl or pyrrolopyrimidinyl,
where Z is bonded to a nitrogen of the radical D,
Y denotes $(CH_2)_n$ or is absent,
X denotes $O(CH_2)_r$, NH, NA, OC(=O), $NHSO_2$ or is absent,
$R^1$ denotes H, A or COA,
R denotes Het,
Het denotes a bicyclic saturated, unsaturated or aromatic heterocycle having 1 to 4 N and/or O and/or S atoms which is unsubstituted or mono-, di- or trisubstituted by Hal, A, $(CH_2)_mOR^6$, $(CH_2)_mN(R^6)_2$, $(CH_2)_mCN$, $(CH_2)_mCOOR^6$, $CONH(CH_2)_mCOOH$, $CONH(CH_2)_mHet^2$, $CO(CH_2)_mN(R^6)_2$, $CO(CH_2)_mNH(CH_2)_rCOOA$, $(CH_2)_rCONH(CH_2)_mAr^1$, $COCH[(CH_2)_mCONH_2]NH_2$, $COCH[(CH_2)_mCONH_2]NHCOOA$, $COCH[(CH_2)_mHet^2]NHCOOA$, $COCH(NHCOA)CH_2CONH_2$, $(CH_2)_mNHCOOA$, $COCH[(CH_2)_mCN]NHCOOA$, $COO(CH_2)_mCN$, $COO(CH_2)_mN(R^6)_2$, $CO(CH_2)_mCON(R^6)_2$, $COR^6$, $COHet^2$, $O(CH_2)_mAr^1$ and/or =O (carbonyl oxygen),
$Het^2$ denotes imidazolyl, indolyl, isoxazolyl, pyridazinyl, pyrazolyl, benzimidazolyl, pyridyl, dibenzofuranyl, carbazolyl, benzofuranyl, isoindolyl, quinazolinyl, quinoxalinyl, pyrimidinyl, indazolyl, furyl, thienyl, pyrrolyl, oxazolyl, thiazolyl, triazolyl, tetrazolyl, thiadiazole, benzothiazolyl, imidazo[1,2-*a*]-pyridinyl or 1,3-benzodioxolyl,
$R^6$ denotes H or alkyl having 1-6 C atoms,
$Ar^1$ denotes phenyl which is unsubstituted or mono-, di- or trisubstituted by Hal and/or $SO_2NH_2$,
A denotes unbranched or branched alkyl having 1-10 C atoms, in which 1-7 H atoms may be replaced by F, Cl, Br and/or OH,
or
cyclic alkyl having 3-7 C atoms,
Hal denotes F, Cl, Br or I,
m denotes 0, 1, 2, 3, 4, 5 or 6,
n denotes 1 or 2,
q denotes 0, 1, 2, 3 or 4,
r denotes 0, 1 or 2,

and pharmaceutically usable salts, tautomers and stereoisomers thereof, including mixtures thereof in all ratios.

**2.** Compounds according to Claim 1, selected from the group

| Compound No. | Name and/or structure |
|---|---|
| "A5" | 5-((R)-1-(1,2,4-Triazolo[1,5-a]pyrimidin-7-yl)pyrrolidin-2-yl-methoxy)quinoline |
| "A8" | 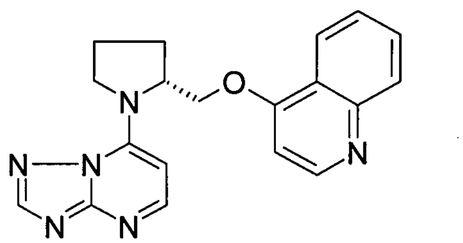 4-((R)-1-(1,2,4-Triazolo[1,5-a]pyrimidin-7-yl)pyrrolidin-2-yl-methoxy)quinoline |

(continued)

| Compound No. | Name and/or structure |
|---|---|
| "A9" | 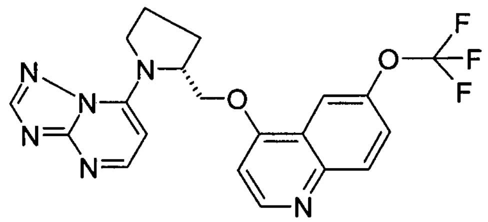<br>4-((R)-1-(1,2,4-Triazolo[1,5-a]pyrimidin-7-yl)pyrrolidin-2-yl-methoxy)-6-trifluoromethoxyquinoline |
| "A10" | 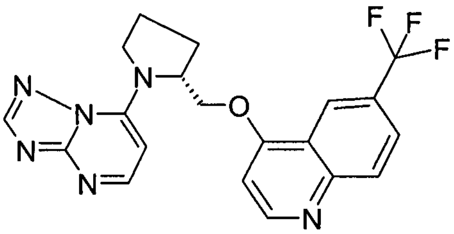<br>4-((R)-1-(1,2,4-Triazolo[1,5-a]pyrimidin-7-yl)pyrrolidin-2-yl-methoxy)-6-trifluoromethylquinoline |
| "A11" | 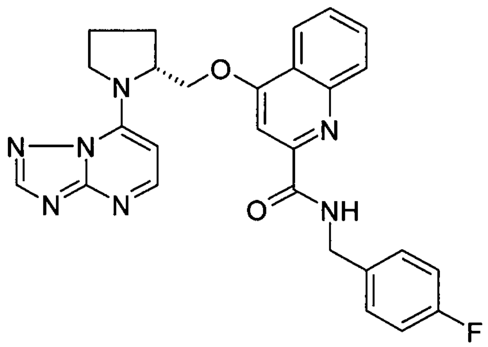<br>N-4-Fluorobenzyl-4-((R)-1-(1,2,4-triazolo[1,5-a]pyrimidin-7-yl)pyrrolidin-2-ylmethoxy)quinoline-2-carboxamide |
| "A12" | 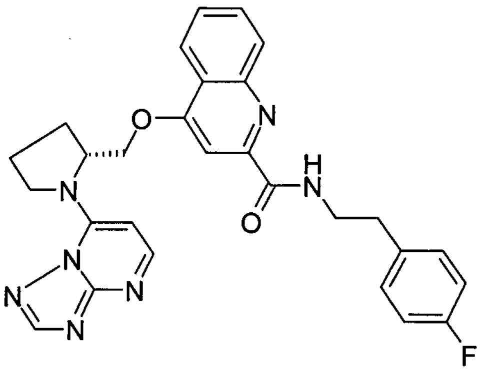<br>N-[2-(4-Fluorophenyl)ethyl]-4-((R)-1-(1,2,4-triazolo[1,5-a]-pyrimidin-7-yl)pyrrolidin-2-ylmethoxy)quinoline-2-carboxamide |

(continued)

| Compound No. | Name and/or structure |
|---|---|
| "A13" | 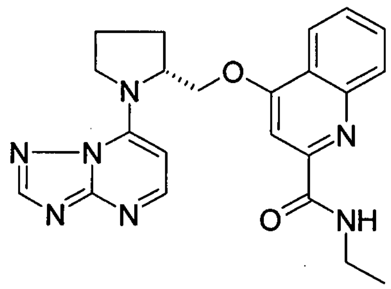 N-Ethyl-4-((R)-1-(1,2,4-triazolo[1,5-a]pyrimidin-7-yl)-pyrrolidin-2-ylmethoxy)quinoline-2-carboxamide |
| "A16" | 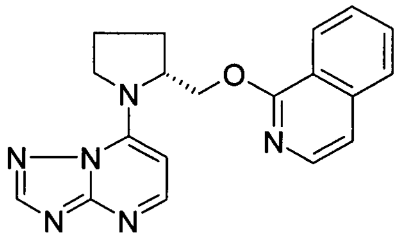 1-((R)-1-(1,2,4-Triazolo[1,5-a]pyrimidin-7-yl)pyrrolidin-2-yl-methoxy)isoquinoline |
| "A17" | 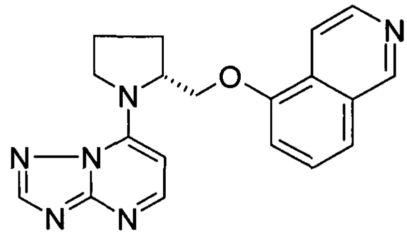 5-((R)-1-(1,2,4-Triazolo[1,5-a]pyrimidin-7-yl)pyrrolidin-2-yl-methoxy)isoquinoline |
| "A18" | 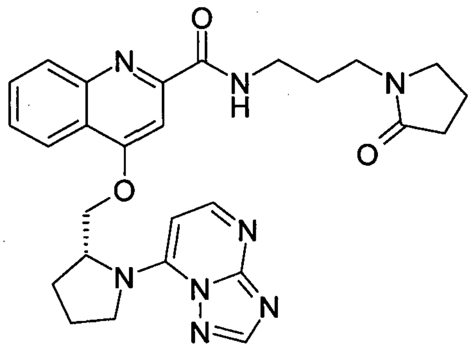 N-[3-(2-Oxopyrrolidin-1-yl)propyl]-4-((R)-1-(1,2,4-triazolo-[1,5-a]pyrimidin-7-yl)pyrrolidin-2-ylmethoxy)quinoline-2-carboxamide |

(continued)

| Compound No. | Name and/or structure |
|---|---|
| "A22" | 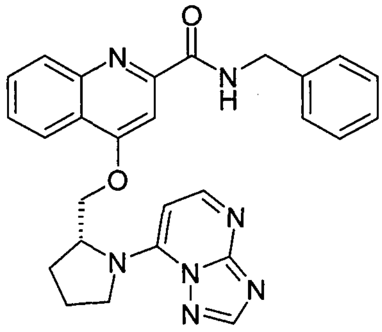<br>N-Benzyl-4-((R)-1-(1,2,4-triazolo[1,5-a]pyrimidin-7-yl)-pyrrolidin-2-ylmethoxy)quinoline-2-carboxamide |
| "A23" | 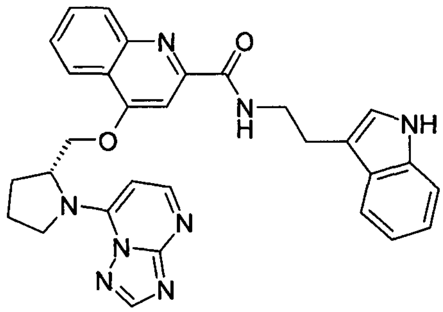<br>N-[2-(1H-Indol-3-yl)ethyl]-4-((R)-1-(1,2,4-triazolo[1,5-a]-pyrimidin-7-yl)pyrrolidin-2-ylmethoxy)quinoline-2-carboxamide |
| "A24" | 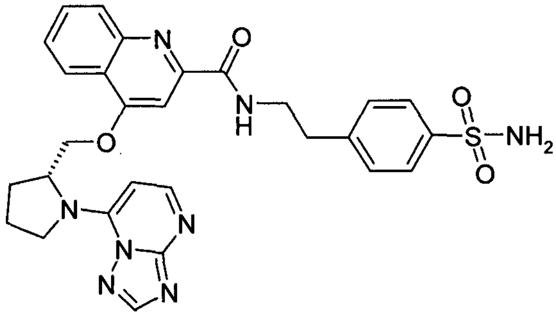<br>N-[2-(4-Sulfamoylphenyl)ethyl]-4-((R)-1-(1,2,4-triazolo-[1,5-a]pyrimidin-7-yl)pyrrolidin-2-ylmethoxy)quinoline-2-carboxamide |
| "A25" | 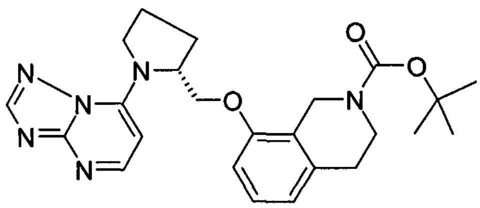<br>tert-Butyl 8-((R)-1-(1,2,4-triazolo[1,5-a]pyrimidin-7-yl)-pyrrolidin-2-ylmethoxy)-3,4-dihydro-1H-isoquinoline-2-carboxylate |

(continued)

| Compound No. | Name and/or structure |
|---|---|
| "A27" | 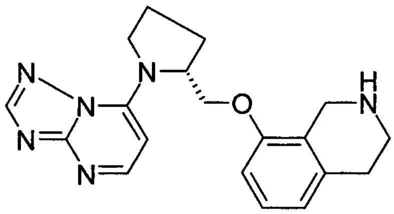 8-((R)-1-(1,2,4-Triazolo[1,5-a]pyrimidin-7-yl)pyrrolidin-2-yl-methoxy)-1,2,3,4-tetrahydroisoquinoline |
| "A28" | 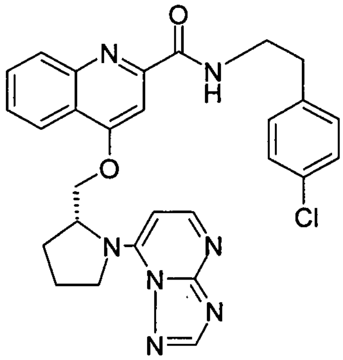 N-[2-(4-Chlorophenyl)ethyl]-4-((R)-1-(1,2,4-triazolo[1,5-a]-pyrimidin-7-yl)pyrrolidin-2-ylmethoxy)quinoline-2-carboxamide |
| "A29" | 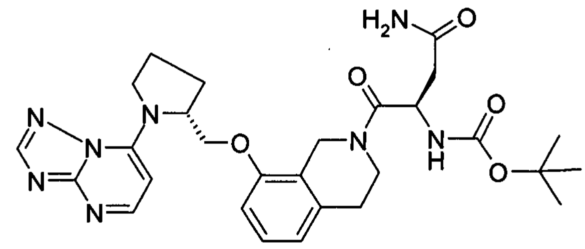 tert-Butyl {(R)-1-carbamoylmethyl-2-oxo-2-[8-((R)-1-(1,2,4-triazolo[1,5-a]pyrimidin-7-yl)pyrrolidin-2-ylmethoxy)-3,4-dihydro-1H-isoquinolin-2-yl]ethyl}carbamate |
| "A30" | 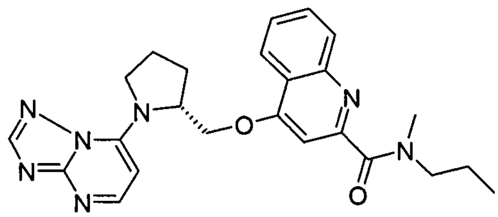 N-Ethylpropyl-4-((R)-1-(1,2,4-triazolo[1,5-a]pyrimidin-7-yl)-pyrrolidin-2-ylmethoxy)quinoline-2-carboxamide |
| "A31" | 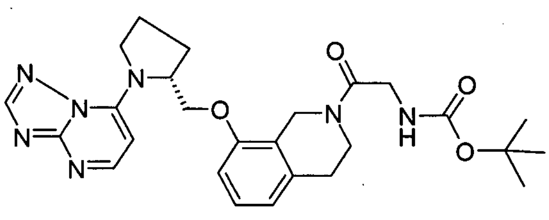 tert-Butyl {2-oxo-2-[8-((R)-1-(1,2,4-triazolo[1,5-a]pyrimidin-7-yl)pyrrolidin-2-ylmethoxy)-3,4-dihydro-1H-isoquinolin-2-yl]ethyl}carbamate |

(continued)

| Compound No. | Name and/or structure |
|---|---|
| "A32" | 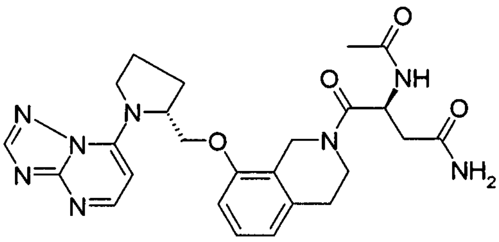 (S)-3-Acetylamino-4-oxo-4-[8-((R)-1-(1,2,4-triazolo[1,5-a]-pyrimidin-7-yl)pyrrolidin-2-ylmethoxy)-3,4-dihydro-1H-isoquinolin-2-yl]butyramide |
| "A33" | 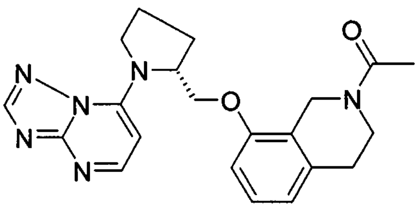 1-[8-((R)-1-(1,2,4-Triazolo[1,5-a]pyrimidin-7-yl)pyrrolidin-2-ylmethoxy)-3,4-dihydro-1H-isoquinolin-2-yl]ethanone |
| "A34" | 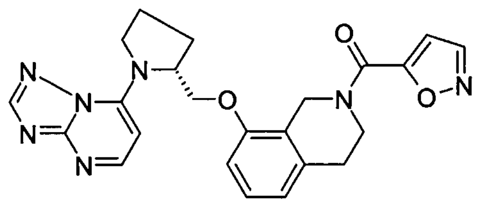 Isoxazol-5-yl-[8-((R)-1-(1,2,4-triazolo[1,5-a]pyrimidin-7-yl)-pyrrolidin-2-ylmethoxy)-3,4-dihydro-1H-isoquinolin-2-yl]-methanone |
| "A35" | 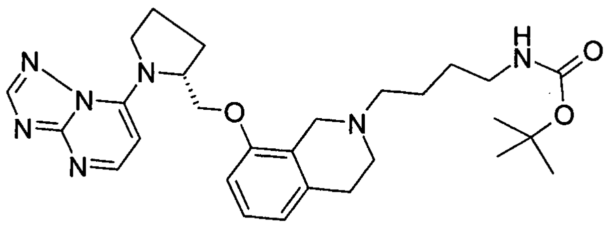 tert-Butyl {4-[8-((R)-1-(1,2,4-triazolo[1,5-a]pyrimidin-7-yl)-pyrrolidin-2-ylmethoxy)-3,4-dihydro-1H-isoquinolin-2-yl]-butyl}carbamate |
| "A36" | 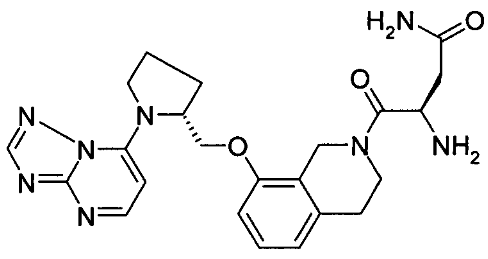 (R)-3-Amino-4-oxo-4-[8-((R)-1-(1,2,4-triazolo[1,5-a]-pyrimidin-7-yl)pyrrolidin-2-ylmethoxy)-3,4-dihydro-1H-isoquinolin-2-yl]butyramide |

(continued)

| Compound No. | Name and/or structure |
|---|---|
| "A37" | 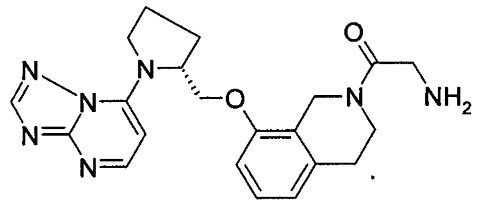 2-Amino-1-[8-((R)-1-(1,2,4-triazolo[1,5-a]pyrimidin-7-yl)-pyrrolidin-2-ylmethoxy)-3,4-dihydro-1H-isoquinolin-2-yl]-ethanone |
| "A38" | 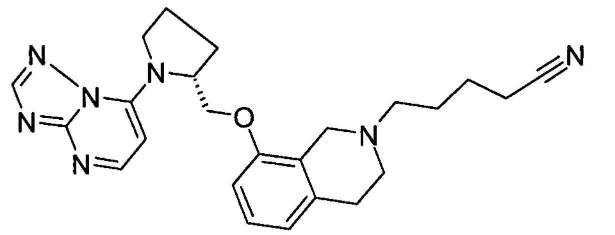 5-[8-((R)-1-(1,2,4-Triazolo[1,5-a]pyrimidin-7-yl)pyrrolidin-2-ylmethoxy)-3,4-dihydro-1H-isoquinolin-2-yl]pentanenitrile |
| "A39" | 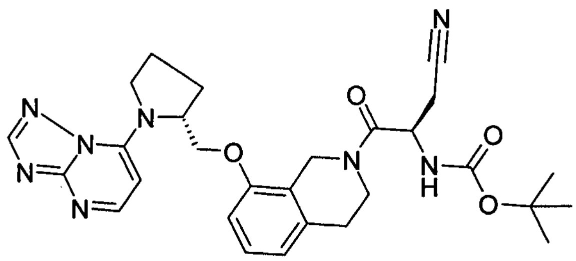 tert-Butyl {(R)-2-cyano-1-[8-((R)-1-(1,2,4-triazolo[1,5-a]-pyrimidin-7-yl)pyrrolidin-2-ylmethoxy)-3,4-dihydro-1H-isoquinoline-2-carbonyl]ethyl}carbamate |
| "A40" | 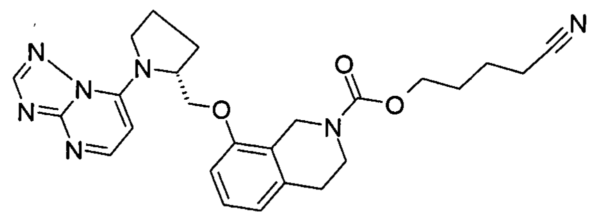 4-Cyanobutyl 8-((R)-1-(1,2,4-triazolo[1,5-a]pyrimidin-7-yl)-pyrrolidin-2-ylmethoxy)-3,4-dihydro-1H-isoquinoline-2-carboxylate |
| "A41" | 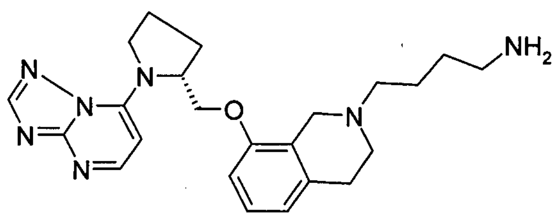 4-[8-((R)-1-(1,2,4-Triazolo[1,5-a]pyrimidin-7-yl)pyrrolidin-2-ylmethoxy)-3,4-dihydro-1H-isoquinolin-2-yl]butylamine |

(continued)

| Compound No. | Name and/or structure |
|---|---|
| "A42" | 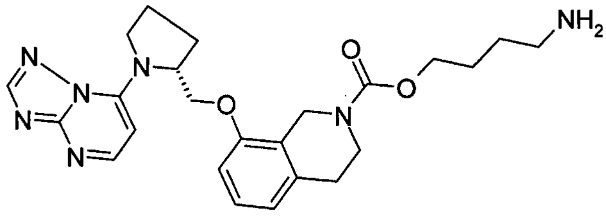 4-Aminobutyl 8-((R)-1-(1,2,4-triazolo[1,5-a]pyrimidin-7-yl)-pyrrolidin-2-ylmethoxy)-3,4-dihydro-1H-isoquinoline-2-carboxylate |
| "A46" | 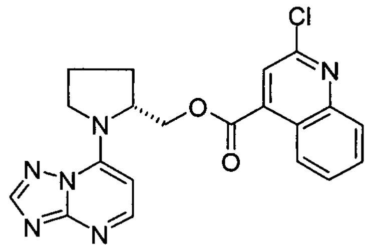 (R)-1-(1,2,4-Triazolo[1,5-a]pyrimidin-7-yl)pyrrolidin-2-yl-methyl 2-chloroquinoline-4-carboxylate |
| "A53" | 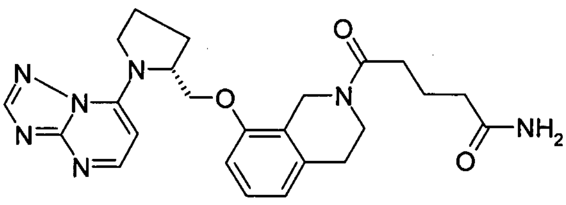 5-Oxo-5-[8-((R)-1-(1,2,4-triazolo[1,5-a]pyrimidin-7-yl)-pyrrolidin-2-ylmethoxy)-3,4-dihydro-1H-isoquinolin-2-yl]-pentanamide |
| "A59" | 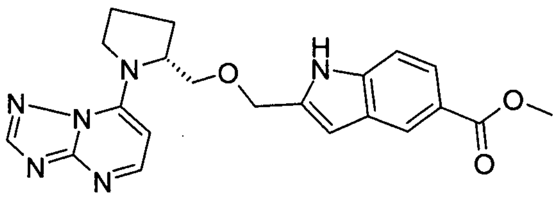 Methyl 2-((R)-1-(1,2,4-triazolo[1,5-a]pyrimidin-7-yl)-pyrrolidin-2-ylmethoxymethyl)-1H-indole-5-carboxylate |
| "A75" | 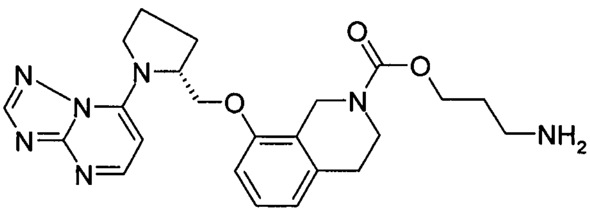 3-Aminopropyl 8-((R)-1-(1,2,4-triazolo[1,5-a]pyrimidin-7-yl)pyrrolidin-2-ylmethoxy)-3,4-dihydro-1H-isoquinoline-2-carboxylate |

(continued)

| Compound No. | Name and/or structure |
|---|---|
| "A76" | 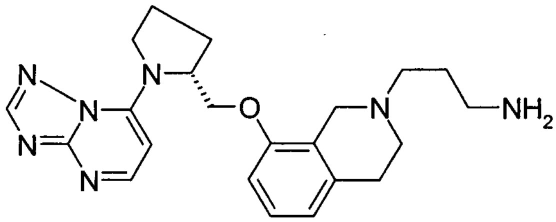 3-[8-((R)-1-(1,2,4-Triazolo[1,5-a]pyrimidin-7-yl)pyrrolidin-2-ylmethoxy)-3,4-dihydro-1H-isoquinolin-2-yl]propylamine |
| "A77" | 7-[(R)-2-(Naphthalen-1-yloxymethyl)pyrrolidin-1-yl]-[1,2,4]-triazolo[1,5-a]-1,3,5-triazine |
| "A78" | 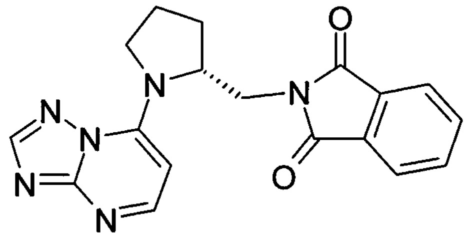 2-((R)-1-(1,2,4-Triazolo[1,5-a]pyrimidin-7-yl)pyrrolidin-2-yl-methyl)isoindole-1,3-dione |
| "A81" | 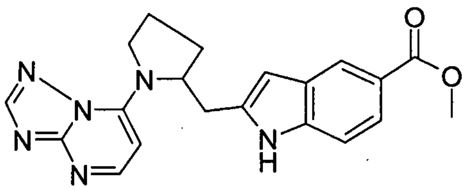 Methyl 2-(1-(1,2,4-triazolo[1,5-a]pyrimidin-7-yl)pyrrolidin-2-ylmethyl)-1H-indole-5-carboxylate |
| "A93" | 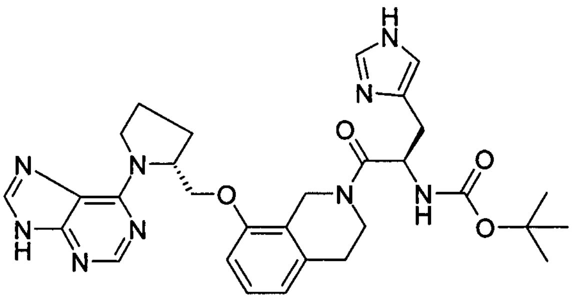 tert-Butyl ((R)-1-(1H-imidazol-4-ylmethyl)-2-oxo-2-{8-[(R)-1-(9H-purin-6-yl)pyrrolidin-2-ylmethoxy]-3,4-dihydro-1H-isoquinolin-2-yl}ethyl)carbamate |

and pharmaceutically usable salts, tautomers and stereoisomers thereof, including mixtures thereof in all ratios.

3. Process for the preparation of compounds of the formula I according to Claims 1-2 and pharmaceutically usable salts, tautomers and stereoisomers thereof, **characterised in that**
a compound of the formula II

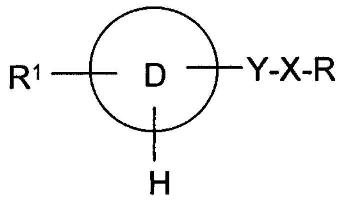

II

in which $R^1$, X, Y and R have the meanings indicated in Claim 1, is reacted with a compound of the formula III

Z-Cl III

in which Z has the meaning indicated in Claim 1,
and/or
a base or acid of the formula I is converted into one of its salts.

**4.** Medicaments comprising at least one compound of the formula I according to Claims 1-2 and/or pharmaceutically usable salts, tautomers and stereoisomers thereof, including mixtures thereof in all ratios, and optionally excipients and/or adjuvants.

**5.** Use of compounds according to Claims 1-2
and pharmaceutically usable salts, tautomers and stereoisomers thereof, including mixtures thereof in all ratios, for the preparation of a medicament for the treatment of tumours, tumour metastases, proliferative diseases of the mesangial cells, haemangioma, proliferative retinopathy, rheumatoid arthritis, atherosclerotic neovascularisation, psoriasis, ocular neovascularisation, osteoporosis, diabetes and obesity, lymphoid leukaemia and lymphoma.

**6.** Use according to Claim 5, where the tumour disease is selected from the group
of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx, of the lung, of the skin, monocytic leukaemia, lung adenocarcinoma, small-cell lung carcinoma, pancreatic cancer, glioblastoma, breast carcinoma, acute myeloid leukaemia, chronic myeloid leukaemia, acute lymphatic leukaemia, chronic lymphatic leukaemia, Hodgkin's lymphoma, non-Hodgkin's lymphoma.

**7.** Use of compounds of the formula I according to Claims 1-2 and/or physiologically acceptable salts thereof for the preparation of a medicament for the treatment of tumours, where a therapeutically effective amount of a compound of the formula I is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

**8.** Use of compounds of the formula I according to Claims 1-2 and/or physiologically acceptable salts thereof for the preparation of a medicament for the treatment of tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

**9.** Compounds according to Claim 1, selected from the group

| Compound No. | Name and/or structure |
|---|---|
| "A19" | 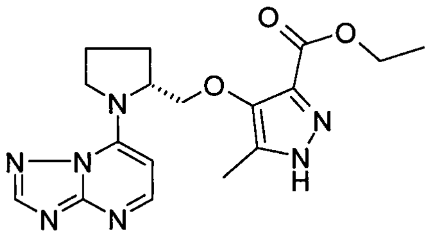<br>Ethyl 5-methyl-4-((R)-1-(1,2,4-triazolo[1,5-a]pyrimidin-7-yl)-pyrrolidin-2-ylmethoxy)-1H-pyrazole-3-carboxylate |
| "A26" | 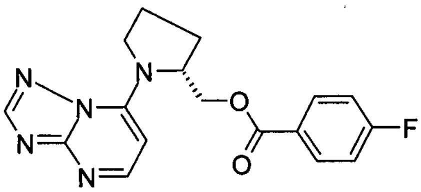<br>(R)-1-(1,2,4-Triazolo[1,5-a]pyrimidin-7-yl)pyrrolidin-2-yl-methyl 4-fluorobenzoate |
| "A43" | 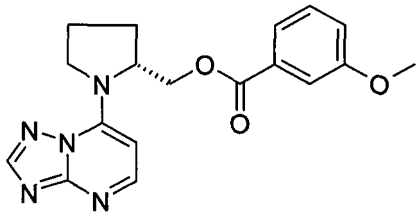<br>(R)-1-(1,2,4-Triazolo[1,5-a]pyrimidin-7-yl)pyrrolidin-2-yl-methyl 3-methoxybenzoate |
| "A44" | 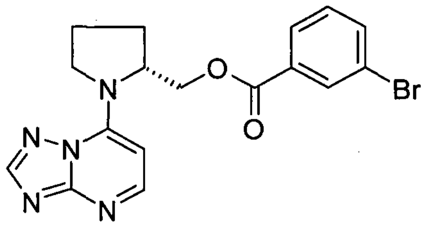<br>(R)-1-(1,2,4-Triazolo[1,5-a]pyrimidin-7-yl)pyrrolidin-2-yl-methyl 3-bromobenzoate |
| "A45" | 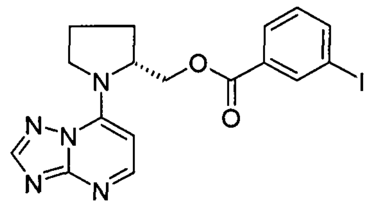<br>(R)-1-(1,2,4-Triazolo[1,5-a]pyrimidin-7-yl)pyrrolidin-2-yl-methyl 3-iodobenzoate |
| "A50" | 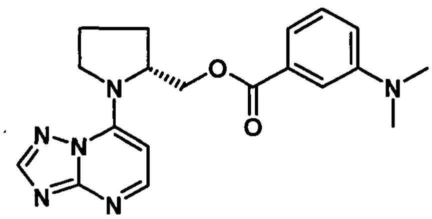<br>(R)-1-(1,2,4-Triazolo[1,5-a]pyrimidin-7-yl)pyrrolidin-2-yl-methyl 3-dimethylaminobenzoate |

(continued)

| Compound No. | Name and/or structure |
|---|---|
| "A52" | 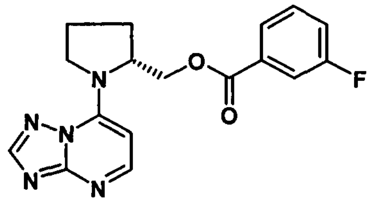 (R)-1-(1,2,4-Triazolo[1,5-a]pyrimidin-7-yl)pyrrolidin-2-ylmethyl 3-fluorobenzoate |
| "A61" | 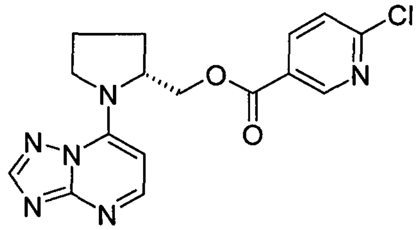 (R)-1-(1,2,4-Triazolo[1,5-a]pyrimidin-7-yl)pyrrolidin-2-yl-methyl 6-chloronicotinate |
| "A63" | 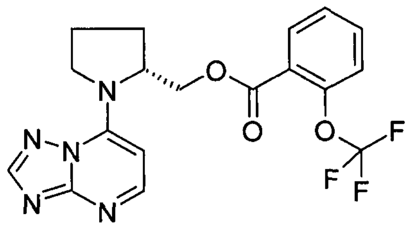 (R)-1-(1,2,4-Triazolo[1,5-a]pyrimidin-7-yl)pyrrolidin-2-yl-methyl 2-trifluoromethoxybenzoate |
| "A64" | 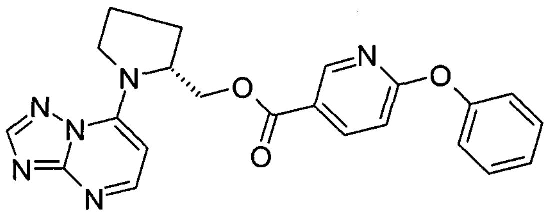 (R)-1-(1,2,4-Triazolo[1,5-a]pyrimidin-7-yl)pyrrolidin-2-yl-methyl 6-phenoxynicotinate |
| "A68" | (R)-1-(1,2,4-Triazolo[1,5-a]pyrimidin-7-yl)pyrrolidin-2-yl-methyl 2-iodobenzoate |
| "A69" | (R)-1-(1,2,4-Triazolo[1,5-a]pyrimidin-7-yl)pyrrolidin-2-yl-methyl pyridine-2-carboxylate |
| "A70" | 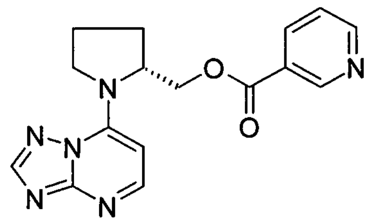 (R)-1-(1,2,4-Triazolo[1,5-a]pyrimidin-7-yl)pyrrolidin-2-yl-methyl nicotinate |
| "A71" | (R)-1-(1,2,4-Triazolo[1,5-a]pyrimidin-7-yl)pyrrolidin-2-yl-methyl benzoate |
| "A72" | (R)-1-(1,2,4-Triazolo[1,5-a]pyrimidin-7-yl)pyrrolidin-2-yl-methyl 3-ethoxybenzoate |
| "A73" | (R)-1-(1,2,4-Triazolo[1,5-a]pyrimidin-7-yl)pyrrolidin-2-yl-methyl 5-chloro-2-fluorobenzoate |
| "A74" | (R)-1-(1,2,4-Triazolo[1,5-a]pyrimidin-7-yl)pyrrolidin-2-yl-methyl 3-chloromethylbenzoate |
| "A79" | (R)-1-(1,2,4-Triazolo[1,5-a]pyrimidin-7-yl)pyrrolidin-2-yl-methyl 3-cyanobenzoate |

(continued)

| Compound No. | Name and/or structure |
|---|---|
| "A82" | (R)-1-(1,2,4-Triazolo[1,5-a]pyrimidin-7-yl)pyrrolidin-2-yl-methyl 3-trifluoromethoxybenzoate |
| "A83" | 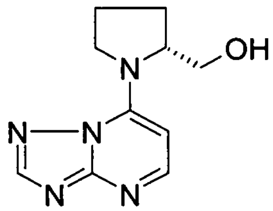 ((R)-1-(1,2,4-Triazolo[1,5-a]pyrimidin-7-yl)pyrrolidin-2-yl)-methanol |

and pharmaceutically usable salts, tautomers and stereoisomers thereof, including mixtures thereof in all ratios.

**10.** Medicaments comprising at least one compound according to Claim 9 and/or pharmaceutically usable salts, tautomers and stereoisomers thereof, including mixtures thereof in all ratios, and optionally excipients and/or adjuvants.

**Revendications**

**1.** Composés de formule I

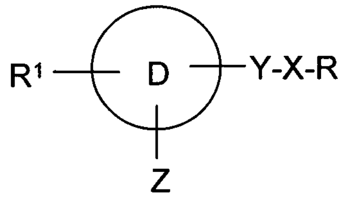

I

dans lesquels

Ⓓ désigne pyrrolidinyle, imidazolinyle, pyrazolyle, 2-oxa-5-azabicyclo[2.2.1]hept-5-yle, morpholinyle ou N,
Z désigne imidazopyrimidinyle, triazolopyrimidinyle ou pyrrolopyrimidinyle, où Z est lié à un azote du radical D,
Y désigne $(CH_2)n$ ou est absent,
X désigne $O(CH_2)_r$, NH, NA, OC(=O), $NHSO_2$ ou est absent,
$R^1$ désigne H, A ou COA,
R désigne Hét,
Hét désigne un hétérocycle bicyclique saturé, insaturé ou aromatique ayant de 1 à 4 atomes de N et/ou O et/ou S qui est non substitué ou mono-, di- ou trisubstitué par Hal, A, $(CH_2)_mOR^6$, $(CH_2)_mN(R^6)_2$, $(CH_2)_mCN$, $(CH_2)_mCOOR^6$, $CONH(CH_2)_mCOOH$, $CONH(CH_2)_mHét^2$, $CO(CH_2)_mN(R^6)_2$, $CO(CH_2)_mNH(CH_2)_rCOOA$, $(CH_2)_rCONH(CH_2)_mAr^1$, $COCH[(CH_2)_mCONH_2]NH_2$, $COCH[(CH_2)_mCONH_2]NHCOOA$, $COCH[(CH_2)_mHét^2]NHCOOA$, $COCH(NHCOA)CH_2CONH_2$, $(CH_2)_mNHCOOA$, $COCH[(CH_2)_mCN]NHCOOA$, $COO(CH_2)_mCN$, $COO(CH_2)_mN(R^6)_2$, $CO(CH_2)_mCON(R^6)_2$, $COR^6$, $COHét^2$, $O(CH_2)_mAr^1$ et/ou =O (oxygène du carbonyle),
$Hét^2$ désigne imidazolyle, indolyle, isoxazolyle, pyridazinyle, pyrazolyle, benzimidazolyle, pyridyle, dibenzofuranyle, carbazolyle, benzofuranyle, isoindolyle, quinazolinyle, quinoxalinyle, pyrimidinyle, indazolyle, furyle, thiényle, pyrrolyle, oxazolyle, thiazolyle, triazolyle, tétrazolyle, thiadiazole, benzothiazolyle, imidazo[1,2-a]pyridinyle ou 1,3-benzodioxolyle,
$R^6$ désigne H ou alkyle ayant 1-6 atomes de C,
$Ar^1$ désigne phényle qui est non substitué ou mono-, di- ou tri-substitué par Hal et/ou $SO_2NH_2$,
A désigne alkyle non ramifié ou ramifié ayant 1-10 atomes de C, où 1-7 atomes de H peuvent être remplacés par F, Cl, Br et/ou OH, ou alkyle cyclique ayant 3-7 atomes de C,
Hal désigne F, Cl, Br ou I,
m désigne 0, 1, 2, 3, 4, 5 ou 6,
n désigne 1 ou 2,

q désigne 0, 1, 2, 3 ou 4,
r désigne 0, 1 ou 2,

et les sels, tautomères et stéréoisomères pharmaceutiquement utilisables de ceux-ci, y compris des mélanges de ceux-ci en toutes proportions.

**2.** Composés selon la revendication 1, choisis dans le groupe constitué par

| Composé n° | Nom et/ou structure |
|---|---|
| « A5 » | 5-((R)-1-(1,2,4-Triazolo[1,5-a]pyrimidin-7-yl)pyrrolidin-2-yl-méthoxy)quinoléine |
| « A8 » | 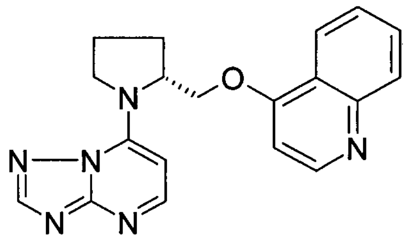 4-((R)-1-(1,2,4-Triazolo[1,5-a]pyrimidin-7-yl)pyrrolidin-2-yl-méthoxy)quinoléine |
| « A9 » | 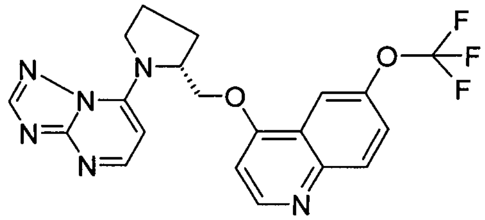 4-((R)-1-(1,2,4-Triazolo[1,5-a]pyrirnidin-7-yl)pyrrolidin-2-yl-méthoxy)-6-trifluorométhoxyquinoléine |
| « A10 » | 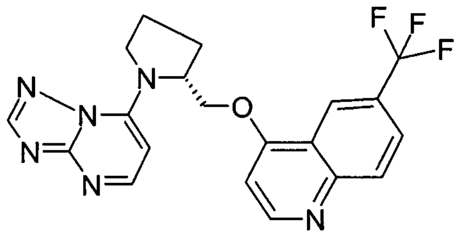 4-((R)-1-(1,2,4-Triazolo[1,5-a]pyrimidin-7-yl)pyrrolidin-2-yl-méthoxy)-6-trifluorométhylquinoléine |
| « A11 » | 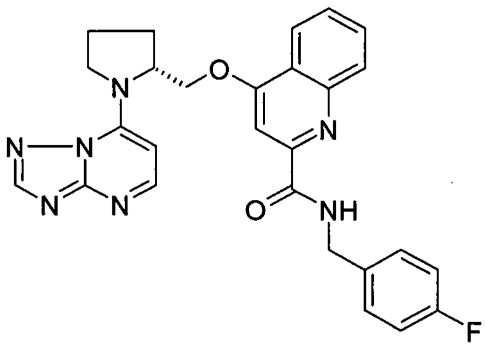 N-4-Fluorobenzyl-4-((R)-1-(1,2,4-triazolo[1,5-a]pyrimidin-7-yl)pyrrolidin-2-ylméthoxy)quinoléine-2-carboxamide |

(suite)

| Composé n° | Nom et/ou structure |
|---|---|
| « A12 » | 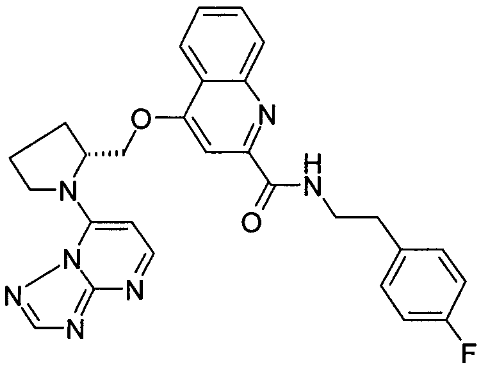 N-[2-(4-Fluorophényl)éthyl]-4-((R)-1-(1,2,4-triazolo[1,5-a]pyrimidin-7-yl)pyrrolidin-2-yl-méthoxy)quinoléine-2-carboxamide |
| « A13 » | 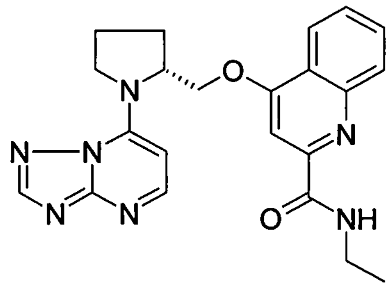 N-Éthyl-4-((R)-1-(1,2,4-triazolo[1,5-a]pyrimidin-7-yl)-pyrrolidin-2-ylméthoxy)quinoléine-2-carboxamide |
| « A16 » | 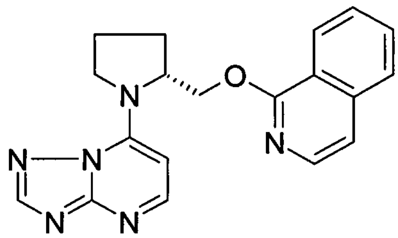 1-((R)-1-(1,2,4-Triazolo[1,5-a]pyrimidin-7-yl)pyrrolidin-2-yl-méthoxy)isoquinoléine |
| « A17 » | 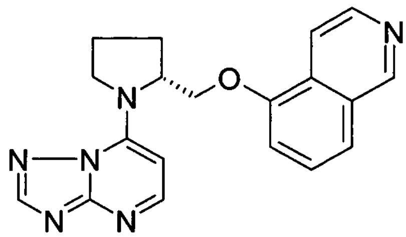 5-((R)-1-(1,2,4-Triazolo[1,5-a]pyrimidin-7-yl)pyrrolidin-2-yl-méthoxy)isoquinoléine |

(suite)

| Composé n° | Nom et/ou structure |
|---|---|
| « A18 » | 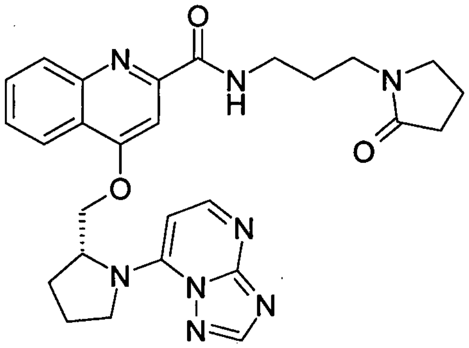<br>N-[3-(2-Oxopyrrolidin-1-yl)propyl]-4-((R)-1-(1,2,4-triazolo[1,5-a]pyrimidin-7-yl)pyrrolidin-2-yl-méthoxy)quinoléine-2-carboxamide |
| « A22 » | 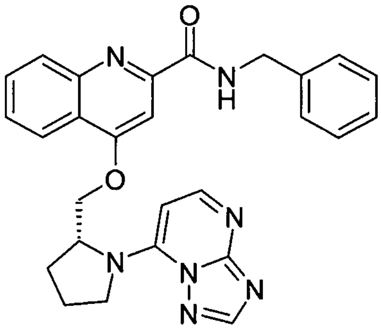<br>N-Benzyl-4-((R)-1-(1,2,4-triazolo[1,5-a]pyrimidin-7-yl)-pyrrolidin-2-ylméthoxy)quinoléine-2-carboxamide |
| « A23 » | 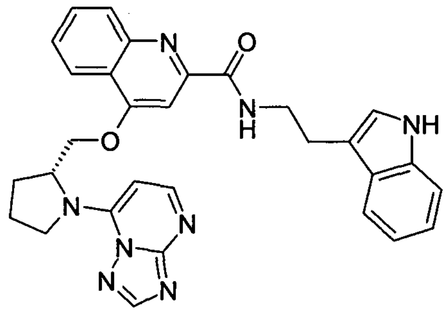<br>N-[2-(1H-Indol-3-yl)éthyl]-4-((R)-1-(1,2,4-triazolo[1,5-a]pyrimidin-7-yl)pyrrolidin-2-yl-méthoxy)quinoléine-2-carboxamide |
| « A24 » | 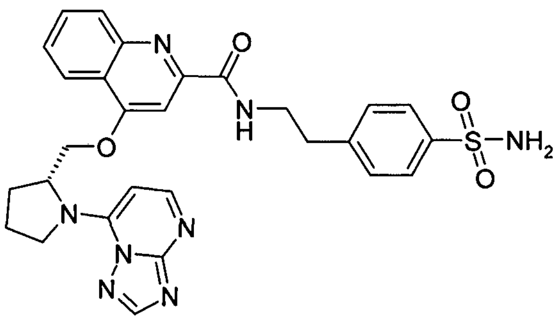<br>N-[2-(4-Sulfamoylphényl)éthyl]-4-((R)-1-(1,2,4-triazolo[1,5-a]pyrimidin-7-yl)pyrrolidin-2-yl-méthoxy)quinoléine-2-carboxamide |

(suite)

| Composé n° | Nom et/ou structure |
|---|---|
| « A25 » | 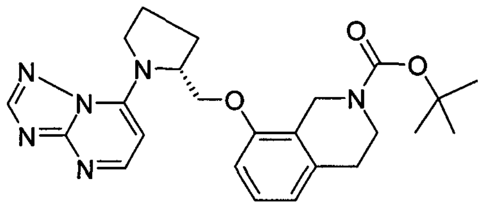<br>8-((R)-1-(1,2,4-Triazolo[1,5-a]pyrimidin-7-yl)pyrrolidin-2-yl-méthoxy)-3,4-dihydro-1H-isoquinoléine-2-carboxylate de tertio-butyle |
| « A27 » | 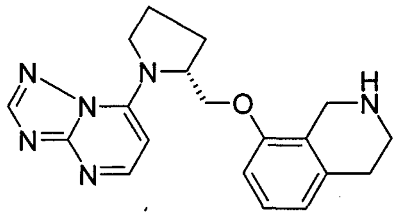<br>8-((R)-1-(1,2,4-Triazolo[1,5-a]pyrimidin-7-yl)pyrrolidin-2-yl-méthoxy)-1,2,3,4-tétrahydroisoquinoléine |
| « A28 » | 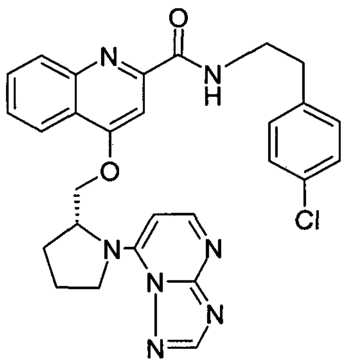<br>N-[2-(4-Chlorophényl)éthyl]-4-((R)-1-(1,2,4-triazolo[1,5-a]pyrimidin-7-yl)pyrrolidin-2-yl-méthoxy)quinoléine-2-carboxamide |
| « A29 » | 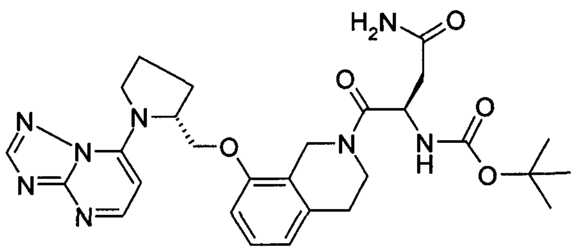<br>{(R)-1-Carbamoylméthyl-2-oxo-2-[8-((R)-1-(1,2,4-triazolo[1,5-a]pyrimidin-7-yl)pyrrolidin-2-ylméthoxy)-3,4-dihydro-1H-isoquinoléin-2-yl]éthyl}carbamate de tertio-butyle |
| « A30 » | 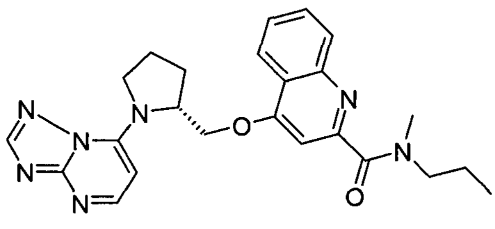<br>N-Éthylpropyl-4-((R)-1-(1,2,4-triazolo[1,5-a]pyrimidin-7-yl)-pyrrolidin-2-ylméthoxy)quinoléine-2-carboxamide |

(suite)

| Composé n° | Nom et/ou structure |
|---|---|
| « A31 » | 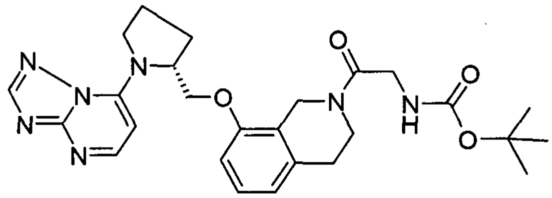 {2-Oxo-2-[8-((R)-1-(1,2,4-triazolo[1,5-a]pyrimidin-7-yl)pyrrolidin-2-ylméthoxy)-3,4-dihydro-1H-isoquinoléin-2-yl]éthyl}carbamate de tertio-butyle |
| « A32 » | 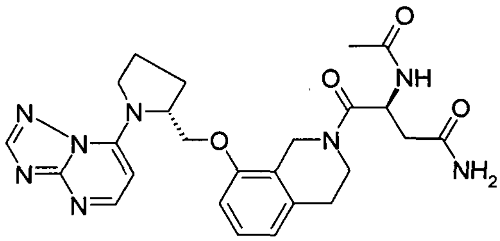 (S)-3-Acétylamino-4-oxo-4-[8-((R)-1-(1,2,4-triazolo[1,5-a]pyrimidin-7-yl)pyrrolidin-2-ylméthoxy)-3,4-dihydro-1H-isoquinoléin-2-yl]butyramide |
| « A33 » | 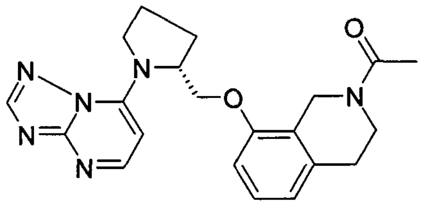 1-[8-((R)-1-(1,2,4-Triazolo[1,5-a]pyrimidin-7-yl)pyrrolidin-2-ylméthoxy)-3,4-dihydro-1H-isoquinoléin-2-yl]éthanone |
| « A34 » | 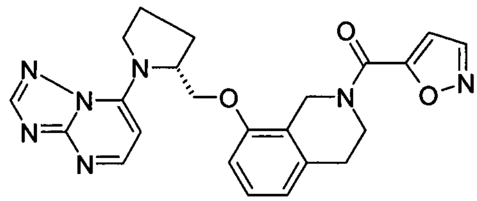 Isoxazol-5-yl-[8-((R)-1-(1,2,4-triazolo[1,5-a]pyrimidin-7-yl)-pyrrolidin-2-ylméthoxy)-3,4-dihydro-1H-isoquinoléin-2-yl]méthanone |
| « A35 » | 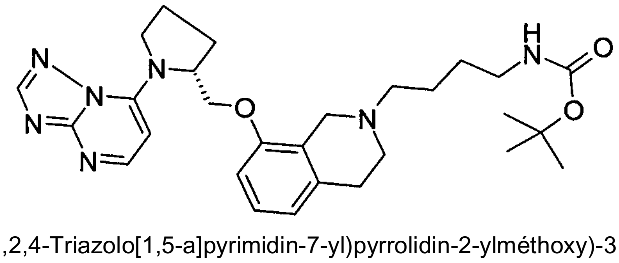 {4-[8-((R)-1-(1,2,4-Triazolo[1,5-a]pyrimidin-7-yl)pyrrolidin-2-ylméthoxy)-3,4-dihydro-1H-isoquinoléin-2-yl]-butyl}carbamate de tertio-butyle |

(suite)

| Composé n° | Nom et/ou structure |
|---|---|
| « A36 » | 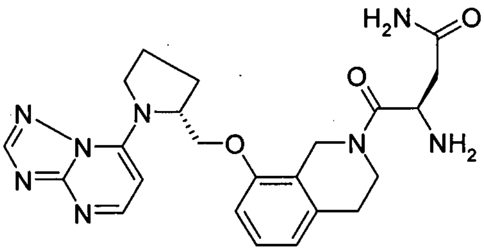<br>(R)-3-Amino-4-oxo-4-[8-((R)-1-(1,2,4-triazolo[1,5-a]pyrimidin-7-yl)pyrrolidin-2-ylméthoxy)-3,4-dihydro-1H-isoquinoléin-2-yl]butyramide |
| « A37 » | 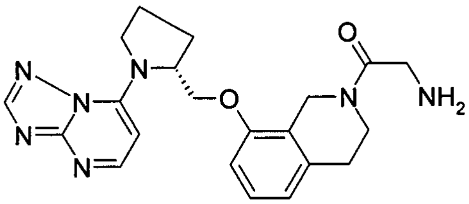<br>2-Amino-1-[8-((R)-1-(1,2,4-triazolo[1,5-a]pyrimidin-7-yl)-pyrrolidin-2-ylméthoxy)-3,4-dihydro-1H-isoquinoléin-2-yl]éthanone |
| « A38 » | 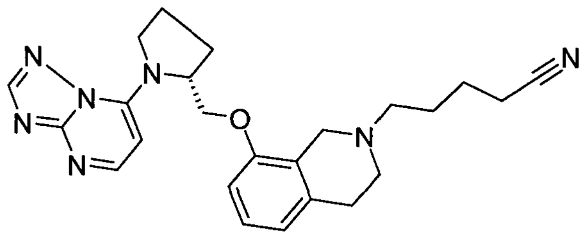<br>5-[8-((R)-1-(1,2,4-Triazolo[1,5-a]pyrimidin-7-yl)pyrrolidin-2-ylméthoxy)-3,4-dihydro-1H-isoquinoléin-2-yl]pentanenitrile |
| « A39 » | 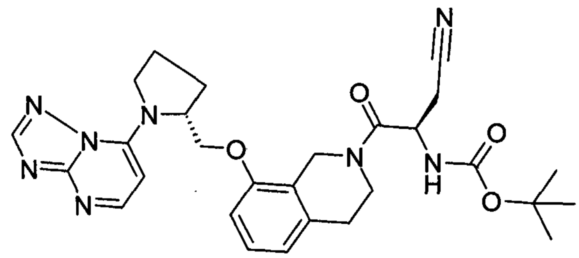<br>{(R)-2-Cyano-1-[8-((R)-1-(1,2,4-triazolo[1,5-a]pyrimidin-7-yl)pyrrolidin-2-ylméthoxy)-3,4-dihydro-1H-isoquinoléine-2-carbonyl]éthyl}carbamate de tertio-butyle |
| « A40 » | 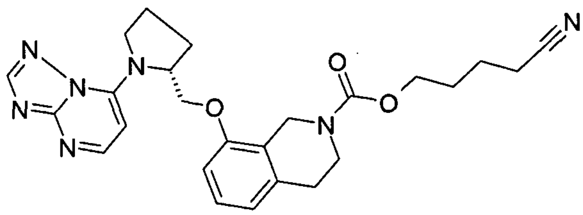<br>8-((R)-1-(1,2,4-Triazolo[1,5-a]pyrimidin-7-yl)pyrrolidin-2-yl-méthoxy)-3,4-dihydro-1H-isoquinoléine-2-carboxylate de 4-cyanobutyle |

(suite)

| Composé n° | Nom et/ou structure |
|---|---|
| « A41 » | 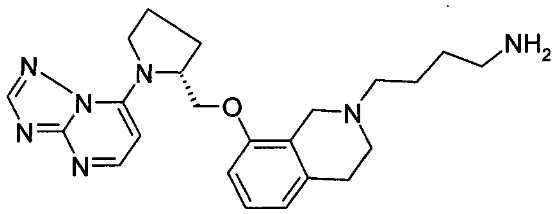<br>4-[8-((R)-1-(1,2,4-Triazolo[1,5-a]pyrimidin-7-yl)pyrrolidin-2-ylméthoxy)-3,4-dihydro-1H-isoquinoléin-2-yl]butylamine |
| « A42 » | 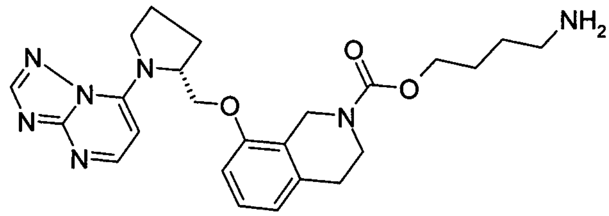<br>8-((R)-1-(1,2,4-Triazolo[1,5-a]pyrimidin-7-yl)pyrrolidin-2-yl-méthoxy)-3,4-dihydro-1H-isoquinoléine-2-carboxylate de 4-aminobutyle |
| « A46 » | 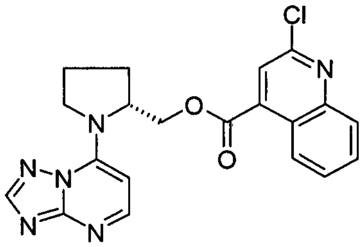<br>2-Chloroquinoléine-4-carboxylate de (R)-1-(1,2,4-triazolo[1,5-a]pyrimidin-7-yl)pyrrolidin-2-ylméthyle |
| « A53 » | 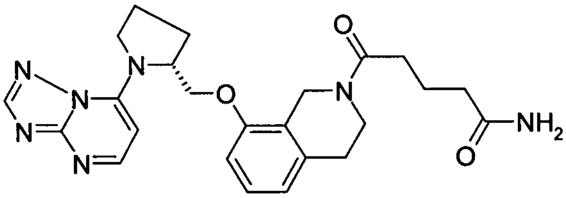<br>5-Oxo-5-[8-((R)-1-(1,2,4-triazolo[1,5-a]pyrimidin-7-yl)-pyrrolidin-2-ylméthoxy)-3,4-dihydro-1H-isoquinoléin-2-yl]-pentanamide |
| « A59 » | 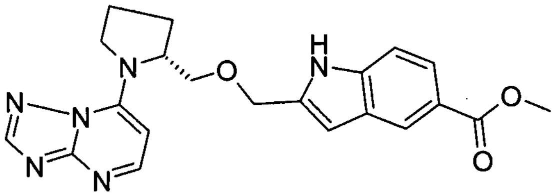<br>2-((R)-1-(1,2,4-Triazolo[1,5-a]pyrimidin-7-yl)pyrrolidin-2-yl-méthoxyméthyl)-1H-indole-5-carboxylate de méthyle |

(suite)

| Composé n° | Nom et/ou structure |
|---|---|
| « A75 » | 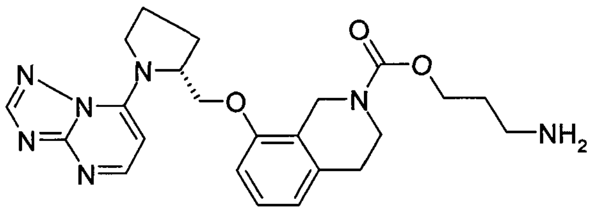<br>8-((R)-1-(1,2,4-Triazolo[1,5-a]pyrimidin-7-yl)pyrrolidin-2-yl-méthoxy)-3,4-dihydro-1H-isoquinoléine-2-carboxylate de 3-aminopropyle |
| « A76 » | 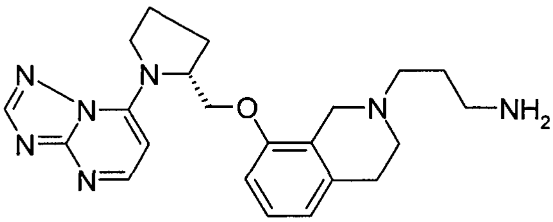<br>3-[8-((R)-1-(1,2,4-Triazolo[1,5-a]pyrimidin-7-yl)pyrrolidin-2-ylméthoxy)-3,4-dihydro-1H-isoquinoléin-2-yl]propylamine |
| « A77 » | 7-[(R)-2-(Naphtalén-1-yloxyméthyl)pyrrolidin-1-yl]-[1,2,4]triazolo[1,5-a]-1,3,5-triazine |
| « A78 » | 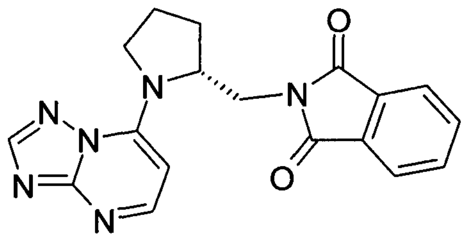<br>2-((R)-1-(1,2,4-Triazolo[1,5-a]pyrimidin-7-yl)pyrrolidin-2-yl-méthyl)isoindole-1,3-dione |
| « A81 » | 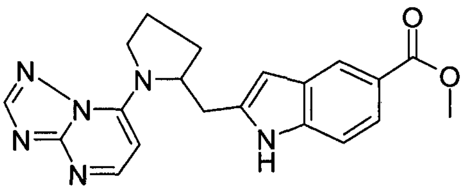<br>2-(1-(1,2,4-Triazolo[1,5-a]pyrimidin-7-yl)pyrrolidin-2-yl-méthyl)-1H-indole-5-carboxylate de méthyle |
| « A93 » | 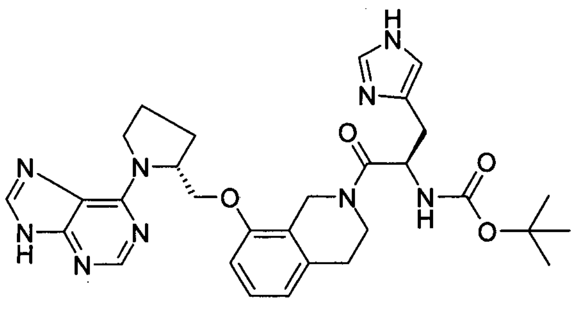<br>((R)-1-(1H-Imidazol-4-ylméthyl)-2-oxo-2-{8-[(R)-1-(9H-purin-6-yl)pyrrolidin-2-ylméthoxy]-3,4-dihydro-1H-isoquinoléin-2-yl}éthyl)carbamate de tertio-butyle |

et les sels, tautomères et stéréoisomères pharmaceutiquement utilisables de ceux-ci, y compris des mélanges de ceux-ci en toutes proportions.

**3.** Procédé de préparation de composés de formule I selon les revendications 1-2 et de sels, tautomères et stéréoisomères pharmaceutiquement utilisables de ceux-ci, **caractérisé en ce que**
un composé de formule II

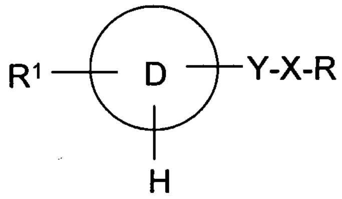

II

dans laquelle $R^1$, X, Y et R revêtent les significations indiquées selon la revendication 1,
est réagi avec un composé de formule III

Z-Cl III

dans laquelle Z revêt la signification indiquée selon la revendication 1,
et/ou
une base ou un acide de formule I est converti(e) en l'un de ses sels.

**4.** Médicaments comprenant au moins un composé de formule I selon les revendications 1-2 et/ou des sels, tautomères et stéréoisomères pharmaceutiquement utilisables de celui-ci, y compris des mélanges de ceux-ci en toutes proportions, et éventuellement des excipients et/ou des adjuvants.

**5.** Utilisation de composés selon les revendications 1-2 et de sels, tautomères et stéréoisomères pharmaceutiquement utilisables de ceux-ci, y compris des mélanges de ceux-ci en toutes proportions, pour la préparation d'un médicament destiné au traitement de tumeurs, de métastases tumorales, de maladies prolifératives des cellules mésangiales, de l'hémangiome, de la rétinopathie proliférative, de la polyarthrite rhumatoïde, de la néovascularisation athérosclérotique, du psoriasis, de la néovascularisation oculaire, de l'ostéoporose, du diabète et de l'obésité, de la leucémie lymphoïde et du lymphome.

**6.** Utilisation selon la revendication 5, **caractérisée en ce que** la maladie tumorale est choisie dans le groupe de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital , du système lymphatique, de l'estomac, du larynx, du poumon, de la peau, la leucémie monocytique, l'adénocarcinome du poumon, le carcinome du poumon à petites cellules, le cancer du pancréas, le glioblastome, le carcinome du sein, la leucémie myéloïde aiguë, la leucémie myéloïde chronique, la leucémie lymphoïde aiguë, la leucémie lymphoïde chronique, le lymphome hodgkinien, le lymphome non hodgkinien.

**7.** Utilisation de composés de formule I selon les revendications 1-2 et/ou de sels physiologiquement acceptables de ceux-ci, pour la préparation d'un médicament destiné au traitement de tumeurs, où une quantité thérapeutiquement efficace d'un composé de formule I est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

**8.** Utilisation de composés de formule I selon les revendications 1-2 et/ou de sels physiologiquement acceptables de ceux-ci, pour la préparation d'un médicament destiné au traitement de tumeurs, où une quantité thérapeutiquement efficace d'un composé de formule I est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

**9.** Composés selon la revendication 1, choisis dans le groupe constitué par

| Composé n° | Nom et/ou structure |
|---|---|
| « A19 » | 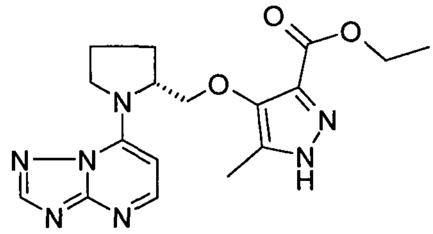 5-Méthyl-4-((R)-1-(1,2,4-triazolo[1,5-a]pyrimidin-7-yl)-pyrrolidin-2-ylméthoxy)-1H-pyrazole-3-carboxylate d'éthyle |
| « A26 » | 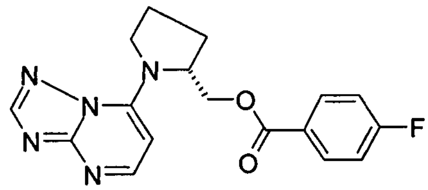 4-Fluorobenzoate de (R)-1-(1,2,4-triazolo[1,5-a]pyrimidin-7-yl)pyrrolidin-2-ylméthyle |
| « A43 » | 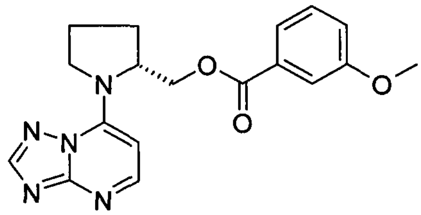 3-Méthoxybenzoate de (R)-1-(1,2,4-triazolo[1,5-a]-pyrimidin-7-yl)pyrrolidin-2-ylméthyle |
| « A44 » | 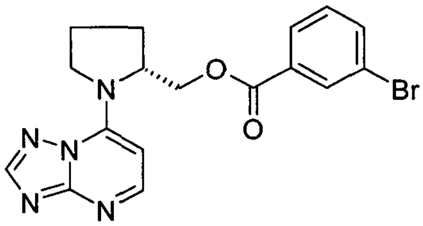 3-Bromobenzoate de (R)-1-(1,2,4-triazolo[1,5-a]pyrimidin-7-yl)pyrrolidin-2-ylméthyle |
| « A45 » | 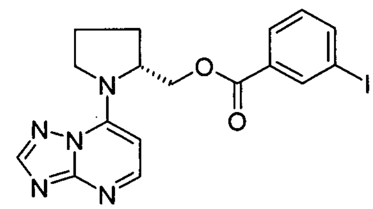 3-Iodobenzoate de (R)-1-(1,2,4-triazolo[1,5-a]pyrimidin-7-yl)pyrrolidin-2-ylméthyle |
| « A50 » | 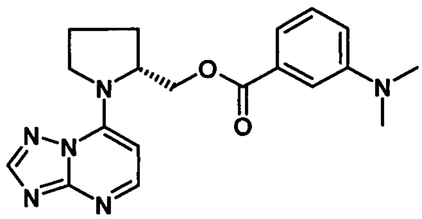 3-Diméthylaminobenzoate de (R)-1-(1,2,4-triazolo[1,5-a]pyrimidin-7-yl)pyrrolidin-2-ylméthyle |

(suite)

| Composé n° | Nom et/ou structure |
|---|---|
| « A52 » | 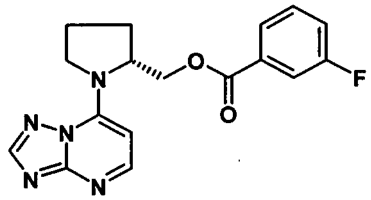 3-Fluorobenzoate de (R)-1-(1,2,4-triazolo[1,5-a]pyrimidin-7-yl)pyrrolidin-2-ylméthyle |
| « A61 » | 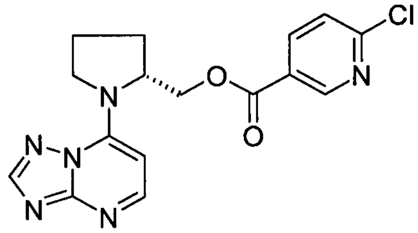 6-Chloronicotinate de (R)-1-(1,2,4-triazolo[1,5-a]pyrimidin-7-yl)pyrrolidin-2-ylméthyle |
| « A63 » | 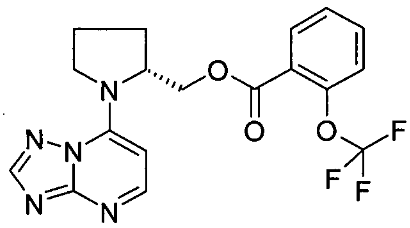 2-Trifluorométhoxybenzoate de (R)-1-(1,2,4-triazolo[1,5-a]pyrimidin-7-yl)pyrrolidin-2-ylméthyle |
| « A64 » | 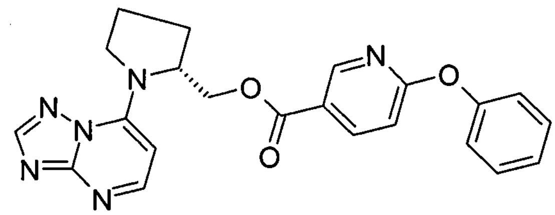 6-Phénoxynicotinate de (R)-1-(1,2,4-triazolo[1,5-a]pyrimidin-7-yl)pyrrolidin-2-ylméthyle |
| « A68 » | 2-Iodobenzoate de (R)-1-(1,2,4-triazolo[1,5-a]pyrimidin-7-yl)pyrrolidin-2-ylméthyle |
| « A69 » | Pyridine-2-carboxylate de (R)-1-(1,2,4-triazolo[1,5-a]pyrimidin-7-yl)pyrrolidin-2-ylméthyle |
| « A70 » | 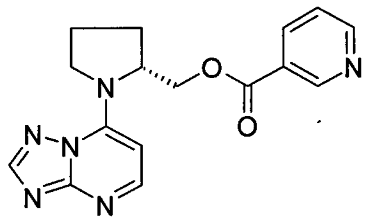 Nicotinate de (R)-1-(1,2,4-triazolo[1,5-a]pyrimidin-7-yl)pyrrolidin-2-ylméthyle |
| « A71 » | Benzoate de (R)-1-(1,2,4-Triazolo[1,5-a]pyrimidin-7-yl)-pyrrolidin-2-ylméthyle |
| « A72 » | 3-Ethoxybenzoate de (R)-1-(1,2,4-triazolo[1,5-a]pyrimidin-7-yl)pyrrolidin-2-ylméthyle |
| « A73 » | 5-Chloro-2-fluorobenzoate de (R)-1-(1,2,4-triazolo[1,5-a]pyrimidin-7-yl)pyrrolidin-2-ylméthyle |
| « A74 » | 3-Chlorométhylbenzoate de (R)-1-(1,2,4-triazolo[1,5-a]pyrimidin-7-yl)pyrrolidin-2-ylméthyle |
| « A79 » | 3-Cyanobenzoate de (R)-1-(1,2,4-triazolo[1,5-a]pyrimidin-7-yl)pyrrolidin-2-ylméthyle |

(suite)

| Composé n° | Nom et/ou structure |
|---|---|
| « A82 » | 3-Trifluorométhoxybenzoate de (R)-1-(1,2,4-triazolo[1,5-a]pyrimidin-7-yl)pyrrolidin-2-ylméthyle |
| « A83 » | 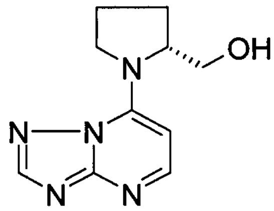 ((R)-1-(1,2,4-Triazolo[1,5-a]pyrimidin-7-yl)pyrrolidin-2-yl)-méthanol |

et les sels, tautomères et stéréoisomères pharmaceutiquement utilisables de ceux-ci, y compris des mélanges de ceux-ci en toutes proportions.

**10.** Médicaments comprenant au moins un composé selon la revendication 9 et/ou des sels, tautomères et stéréoisomères pharmaceutiquement utilisables de celui-ci, y compris des mélanges de ceux-ci en toutes proportions, et éventuellement des excipients et/ou des adjuvants.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2007017069 A
- WO 0179157 A
- WO 02081415 A
- WO 2008011114 A
- WO 0050032 A
- US 6069134 A


### In der Beschreibung aufgeführte Nicht-Patentliteratur


- **F. SPINELLA et al.** *J. Cardiovasc. Pharmacol.,* 2004, vol. 44, 140
- **A. MORABITO et al.** *Crit. Rev. Oncol./Hematol.,* 2004, vol. 49, 91
- **B. NICHOLSON et al.** *Cancer Metastas. Rev.,* 2001, vol. 20, 297
- **E. NG et al.** *Can. J. Ophthalmol.,* 2005, vol. 23, 3706
- *Pharmaceutical Research,* 1986, vol. 3 (6), 318
- **YASUO MAKISUMI.** *Hideo Kano Bull Soc. Chem. Japan,* 1959, vol. 8, 907